# EUROPEAN PATENT APPLICATION

(11) **EP 4 628 098 A1**
(43) Date of publication of application: **08.10.2025**
(21) Application number: 23896923.2
(22) Date of filing: 01.12.2023
(51) Int. Cl.: A61K 45/00, C07D 215/227, A61K 31/496, A61P 25/00

(54) **TPK AGONIST AND METHOD FOR USING SAME TO TREAT NEURODEGENERATIVE DISEASES**

(30) Priority: 02.12.2022 CN 202211547332
(71) Applicant: Shanghai Raising Pharmaceutical Co., Ltd., Pudong New District Shanghai 201321 (CN)
(72) Inventor: ZHANG, Huan, Shanghai 201321 (CN); MENG, Lijuan, Shanghai 201321 (CN)
(74) Representative: Michalski Hüttermann & Partner Patentanwälte mbB
(86) International application number: PCT/CN2023/135776
(87) International publication number: WO 2024/114781

(57) **Abstract**

The present disclosure falls under the field of biomedicine and specifically relates to a method for preventing or treating neurodegenerative diseases or alleviating symptoms of neurodegenerative diseases. An embodiment comprises administering an prophylactically or therapeutically effective dose of a thiamine pyrophosphokinase (TPK) agonist to an individual in need thereof.

## Description

### FIELD OF THE INVENTION

The present invention belongs to the field of biomedicine, and specifically relates to a method for the prophylaxis or treatment of neurodegenerative diseases or alleviating symptoms of neurodegenerative diseases. Said method comprises administering a prophylactically or therapeutically effective amount of a thiamine pyrophosphokinase (TPK) agonist to a subject in need thereof.

### BACKGROUND OF THE INVENTION

Alzheimer's disease (AD) is the most common degenerative disease of the central nervous system. Due to the large number of patients, prolonged disease course, long survival period with loss of self-care ability, and lack of effective prophylactical and therapeutic drugs, it imposes enormous economic and psychological burdens on individuals, families, and society as a whole. According to reports, China's expenditures on AD prevention, treatment, and care in 2015 reached as high as 167.74 billion US dollars, and it is estimated that by 2030, these expenditures will rise to 507.49 billion US dollars. In 2018, global expenditures on dementia-related diseases, predominantly AD, exceeded one trillion US dollars, accounting for over 1% of the global gross domestic product (GDP). AD is the only disease among the top ten major global diseases that lacks effective prophylactical and therapeutic drugs. It has become a significant disease severely affecting the healthcare systems and sustainable economic development of major global economies, including China.

AD is a disease involving multiple pathophysiological alterations, including neuronal loss, glial cell activation, characteristic extracellular β-amyloid protein (Aβ) deposition forming senile plaques, and intracellular hyperphosphorylated Tau protein causing neurofibrillary tangles. Additionally, synaptic loss, impaired cerebral glucose metabolism, oxidative stress, and other pathological changes are consistently observed in AD brains. Reduced cerebral glucose metabolism in patients is closely associated with cognitive dysfunction. Due to unclear pathogenesis, AD still lacks effective treatment methods.

### SUMMARY OF THE INVENTION

The inventors of the present application discovered that glucose metabolism disorders may be an early pre-clinical feature of AD. The intracellular glucose metabolism process in AD patients is mainly characterized by a significant decrease in the activities of three key enzymes (pyruvate dehydrogenase, α-ketoglutarate dehydrogenase, and transketolase) that rely on thiamine diphosphate (TDP) as a coenzyme. A multi-center clinical study has proven that the decrease in TDP levels in AD patients is a specific and common phenomenon with good diagnostic value, while there is no thiamine metabolism abnormality in patients with vascular dementia and frontotemporal dementia. Clinical and experimental studies further indicate that the decrease in TDP is the cause of cerebral glucose metabolism disorders. The inventors of the present application have found that among the four known genes related to thiamine metabolism, only the expression of thiamine pyrophosphokinase (TPK), a key enzyme that converts thiamine into the bioactive TDP, is significantly inhibited, and this inhibition of TPK expression is specific to AD. Therefore, TPK agonists can be used for the prophylaxis or treatment of neurodegenerative diseases (especially Alzheimer's disease).

In one aspect, the present invention provides a method for the prophylaxis or treatment of neurodegenerative diseases or alleviating symptoms of neurodegenerative diseases, which comprises administering to a subject in need thereof a prophylactically or therapeutically effective amount of a thiamine pyrophosphokinase (TPK) agonist.

In another aspect, the present invention provides use of a TPK agonist in the manufacture of a medicament for the prophylaxis or treatment of neurodegenerative diseases or alleviating symptoms of neurodegenerative diseases.

In another aspect, the present invention provides a TPK agonist for use in the prophylaxis or treatment of neurodegenerative diseases or alleviating symptoms of neurodegenerative diseases.

The neurodegenerative disease is preferably Alzheimer's disease; more preferably, the Alzheimer's disease is one in which the subject has decreased TPK enzyme activity, decreased TPK expression level, and/or decreased TDP level.

In another aspect, the present invention relates to TPK agonists with a novel structures.

### DETAILED DESCRIPTION OF THE INVENTION

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

As used herein, when describing a divalent group that connects two other groups, it is understood that the divalent group can be connected to the two groups in any direction. For example, if the other two groups connected by the divalent group -C(=O)NR- are (Group 1) and (Group 2) respectively, then both (Group 1)-C(=O)NR-(Group 2) and (Group 2)-C(=O)NR-(Group 1) are included.

As used herein, the term "alkylene" refers to a saturated divalent hydrocarbyl, preferably refers to a saturated divalent hydrocarbyl having 1, 2, 3, 4, 5 or 6 carbon atoms, e.g., methylene, ethylene, propylene or butylene.

As used herein, the term "alkyl" is defined as a straight or branched saturated aliphatic hydrocarbon. In some embodiments, alkyl has 1-12, e.g., 1-6, carbon atoms. For example, as used herein, the term "C₁₋₆ alkyl" refers to a linear or branched group having 1-6 carbon atoms (such as methyl, ethyl, n-propyl, isopropyl, n-butyl, isobutyl, sec-butyl, *tert-butyl,* n-pentyl, or n-hexyl), which is optionally substituted with one or more (e.g., 1 to 3) suitable substituents such as halogen (in which case the group may be referred to as "haloalkyl") (e.g., CF₃, C₂F₅, CHF₂, CH₂F, CH₂CF₃, CH₂Cl or -CH₂CH₂CF₃ *etc.).* The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or *tert-butyl).*

As used herein, the term "alkenyl" refers to a linear or branched monovalent hydrocarbyl having one or more double bonds and 2-6 carbon atoms ("C₂₋₆ alkenyl"). The alkenyl is e.g., CH=CH₂, -CH₂CH=CH₂, -C(CH₃)=CH₂, -CH₂-CH=CH-CH₃, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present invention contains an alkenyl group, the compound may exist as the pure E (entgegen) form, the pure Z (zusammen) form, or any mixture thereof. The term "alkenylene" refers to a corresponding divalent group, including, e.g., "C₂₋₆ alkenylene", "C₂₋₄ alkenylene", *etc.,* and the specific examples thereof include but are not limited to: -CH=CH-, -CH₂CH=CH-, -C(CH₃)=CH-, butenylene, pentenylene, hexenylene, cyclopentenylene, cyclohexenylene, *etc.*

As used herein, the term "alkynyl" refers to a monovalent hydrocarbyl containing one or more triple bonds, preferably having 2, 3, 4, 5 or 6 carbon atoms, such as ethynyl, 2-propynyl, 2-butynyl, buta-1,3-diynyl, *etc.* The alkynyl group is optionally substituted with one or more (e.g., 1 to 3) same or different substituents. The term "alkynylene" refers to a corresponding divalent group, including e.g., "C₂₋₈ alkynylene", "C₂₋₆ alkynylene", "C₂₋₄ alkynylene", *etc.* Examples thereof include, but are not limited to, 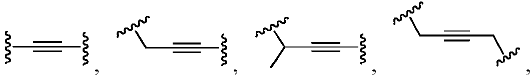 and the like, and the alkynylene group is optionally substituted with one or more (e.g., 1 to 3) same or different substituents.

As used herein, the term "fused ring" refers to a ring system formed by two or more ring structures sharing two adjacent atoms with each other.

As used herein, the term "spiro" refers to a ring system formed by two or more ring structures sharing one ring atom with each other.

As used herein, the term "bridged ring" refers to a ring system formed by two or more ring structures sharing two atoms which are not directly linked with each other.

As used herein, the terms "cyclic hydrocarbylene", "cyclic hydrocarbyl" and "hydrocarbon ring" refer to a saturated (i.e., "cycloalkylene" and "cycloalkyl") or unsaturated (i.e., having one or more double and/or triple bonds in the ring) monocyclic or polycyclic (including spiro ring, fused ring or bridged ring) hydrocarbon ring having e.g., 3-10 (suitably having 3-8, and more suitably having 3-6) ring carbon atoms, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the term "cycloalkyl" refers to a saturated, monocyclic or polycyclic (e.g., bicyclic or tricyclic) hydrocarbon ring (e.g., monocyclic, such as cyclopropyl, cyclobutyl, cyclopentyl, cyclohexyl, cycloheptyl, cyclooctyl, or cyclononyl, or bicyclic, including spiro, fused or bridged cyclic system (such as bicyclo[1.1.1]pentyl, bicyclo[2.2.1]heptyl, bicyclo[3.2.1]octyl or bicyclo[5.2.0]nonyl, or decahydronaphthalene *etc.)),* which is optionally substituted with one or more (e.g., 1 to 3) suitable substituents. The cycloalkyl has 3 to 15 carbon atoms. For example, the term "C₃₋₁₀ cycloalkyl" refers to a saturated, monocyclic or polycyclic (e.g., bicyclic) hydrocarbon ring having 3 to 10 ring forming carbon atoms (e.g., cyclopropyl, cyclobutyl, cyclopentyl, or cyclohexyl), which is optionally substituted with one or more (e.g., 1 to 3) suitable substituents, e.g., methyl substituted cyclopropyl.

As used herein, the term "heterocyclyl" refers to a saturated or unsaturated monovalent monocyclic or polycyclic (e.g., bicyclic or tricyclic) group having 2, 3, 4, 5, 6, 7, 8 or 9 carbon atoms and one or more (e.g., one, two, three or four) heteroatom-containing groups in the ring, wherein the heteroatom-containing group is selected from O, S, N, S(=O), S(=O)₂, S(=O)(=NR^{Z}), NR^{Z} or P(=O)(R^{Z}), wherein R^{Z}, at each occurrence, independently represents a hydrogen atom or a C₁₋₆ alkyl or a halo-C₁₋₆ alkyl; said heterocycloalkyl may be attached to the remainder of the molecule through any one of said carbon atoms or a nitrogen atom (if present). In particular, a 3- to 14-membered heterocyclyl is a group having 3 to 14 carbon atoms and heteroatoms in the ring, such as but not limited to oxiranyl, aziridinyl, azetidinyl, oxetanyl, tetrahydrofuranyl, dioxolinyl, pyrrolidinyl, pyrrolidonyl, imidazolidinyl, pyrazolidinyl, pyrrolinyl, tetrahydropyranyl, piperidinyl, morpholinyl, dithianyl, thiomorpholinyl, piperazinyl or trithianyl.

As used herein, the term "heterocyclyl" encompasses fused ring structures, the attachment point with another group can be from any ring in the fused ring structure. As such, the heterocyclyl of the present invention also includes but is not limited to heterocyclyl-fused heterocyclyl, heterocyclyl-fused cycloalkyl, monoheterocyclyl-fused monoheterocyclyl, monoheterocyclyl-fused monocycloalkyl, such as 3-7-membered (mono)heterocyclyl-fused 3-7-membered (mono)heterocyclyl, 3-7-membered (mono)heterocyclyl-fused (mono)cycloalkyl, 3-7-membered (mono)heterocyclyl-fused C₄₋₆ (mono)cycloalkyl, and the examples include, but are not limited to, pyrrolidinyl-fused cyclopropyl, cyclopentyl-fused azacyclopropyl, pyrrolidinyl-fused cyclobutyl, pyrrolidinyl-fused pyrrolidinyl, pyrrolidinyl-fused piperidinyl, pyrrolidinyl-fused piperazinyl, piperidinyl-fused morpholinyl,

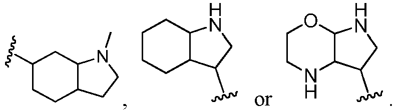

As used herein, the term "heterocyclyl" encompasses bridged heterocyclyl and spiro heterocyclyl.

As used herein, the term "bridged heterocycle" refers to a ring structure comprising one or more (e.g., 1, 2, 3 or 4) heteroatoms (e.g., oxygen, nitrogen, and/or sulfur atoms), formed by two saturated rings sharing two ring atoms which are not directly linked, including, but not limited to, 7-10-membered bridged heterocycle, 8-10-membered bridged heterocycle, 7-10-membered nitrogen-containing bridged heterocycle, 7-10-membered oxygen-containing bridged heterocycle, 7-10-membered sulfur-containing bridged heterocycle, and the like, such as 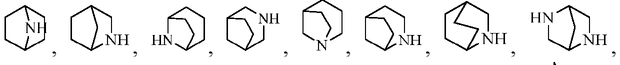 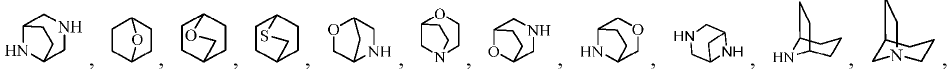 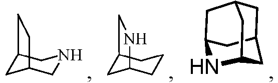 *etc.* The "nitrogen-containing bridged heterocycle", "oxygen-containing bridged heterocycle" and "sulfur-containing bridged heterocycle" optionally further comprise one or more additional heteroatoms selected from oxygen, nitrogen and sulfur.

As used herein, the term "spiro heterocycle" refers to a ring structure comprising one or more (e.g., 1, 2, 3 or 4) heteroatoms (e.g., oxygen atoms, nitrogen atoms, sulfur atoms) formed by two or more saturated rings sharing one ring atom, including, but not limited to, 5-10-membered spiro heterocycle, 6-10-membered spiro heterocycle, 6-10-membered nitrogen-containing spiro heterocycle, 6-10-membered oxygen-containing spiro heterocycle, and 6-10-membered sulfur-containing heterocycle, *etc.,* such as 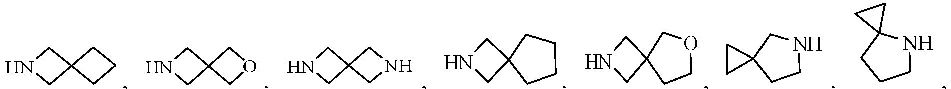 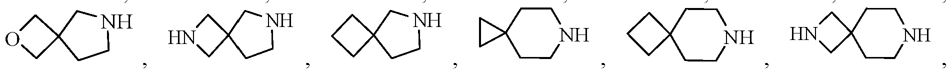 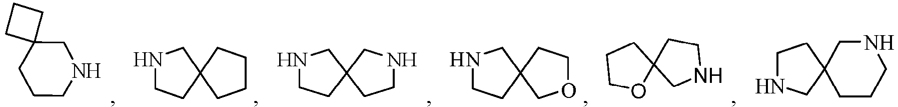 and 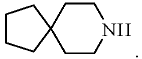 The "nitrogen-containing spiro heterocycle", "oxygen-containing spiro heterocycle" and "sulfur-containing spiro heterocycle" optionally further comprise one or more additional heteroatoms selected from oxygen, nitrogen and sulfur. The term "6-10-membered nitrogen-containing spiro heterocyclyl" refers to a spiro heterocyclyl group comprising a total of 6-10 ring atoms, and at least one of the ring atoms is a nitrogen atom.

As used herein, the terms "aryl(ene)" and "aromatic ring" refer to a monocyclic or fused-ring polycyclic aromatic group having a conjugated π electron system. For example, as used herein, the terms "C₆₋₁₀ aryl(ene)" and "C₆₋₁₀ aromatic ring" refer to an aromatic group containing 6 to 10 carbon atoms, such as phenyl(ene) (benzene ring) or naphthyl(ene) (naphthalene ring). Aryl(ene) or aromatic ring is optionally substituted with one or more (such as 1 to 3) suitable substituents (e.g., halogen, -OH, -CN, -NO₂, and C₁₋₆ alkyl, *etc.).* When an aryl(ene) group and an aromatic ring are fused rings, the fused ring can be a hydrocarbon ring, a heterocyclic ring or a heteroaromatic ring, and the point of attachment of the fused ring structure to another group may be at any ring of the fused ring structure.

The term "aralkyl" preferably means aryl substituted alkyl, wherein the aryl and the alkyl are as defined herein. Normally, the aryl group may have 6-14 carbon atoms, and the alkyl group may have 1-6 carbon atoms. Exemplary aralkyl group includes, but is not limited to, benzyl, phenylethyl, phenylpropyl, phenylbutyl.

As used herein, the terms "heteroaryl(ene)" and "heteroaromatic ring" refer to a monocyclic, bicyclic or tricyclic aromatic ring system having 5, 6, 7, 8, 9, 10, 11, 12, 13 or 14 ring atoms, particularly 1 or 2 or 3 or 4 or 5 or 6 or 9 or 10 carbon atoms, and containing at least one heteroatom (such as O, N, or S), which can be same to different. In particular, "heteroaryl(ene)" or "heteroaromatic ring" is selected from the group consisting of thienyl(ene) (ring), furyl(ene) (ring), pyrrolyl(ene) (ring), oxazolyl(ene) (ring), thiazolyl(ene) (ring), imidazolyl(ene) (ring), pyrazolyl(ene) (ring), isoxazolyl(ene) (ring), isothiazolyl(ene) (ring), oxadiazolyl(ene) (ring), triazolyl(ene) (ring), thiadiazolyl(ene) (ring) *etc.,* and benzo derivatives thereof; or pyridinyl(ene) (ring), pyridazinyl(ene) (ring), pyrimidinyl(ene) (ring), pyrazinyl(ene) (ring), triazinyl(ene) (ring), *etc.* When the heteroaryl(ene) and heteroaromatic ring are fused rings, the ring as fused may be a hydrocarbon ring, a heterocycle, an aromatic ring, or a heteroaromatic ring, and the point of attachment of the fused ring structure to another group may be at any ring of the fused ring structure.

As used herein, the term "halo" or "halogen" are defined to include F, Cl, Br, or I.

As used herein, the term "alkylthio" means an alkyl group as defined above attached to a parent molecular moiety through a sulfur atom. Representative examples of C₁₋₆ alkylthio include, but are not limited to, methylthio, ethylthio, *tert*-butylthio and hexylthio.

As used herein, the term "nitrogen containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may further optionally comprise one or more (e.g., one, two, three or four) ring members selected from the group consisting of N, O, C=O, S, S=O and S(=O)₂. The nitrogen-containing heterocycle is connected with the rest of the molecule through a nitrogen atom. The nitrogen-containing heterocycle is preferably a saturated nitrogen-containing monocyclic ring. In particular, a 3- to 14-membered nitrogen-containing heterocycle is a group having 3-14 carbon atoms and heteroatoms (at least one of which is a nitrogen atom) in the ring, including but not limited to a 3-membered nitrogen-containing heterocycle (such as aziridinyl), a 4-membered nitrogen-containing heterocycle (such as azetidinyl), a 5-membered nitrogen-containing heterocycle (such as pyrrolyl, pyrrolidinyl (pyrrolidinyl ring), pyrrolinyl, pyrrolidonyl, imidazolyl, imidazolidinyl, imidazolinyl, pyrazolyl, pyrazolinyl), 6-membered nitrogen-containing heterocycle (such as piperidinyl (piperidinyl ring), morpholinyl, thiomorpholinyl, piperazinyl), 7-membered nitrogen-containing heterocycle, etc.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted," the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more of a list of substituents, one or more of the hydrogens on the nitrogen (to the extent there are any) may each be replaced with an independently selected optional substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen (such as deuterium (D, ²H), tritium (T, ³H)); carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆*, or DMSO-*d₆*.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, ester, solvate, metabolite or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof.

A suitable acid addition salt is formed from an acid which forms a pharmaceutically acceptable salt. Specific examples include aspartate, benzoate, bicarbonate/carbonate, bisulfate/sulfate, fumarate, gluceptate, gluconate, glucuronate, hexafluorophosphate, hydrobromide/bromide, hydroiodide/iodide, maleate, malonate, methylsulfate, naphthylate, nicotinate, nitrate, orotate, oxalate, palmitate and the like.

A suitable base addition salt is formed from a base which forms a pharmaceutically acceptable salt. Specific examples include aluminum, arginine, choline, diethylamine, lysine, magnesium, meglumine, potassium, and the like.

For a review on suitable salts, see "Hand book of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

As used herein, the term "ester" refers to those derived from the compounds of the various formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

The metabolite of the compound of the present invention, namely a substance formed *in vivo* upon administration of the compound of the present invention, is also included within the scope of the present invention. Such a product may result e.g., from the oxidation, reduction, hydrolysis, amidation, de-amidation, esterification, de-esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted *in vivo* into the compound with desired therapeutic activity. Further information on the use of the prodrug may be found in "Pro-drugs as Novel Delivery Systems, Vol. 14, ACS Symposium Series (T. Higuchi and V. Stella) and "Bioreversible Carriers in Drug Design," Pergamon Press, 1987 (edited by E. B. Roche, American Pharmaceutical Association). The prodrug in accordance with the invention can, for example, be produced by replacing appropriate functionalities present in the compound of the present invention with certain moieties known to those skilled in the art as "pro-moieties" as described, for example, in "Design of Prodrugs" by H. Bundgaard (Elsevier, 1985).

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which is incorporated herein by reference. The protecting groups may be removed at a convenient subsequent stage using methods known from the art.

As used herein, the term "about" refers to a range within ±10%, preferably within ±5%, and more preferably within ±2% of the specified value.

As used herein, the term "effective amount" refers to the amount of a compound being administered which will relieve to some extent one or more of the symptoms of the disorder being treated.

As used herein, the term "prophylaxis" refers to administering a medicament beforehand to avert or forestall the appearance of one or more symptoms of a disease or disorder. The person of ordinary skill in the medical art recognizes that the term "prophylaxis" is not an absolute term. In the medical art, it is understood to refer to the prophylactic administration of a drug to substantially diminish the likelihood or seriousness of a condition, or symptom of the condition, and this is the sense intended in this disclosure. Prophylactic measures are divided between primary prophylaxis (to prevent the development of a disease) and secondary prophylaxis (whereby the disease has already developed, and the patient is protected against worsening of this process).

Unless otherwise indicated, the term "treating" or "treatment", as used herein, means reversing, alleviating, inhibiting the progress of the disorder or condition to which such term applies, or one or more symptoms of such disorder or condition.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g., birds, amphibians, reptiles) and mammals, such as non-human primates, livestock and/or domesticated animals (such as sheep, dog, cat, cow, pig and the like).

As used herein, the term "decreased TPK enzyme activity" means that the TPK enzyme activity in the treated subject is lower than that in a normal subject.

As used herein, the term "decreased TPK expression level" means that the TPK mRNA/DNA level or protein expression level in the treated subject is lower than that in a normal subject.

As used herein, the term "decreased TDP level" means that the TDP level in the treated subject is lower than that in a normal subject.

In some embodiments, the present invention provides a method for the prophylaxis or treatment of neurodegenerative diseases or alleviating symptoms of neurodegenerative diseases, which comprises administering to a subject in need thereof a prophylactically or therapeutically effective amount of a thiamine pyrophosphokinase (TPK) agonist.

In a preferred embodiment, the neurodegenerative disease is Alzheimer's disease.

In a more preferred embodiment, the Alzheimer's disease is one in which the subject has decreased TPK enzyme activity, decreased TPK expression level, and/or decreased TDP level.

In some embodiments, the TPK agonist is a compound of Formula (I), or a pharmaceutically acceptable salt, ester, stereoisomer, polymorph, solvate, N-oxide, isotopically labeled compound, metabolite, or prodrug thereof: 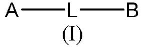 wherein:
A and B are each independently C₃₋₁₀ hydrocarbon ring, 3- to 14-membered heterocycle, C₆₋₁₀ aromatic ring or 5- to 14-membered heteroaromatic ring;
L is selected from the group consisting of -Q¹-, -W-, -Q¹-W-, -W-Q¹-, -Q¹-Q²-, -W-W'-, -W-Q¹-Q²-, -W-Q¹-W'-, -Q¹-W-Q²-, -Q¹-W-Q²-W'-, -W-Q¹-W'-Q²-, -Q¹-Q²-W-W'- and -W-Q¹-Q²-W'-;
Q¹ and Q² are each independently selected from the group consisting of -C₁₋₆ alkylene-, -C₂₋₆ alkenylene-, -C₂₋₆ alkynylene-, -C₃₋₁₀ cyclic hydrocarbylene-, -(3- to 14-membered heterocyclylene)-, -C₆₋₁₀ arylene- and -(5- to 14-membered heteroarylene)-, wherein the alkylene, alkenylene and alkynylene are each optionally interrupted by one group selected from the following or interrupted by multiple adjacent or non-adjacent groups independently selected from the following: -C₃₋₁₀ cyclic hydrocarbylene-, -(3- to 14-membered heterocyclylene)-, -C₆₋₁₀ arylene-, -(5- to 14-membered heteroarylene)-, -O-, -C(=O)-, -C(=O)O-, -NR-, -C(=O)NR-, -NR-C(=O)-NR'-, -NR-C(=O)O-, -(S=O)NR-, -S(=O)₂NR-, -S-, -S(=O)- and -S(=O)₂-;
W and W', at each occurrence, are each independently selected from the group consisting of -O-, -C(=O)-, -C(=O)O-, -NR-, -C(=O)NR-, -NR-C(=O)-NR'-, -NR-C(=O)O-, -(S=O)NR-, -S(=O)₂NR-, -S-, -S(=O)- and -S(=O)₂-;
R and R', at each occurrence, are each independently selected from the group consisting of H, C₁₋₆ alkyl, C₂₋₆ alkenyl, C₂₋₆ alkynyl, C₃₋₁₀ cyclic hydrocarbyl, 3- to 14-membered heterocyclyl, C₆₋₁₀ aryl, 5- to 14-membered heteroaryl and C₆₋₁₂ aralkyl;
the above alkyl, alkylene, alkenyl, alkenylene, alkynyl, alkynylene, cyclic hydrocarbyl, cyclic hydrocarbylene, hydrocarbon ring, heterocyclyl, heterocyclylene, heterocycle, aryl, arylene, aromatic ring, heteroaryl, heteroarylene, heteroaromatic ring and aralkyl, at each occurrence, are each optionally substituted with one or more substituents independently selected from the group consisting of: halogen, -OH, =O, -NH₂, -CN, -NO₂, C₁₋₆ alkyl, haloC₁₋₆ alkyl, C₃₋₁₀ cyclic hydrocarbyl, 3- to 14-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-OR^{a}, -C₁₋₆ alkylene-NR^{a}R^{b} and -O-C₁₋₆ alkylene-NR^{a}R^{b}, the alkyl, alkylene, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₋₆ haloalkyl, C₃₋₁₀ cyclic hydrocarbyl, 3- to 14-membered heterocyclyl, C₆₋₁₀ aryl, 5- to 14-membered heteroaryl, C₆₋₁₂ aralkyl, -O-C₁₋₆ alkyl and -C₁₋₆ alkylene-O-C₁₋₆ alkyl; and
R^{a} and R^{b}, at each occurrence, are each independently selected from the group consisting of H, C₁₋₆ alkyl, C₃₋₁₀ cyclic hydrocarbyl, 3- to 14-membered heterocyclyl, C₆₋₁₀ aryl, 5- to 14-membered heteroaryl and C₆₋₁₂ aralkyl, the alkyl, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₋₆ haloalkyl, C₃₋₁₀ cyclic hydrocarbyl, 3- to 14-membered heterocyclyl, C₆₋₁₀ aryl, 5- to 14-membered heteroaryl, C₆₋₁₂ aralkyl and -C₁₋₆ alkylene-O-C₁₋₆ alkyl.

In some embodiments, A is

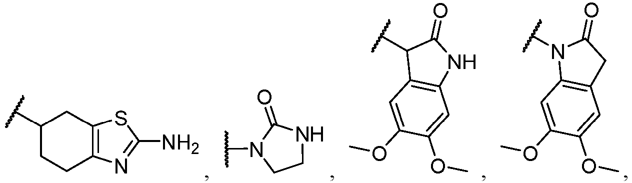

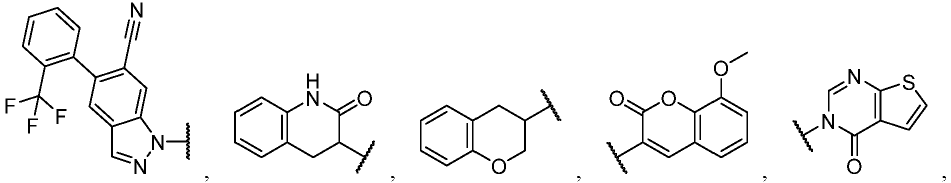

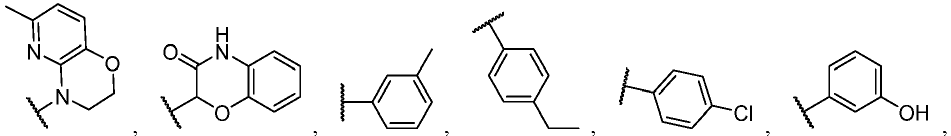

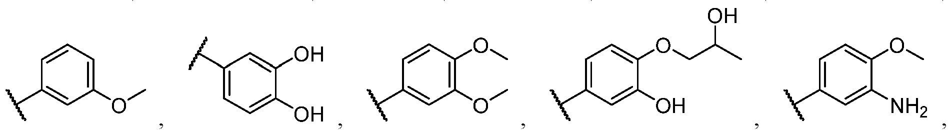

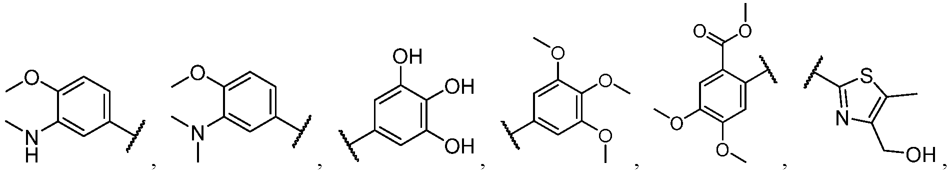

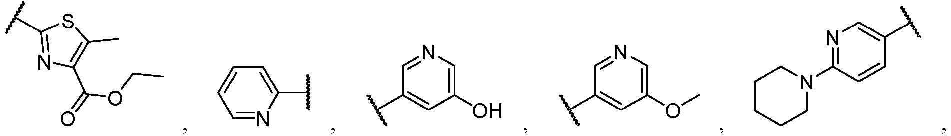

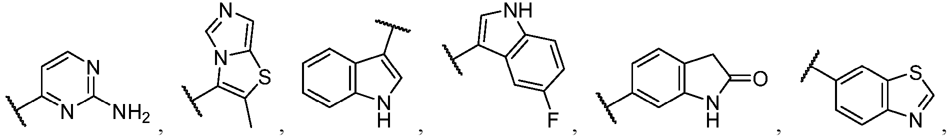

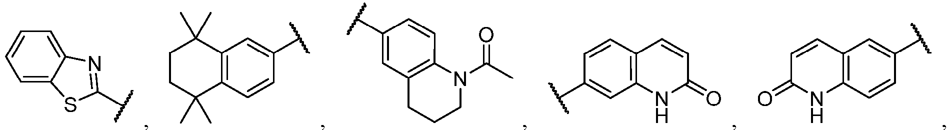

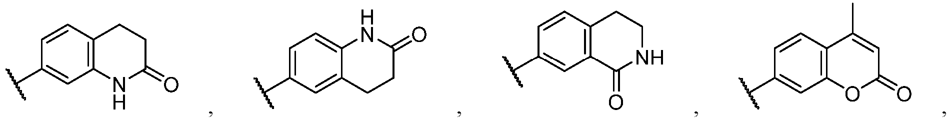

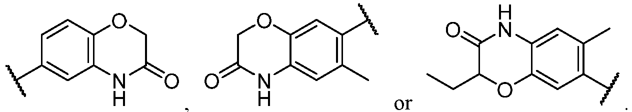

In some embodiments, L is selected from the group consisting of -Q¹-W-, -W-Q¹-, -Q¹-Q²-, -W-Q¹-Q²-, -W-Q¹-W'-, -Q¹-W-Q²-, -Q¹-W-Q²-W'-, -W-Q¹-W'-Q²-, -Q¹-Q²-W-W'- and -W-Q¹-Q²-W'-.

In some embodiments, L is 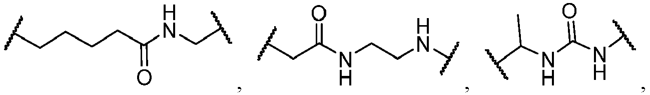 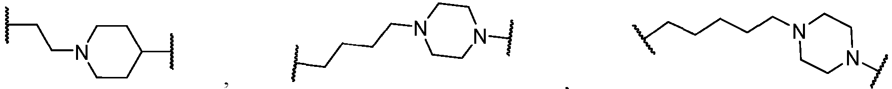 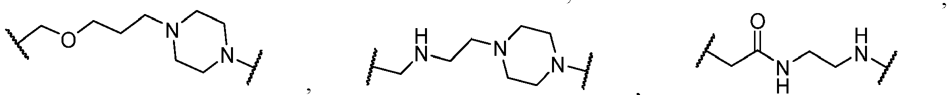 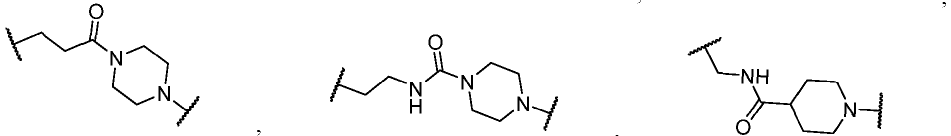 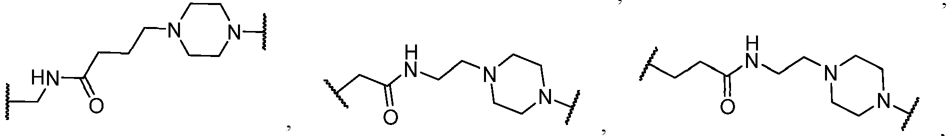 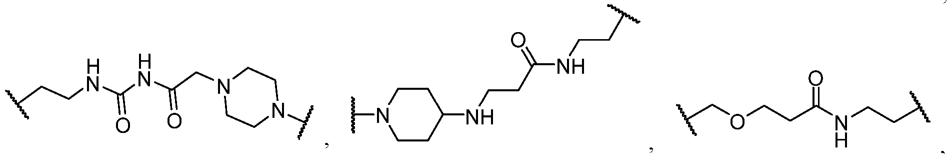 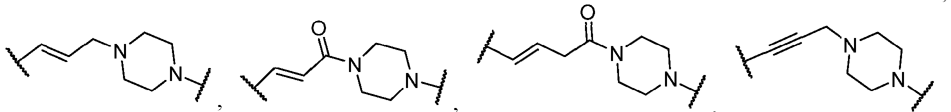 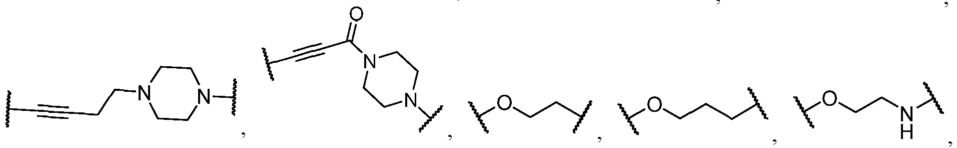 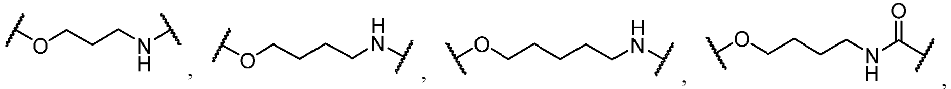 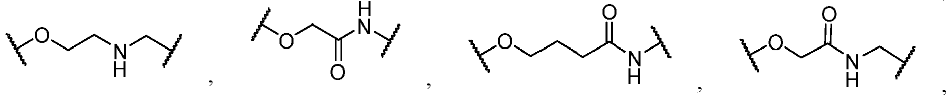 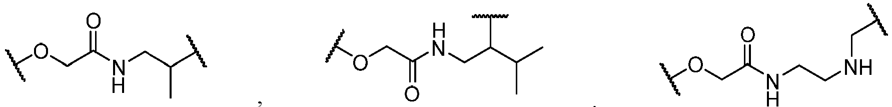 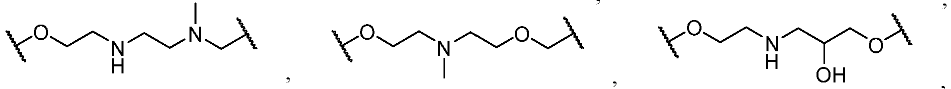 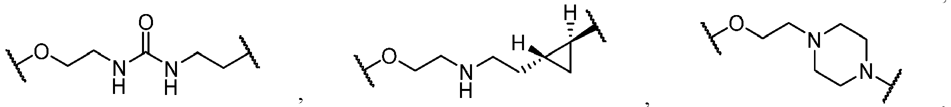 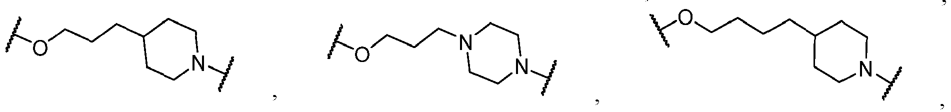 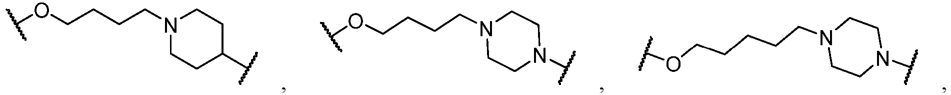 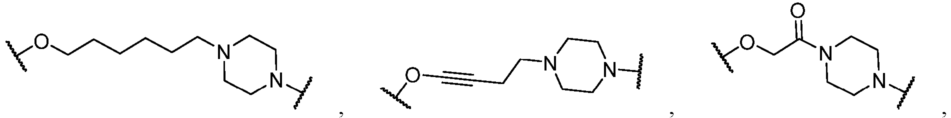 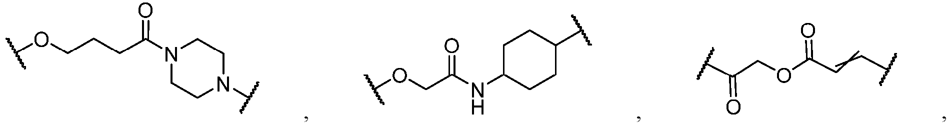 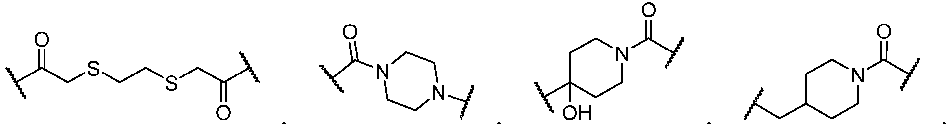 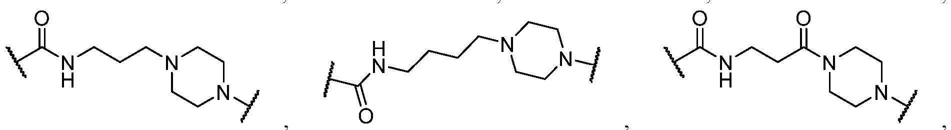 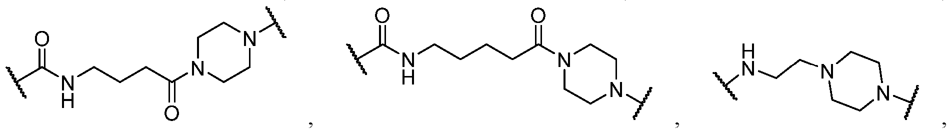 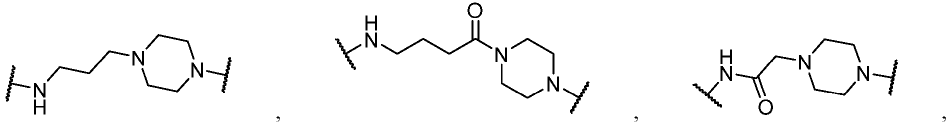 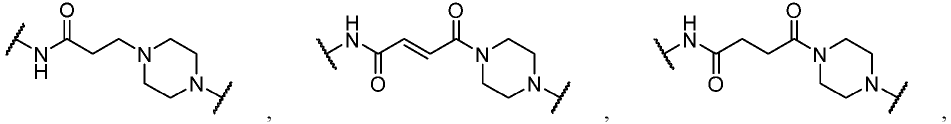 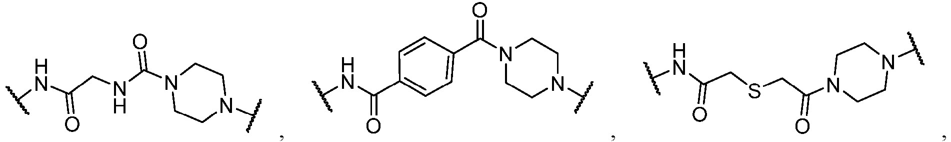 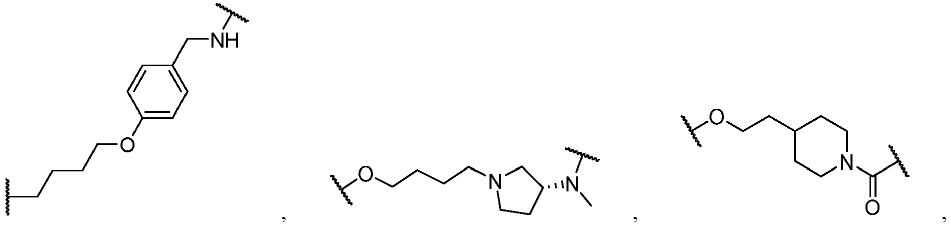 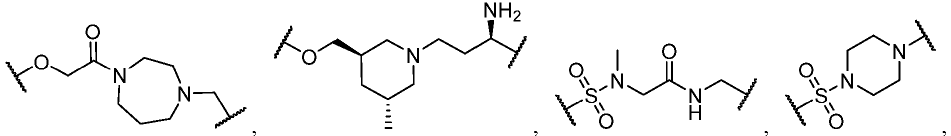 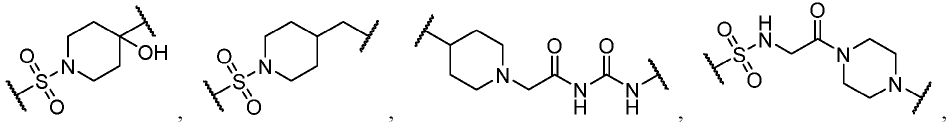 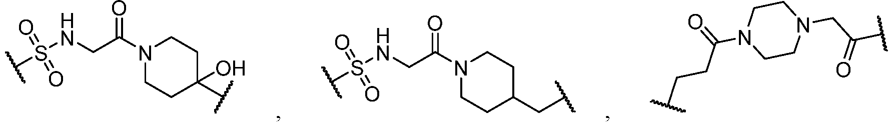 or

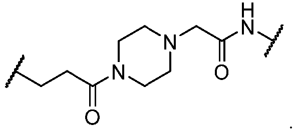

In some embodiments, B is

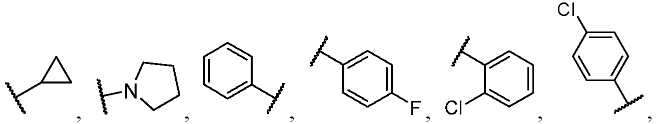

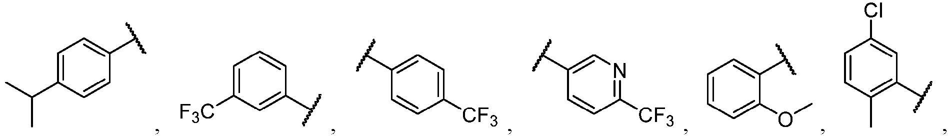

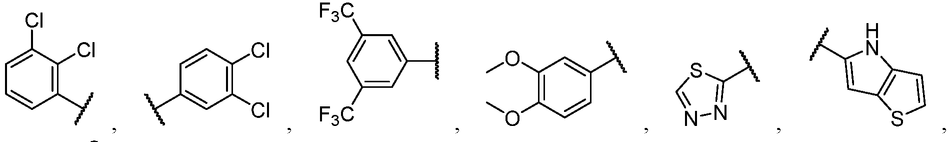

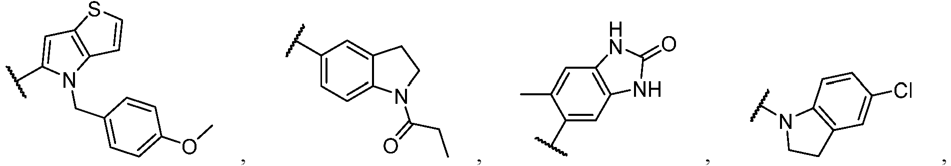

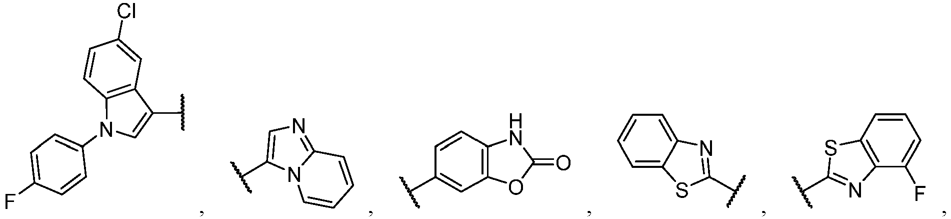

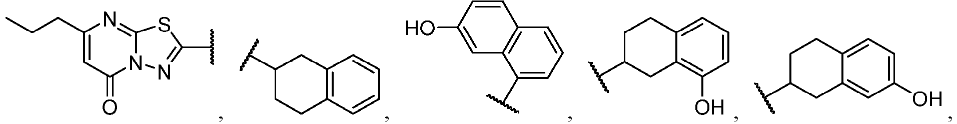

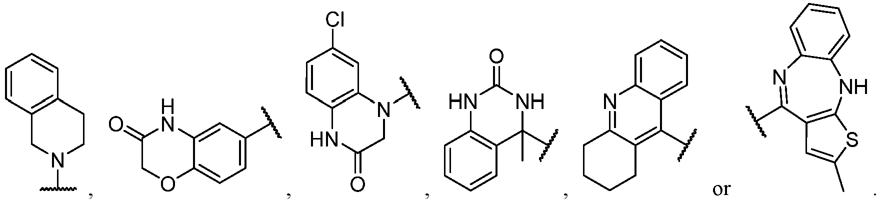

In some embodiments, the TPK agonist is a compound of Formula (II), or a pharmaceutically acceptable salt, ester, stereoisomer, polymorph, solvate, N-oxide, isotopically labeled compound, metabolite, or prodrug thereof:

A-W-Q¹-Q²-B (II)

wherein:
A is a benzene ring optionally fused with a 5- to 6-membered heterocycle or 5- to 6-membered heteroaromatic ring, wherein the benzene ring is optionally substituted with one or more substituents independently selected from the group consisting of: halogen, -OH, -NH₂, C₁₋₆ alkyl, -O-C₁₋₆ alkyl, -NH(C₁₋₆ alkyl) and -N(C₁₋₆ alkyl)₂; preferably, the benzene ring is optionally substituted with one or more substituents independently selected from the group consisting of: -Cl, -OH, -NH₂, -NH(CH₃), -N(CH₃)₂, methyl, ethyl and methoxy; most preferably, A is 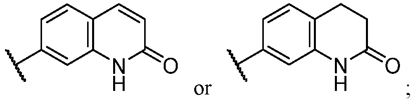
B is C₃₋₁₀ hydrocarbon ring, 3- to 14-membered heterocycle, C₆₋₁₀ aromatic ring or 5- to 14-membered heteroaromatic ring; preferably is a benzene ring, the benzene ring is optionally substituted with one or more substituents independently selected from the group consisting of: halogen, C₁₋₆ alkyl, haloC₁₋₆ alkyl, -NH-C(=O)-C₁₋₆ alkyl, -C(=O)-(3- to 14-membered heterocyclyl), -S(=O)₂-N(C₁₋₆ alkyl)₂ and -S(=O)₂-(3-to 14-membered heterocyclyl); preferably, the benzene ring is optionally substituted with one or more substituents independently selected from the group consisting of: -F, -Cl, methyl, isopropyl, trifluoromethyl, -NHC(=O)CH₃, -C(=O)-piperidinyl, -S(=O)₂-N(CH₃)₂, -S(=O)₂-N(CH₂CH₃)₂, -S(=O)₂-piperidinyl and -S(=O)₂-azepanyl;
Q¹ is selected from the group consisting of -C₁₋₆ alkylene-, -C₂₋₆ alkenylene- and -C₂₋₆ alkynylene-;
Q² is selected from the group consisting of -C₃₋₁₀ cyclic hydrocarbylene-, -(3- to 14-membered heterocyclylene)-, -C₆₋₁₀ arylene- and -(5- to 14-membered heteroarylene)-; preferably is -(3- to 14-membered heterocyclylene)-; and more preferably is piperidinylene or piperazinylene;
W, at each occurrence, is each independently selected from the group consisting of -O-, -C(=O)-, -C(=O)O-, -NR-, -C(=O)NR-, -NR-C(=O)-NR'-, -NR-C(=O)O-, -(S=O)NR-, -S(=O)₂NR-, -S-, -S(=O)- and -S(=O)₂-; preferably is -O-, -NH- or -NH-C(=O)-;
the remaining groups are as defined above.

In some embodiments, the TPK agonist is selected from the group consisting of:

| **No.** | **Structural Formula** |
|---|---|
| **1** | 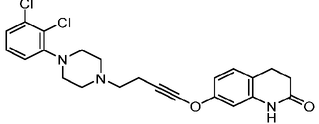 |
| **2** | 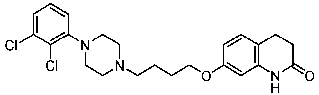 |
| **3** | 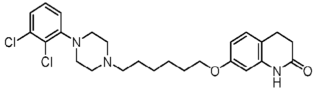 |
| **4** | 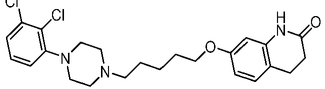 |
| **5** | 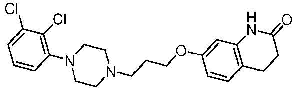 |
| **6** | 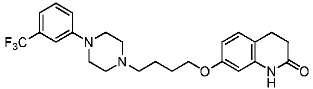 |
| **7** | 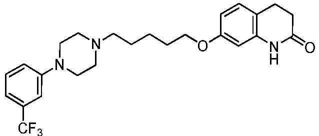 |
| **9** | 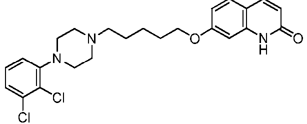 |
| **10** | 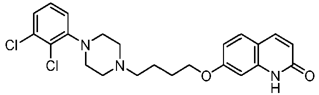 |
| **17** | 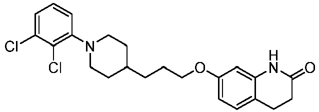 |
| **18** | 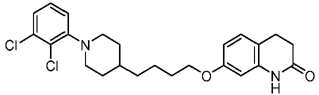 |
| **19** | 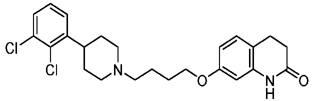 |
| **20** | 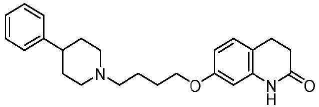 |
| **21** | 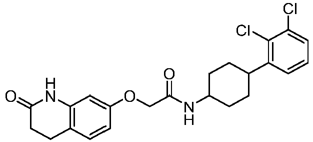 |
| **22** | 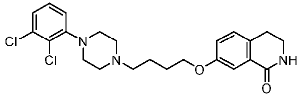 |
| **23** | 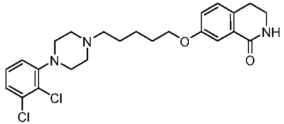 |
| **24** | 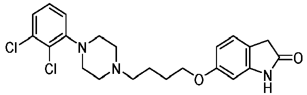 |
| **25** | 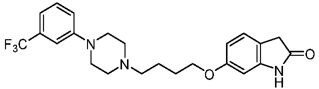 |
| **26** | 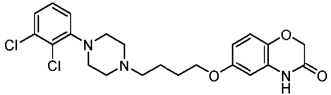 |
| **27** | 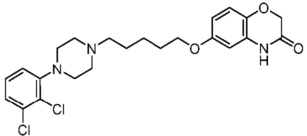 |
| **28** | 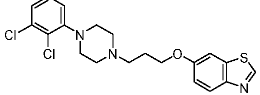 |
| **29** | 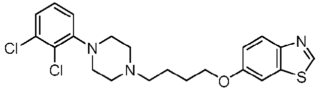 |
| **30** | 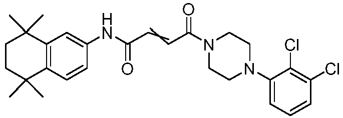 |
| **31** | 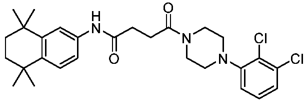 |
| **32** | 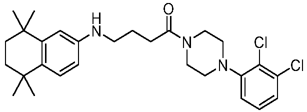 |
| **33** | 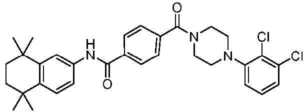 |
| **34** | 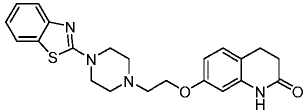 |
| **35** | 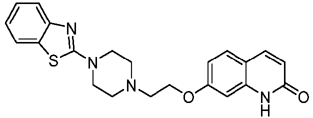 |
| **36** | 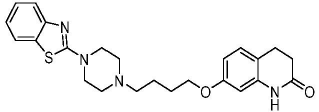 |
| **37** | 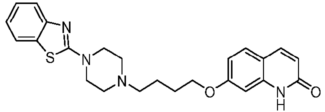 |
| **38** | 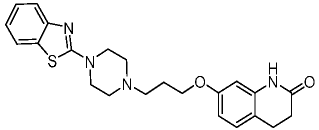 |
| **39** | 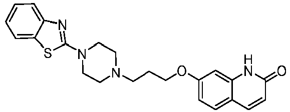 |
| **40** | 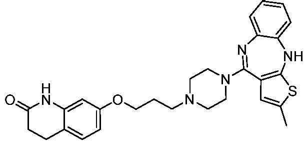 |
| **41** | 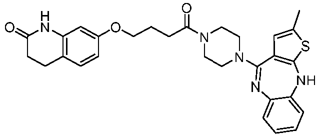 |
| **42** | 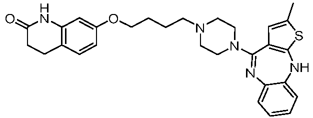 |
| **43** | 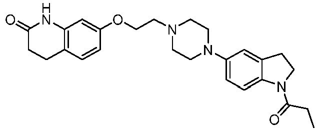 |
| **44** | 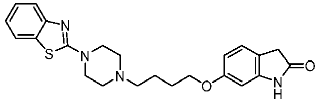 |
| **45** | 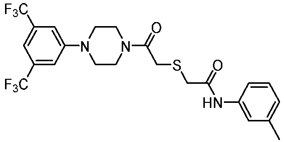 |
| **46** | 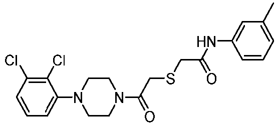 |
| **47** | 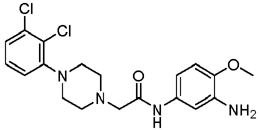 |
| **48** | 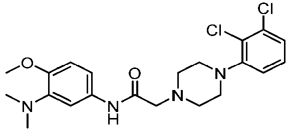 |
| **49** | 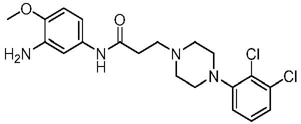 |
| **50** | 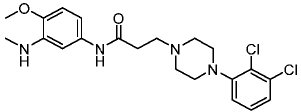 |
| **51** | 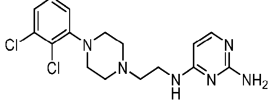 |
| **52** | 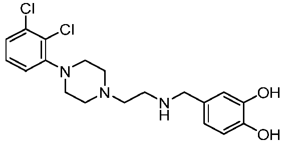 |
| **53** | 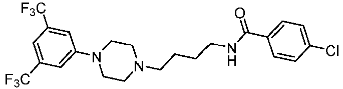 |
| **54** | 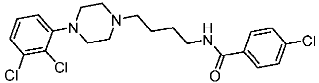 |
| **55** | 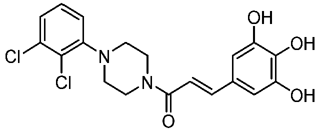 |
| **56** | 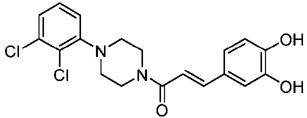 |
| **57** | 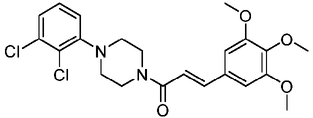 |
| **58** | 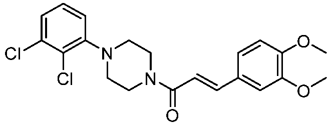 |
| **59** | 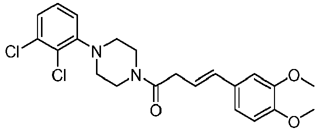 |
| **60** | 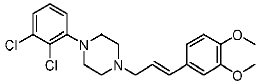 |
| **61** | 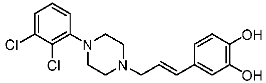 |
| **62** | 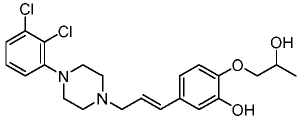 |
| **63** | 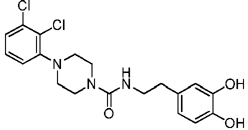 |
| **64** | 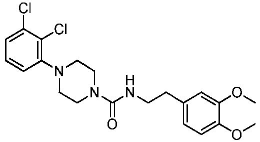 |
| **65** | 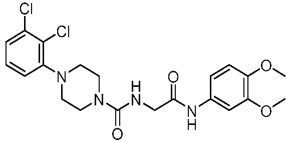 |
| **66** | 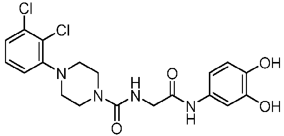 |
| **67** | 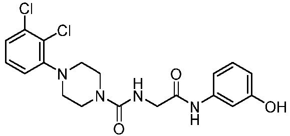 |
| **68** | 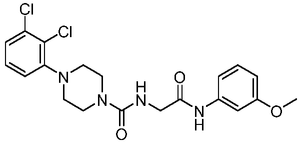 |
| **69** | 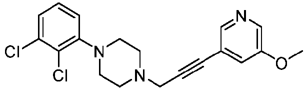 |
| **70** | 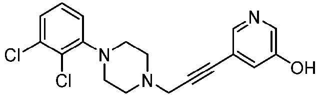 |
| **71** | 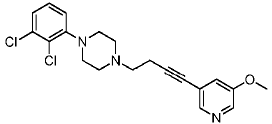 |
| **72** | 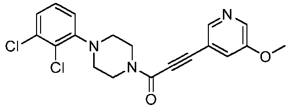 |
| **73** | 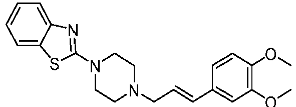 |
| **74** | 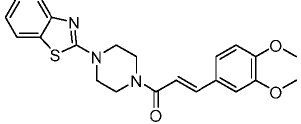 |
| **75** | 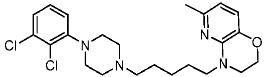 |
| **76** | 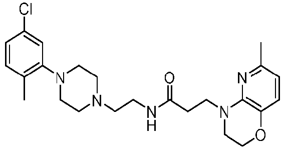 |
| **77** | 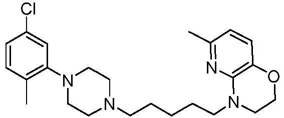 |
| **78** | 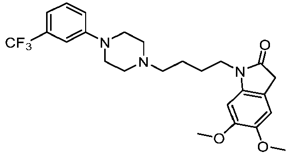 |
| **79** | 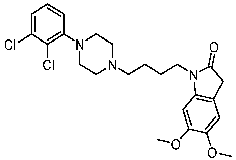 |
| **80** | 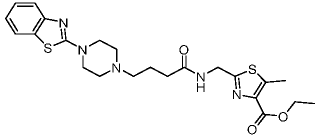 |
| **81** | 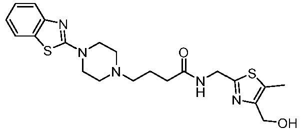 |
| **82** | 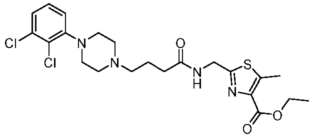 |
| **83** | 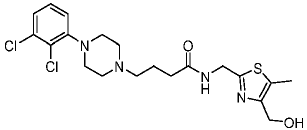 |
| **84** | 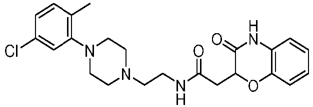 |
| **85** | 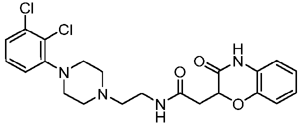 |
| **86** | 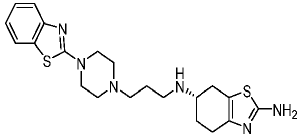 |
| **87** | 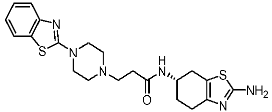 |
| **88** | 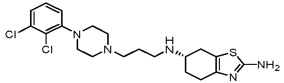 |
| **89** | 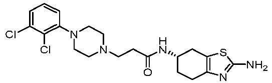 |
| **90** | 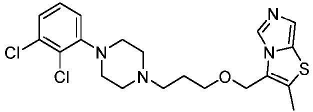 |
| **91** | 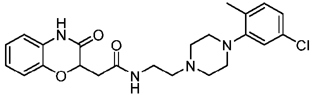 |
| **95** | 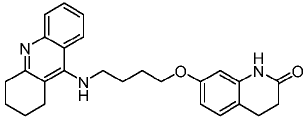 |
| **96** | 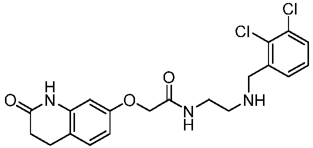 |
| **97** | 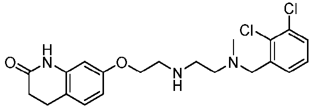 |
| **98** | 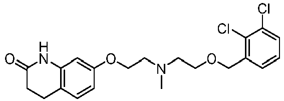 |
| **99** | 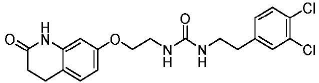 |
| **100** | 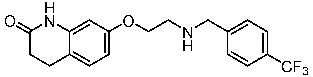 |
| **101** | 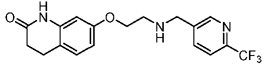 |
| **102** | 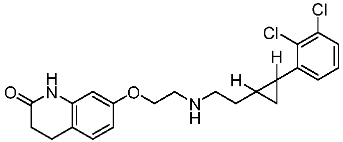 |
| **103** | 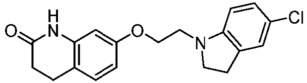 |
| **104** | 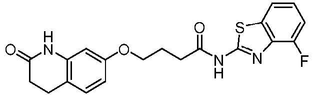 |
| **105** | 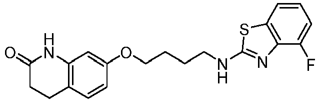 |
| **106** | 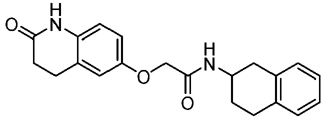 |
| **107** | 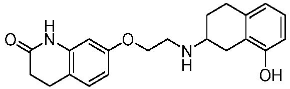 |
| **108** | 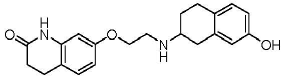 |
| **109** | 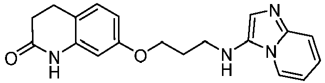 |
| **110** | 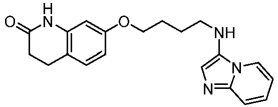 |
| **111** | 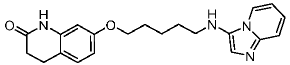 |
| **112** | 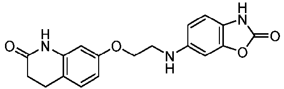 |
| **113** | 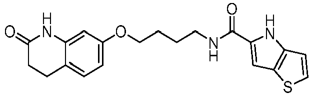 |
| **114** | 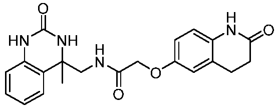 |
| **115** | 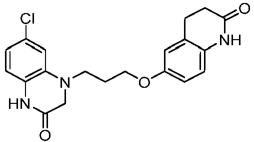 |
| **130** | 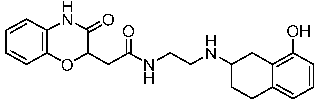 |
| **131** | 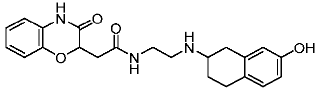 |
| **132** | 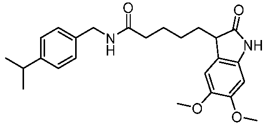 |
| **133** | 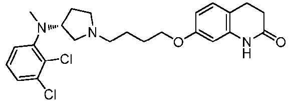 |
| **134** | 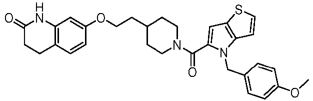 |
| **135** | 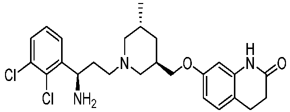 |
| **136** | 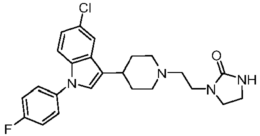 |
| **137** | 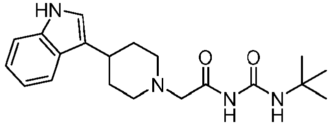 |
| **138** | 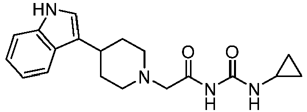 |
| **139** | 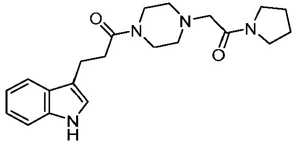 |
| **140** | 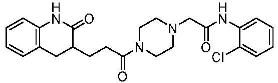 |
| **141** | 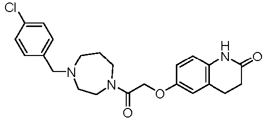 |
| **142** | 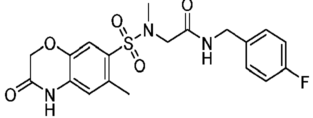 |
| **143** | 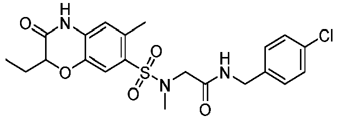 |
| **144** | 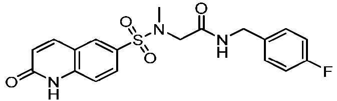 |
| **145** | 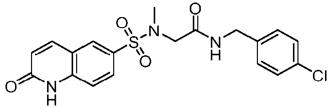 |
| **146** | 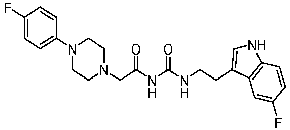 |
| **147** | 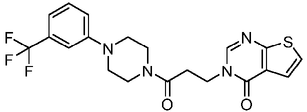 |
| **148** | 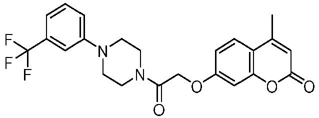 |
| **149** | 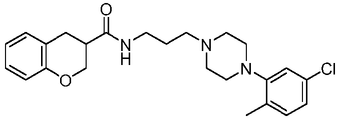 |
| **150** | 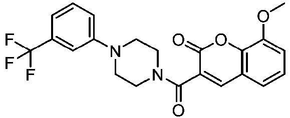 |
| **151** | 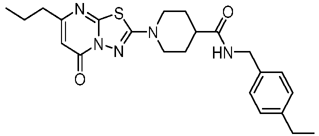 |
| **152** | 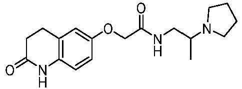 |
| **153** | 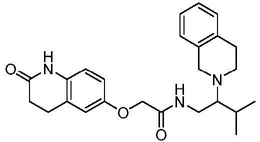 |
| **154** | 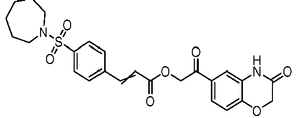 |
| **155** | 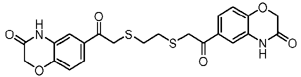 |
| **156** | 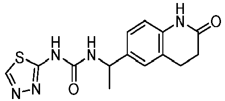 |
| **157** | 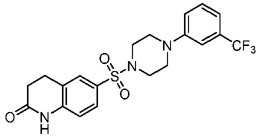 |
| **158** | 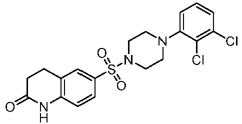 |
| **159** | 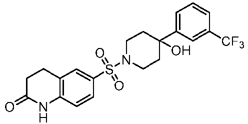 |
| **160** | 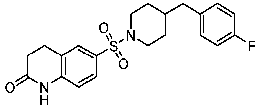 |
| **161** | 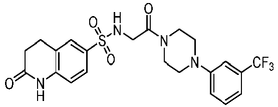 |
| **162** | 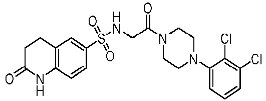 |
| **163** | 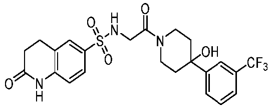 |
| **164** | 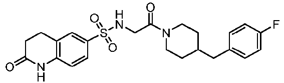 |
| **165** | 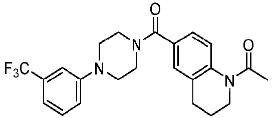 |
| **166** | 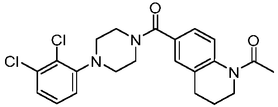 |
| **167** | 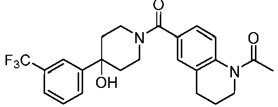 |
| **168** | 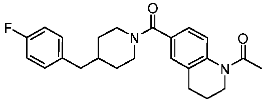 |
| **169** | 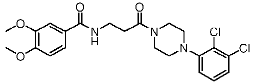 |
| **170** | 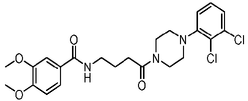 |
| **171** | 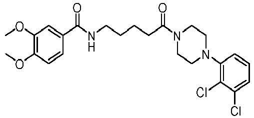 |
| **172** | 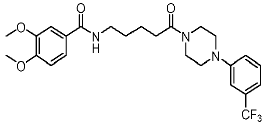 |
| **173** | 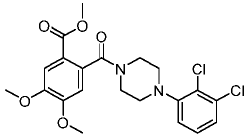 |
| **174** | 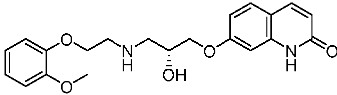 |
| **175** | 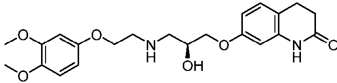 |
| **176** | 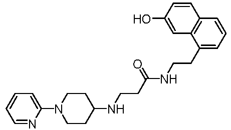 |
| **177** | 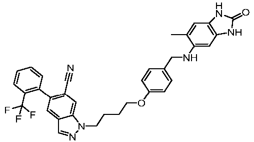 |
| **178** | 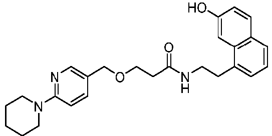 |

In preferred embodiments, the TPK agonist is administered in an amount of about 0.005 mg/day to about 5000 mg/day, e.g., in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

In preferred embodiments, the TPK agonist is administered in an amount of about 1 ng/kg to about 200 mg/kg, about 1 µg/kg to about 100 mg/kg or about 1 mg/kg to about 50 mg/kg body weight per day, e.g., is administered in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg or about 300 mg/kg body weight per day.

In preferred embodiments, the daily dose of the TPK agonist is administered at one time or is administered in two, three or four doses.

In preferred embodiments, the TPK agonist is administered continuously for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days.

In preferred embodiments, the TPK agonist is administered for one or more (e.g., 1, 2, 3, 4, 5, 6, 7, 8, 9 or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days; and the interval between every two courses of treatment is 0, 1, 2, 3, 4, 5, 6, 7, 8, 9, 10 days, two weeks, three weeks, or four weeks.

In preferred embodiments, the TPK agonist is administered through injection (e.g., intravenous, intraarterial, subcutaneous, intraperitoneal, intramuscular injection, including dripping), or transdermal administration, or is administered via oral, buccal, nasal, transmucosal, or topical route, as an ophthalmic formulation, or via inhalation.

In preferred embodiments, the TPK agonist is administered in a dosage form selected from the group consisting of tablet, capsule, lozenge, hard candy, powder, spray, cream, salve, suppository, gel, paste, lotion, ointment, aqueous suspensions, injectable solution, elixir, and syrup.

In preferred embodiments, the method improves the following pathophysiological manifestations in the subject: abnormal cognitive behavior, neurodegenerative changes (e.g., progressive synaptic/neuronal loss and brain atrophy), β-amyloid deposition, abnormal phosphorylation of Tau and the resulting neurofibrillary tangles, glial cell activation and inflammation, and/or impaired cerebral glucose metabolism.

In preferred embodiments, the present disclosure further comprises administering one or more additional therapeutic agents.

In some embodiments, the present disclosure provides a compound, or a pharmaceutically acceptable salt, ester, stereoisomer, polymorph, solvate, N-oxide, isotopically labeled compound, metabolite, or prodrug thereof, wherein the compound is selected from the group consisting of:

| **No.** | **Structural Formula** |
|---|---|
| **1** | 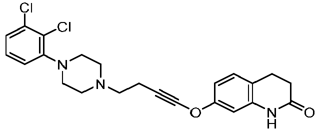 |
| **3** | 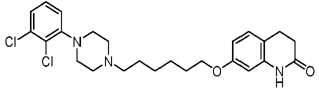 |
| **4** | 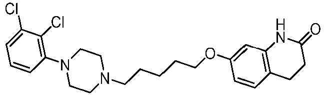 |
| **6** | 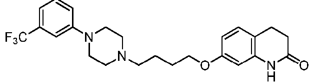 |
| **7** | 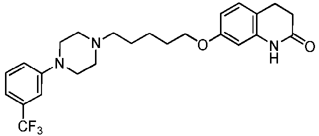 |
| **9** | 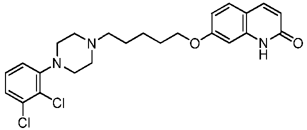 |
| **10** | 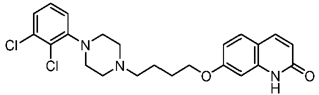 |
| **17** | 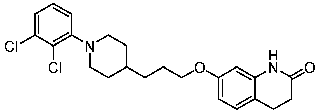 |
| **18** | 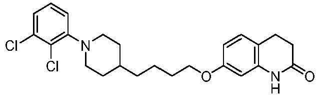 |
| **20** | 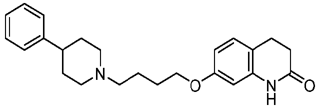 |
| **21** | 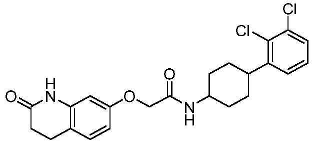 |
| **23** | 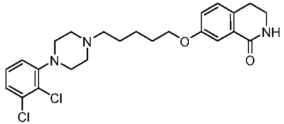 |
| **24** | 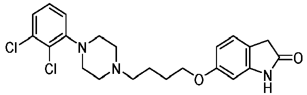 |
| **25** | 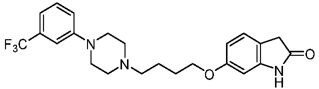 |
| **27** | 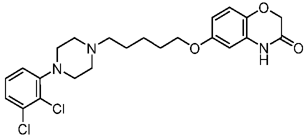 |
| **28** | 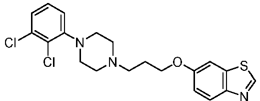 |
| **29** | 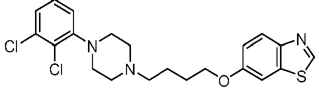 |
| **30** | 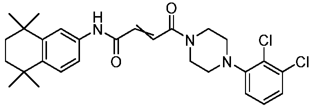 |
| **31** | 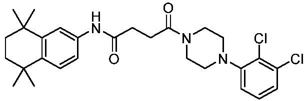 |
| **32** | 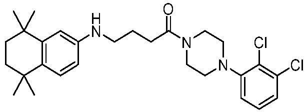 |
| **33** | 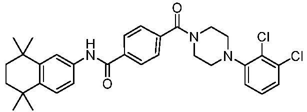 |
| **34** | 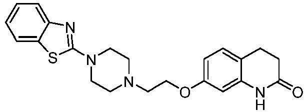 |
| **35** | 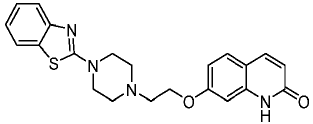 |
| **36** | 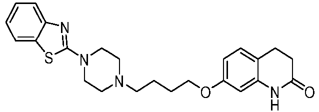 |
| **37** | 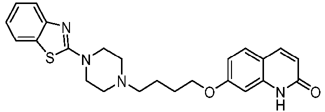 |
| **38** | 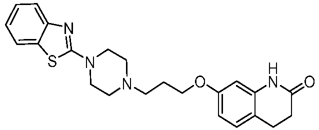 |
| **39** | 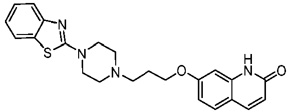 |
| **40** | 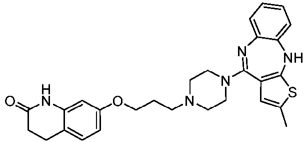 |
| **41** | 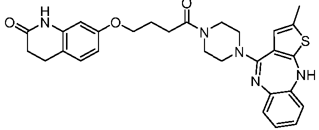 |
| **42** | 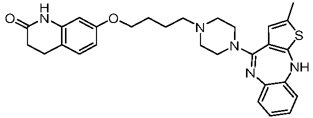 |
| **43** | 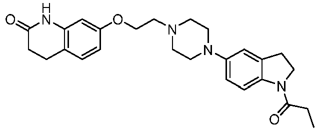 |
| **44** | 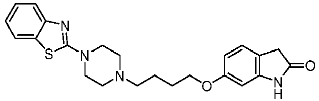 |
| **45** | 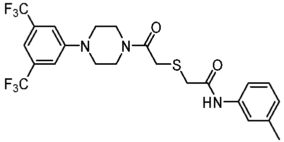 |
| **46** | 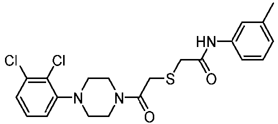 |
| **47** | 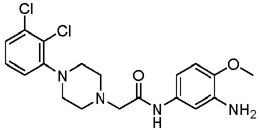 |
| **48** | 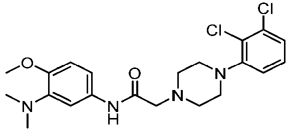 |
| **49** | 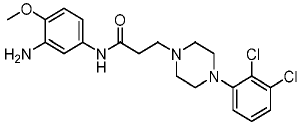 |
| **50** | 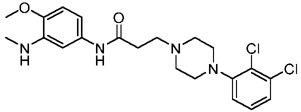 |
| **51** | 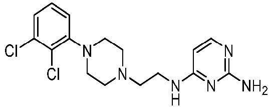 |
| **52** | 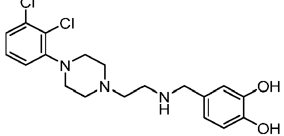 |
| **53** | 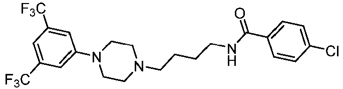 |
| **55** | 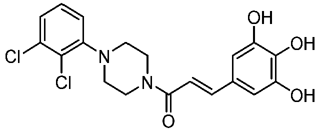 |
| **59** | 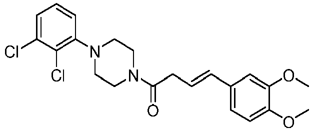 |
| **60** | 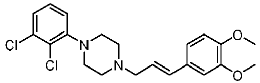 |
| **61** | 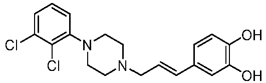 |
| **62** | 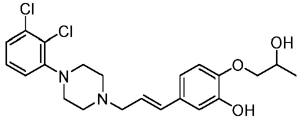 |
| **63** | 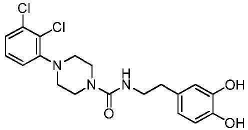 |
| **64** | 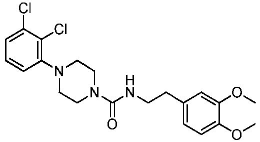 |
| **65** | 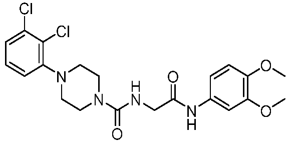 |
| **66** | 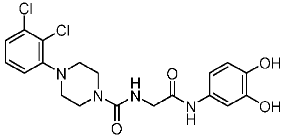 |
| **67** | 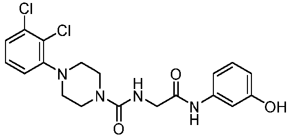 |
| **68** | 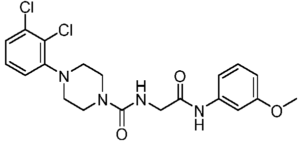 |
| **69** | 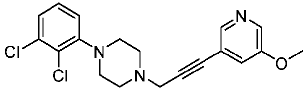 |
| **70** | 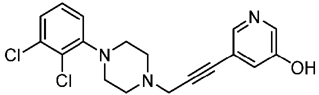 |
| **71** | 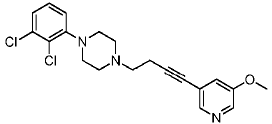 |
| **72** | 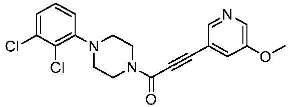 |
| **73** | 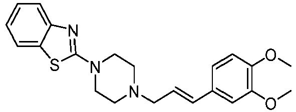 |
| **74** | 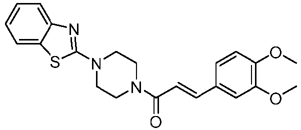 |
| **75** | 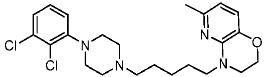 |
| **76** | 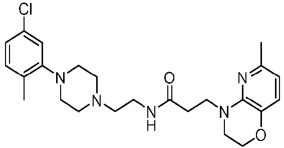 |
| **77** | 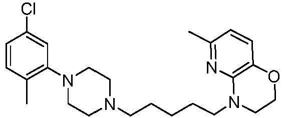 |
| **78** | 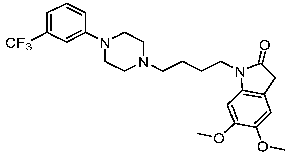 |
| **79** | 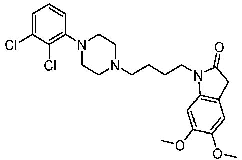 |
| **80** | 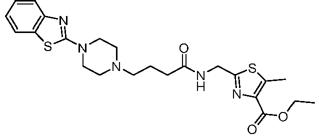 |
| **81** | 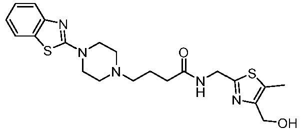 |
| **82** | 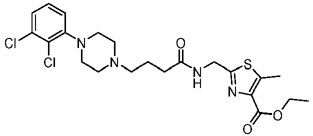 |
| **83** | 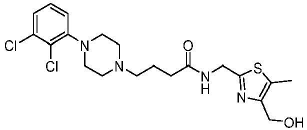 |
| **85** | 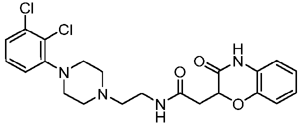 |
| **86** | 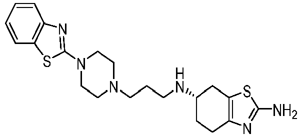 |
| **87** | 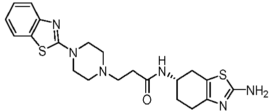 |
| **88** | 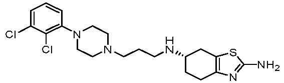 |
| **89** | 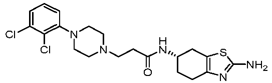 |
| **90** | 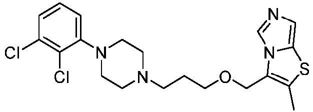 |
| **91** | 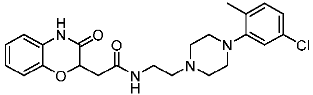 |
| **95** | 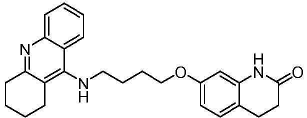 |
| **96** | 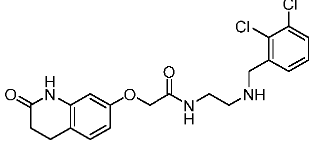 |
| **97** | 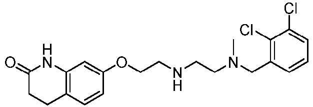 |
| **98** | 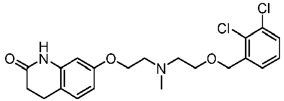 |
| **99** | 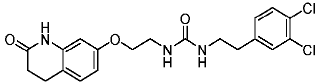 |
| **100** | 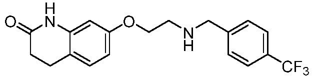 |
| **101** | 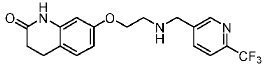 |
| **102** | 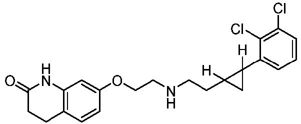 |
| **103** | 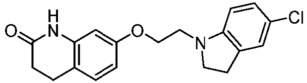 |
| **104** | 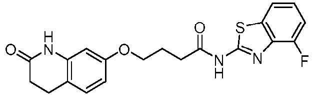 |
| **105** | 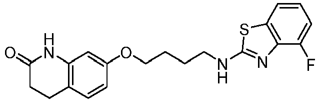 |
| **107** | 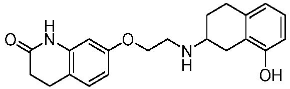 |
| **108** | 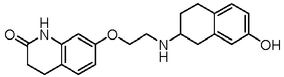 |
| **109** | 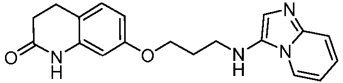 |
| **110** | 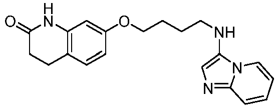 |
| **111** | 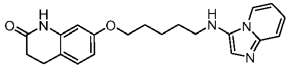 |
| **112** | 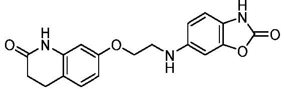 |
| **113** | 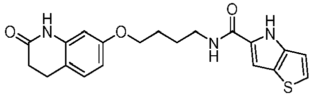 |
| **114** | 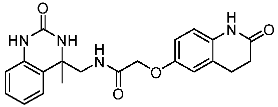 |
| **115** | 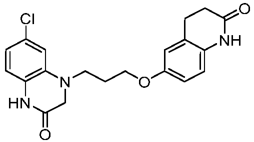 |
| **130** | 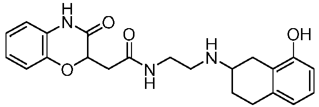 |
| **131** | 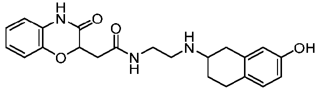 |
| **132** | 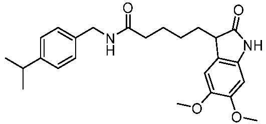 |
| **133** | 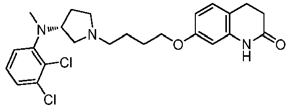 |
| **134** | 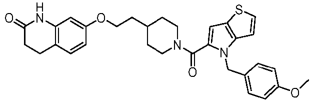 |
| **135** | 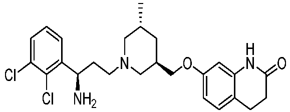 |
| **158** | 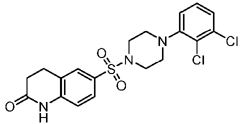 |
| **159** | 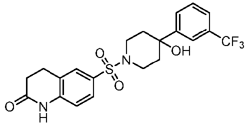 |
| **160** | 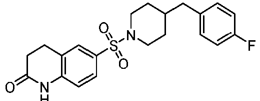 |
| **161** | 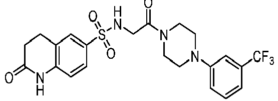 |
| **162** | 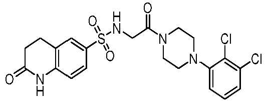 |
| **163** | 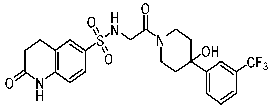 |
| **164** | 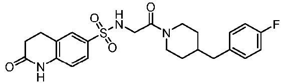 |
| **165** | 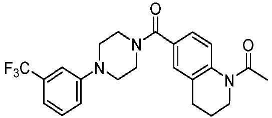 |
| **166** | 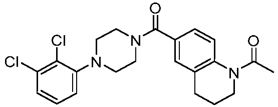 |
| **167** | 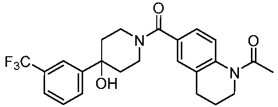 |
| **168** | 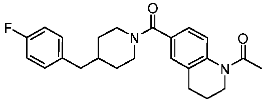 |
| **169** | 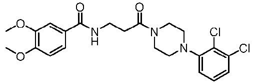 |
| **170** | 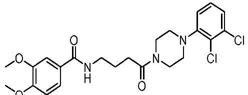 |
| **171** | 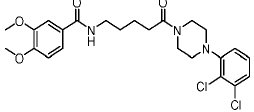 |
| **172** | 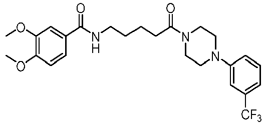 |
| **173** | 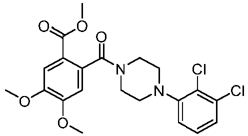 |
| **174** | 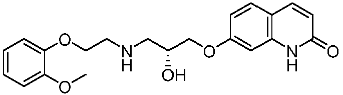 |
| **175** | 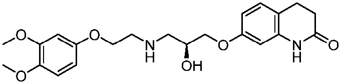 |
| **176** | 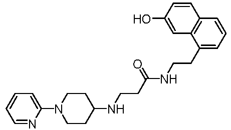 |
| **177** | 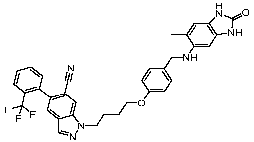 |
| **178** | 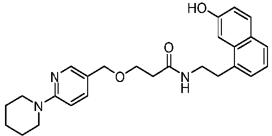 |

### Example

The present invention is further described below in conjunction with examples, but the provision of these examples is not intended to limit the scope of the present invention.

The abbreviations in the present invention have the following meanings:

| **Abbreviation** | **Meaning** |
|---|---|
| AcOH | Acetic Acid |
| BINAP | 2,2'-Bis(diphenylphosphino)-1,1'-binaphthyl |
| Boc | *tert*-Butoxycarbonyl |
| CDCl₃ | Deuterated chloroform |
| CD₃OD | Deuterated methanol |
| DCM | Dichloromethane |
| DIPEA/DIEA | *N,N*-Diisopropylethylamine |
| DMF | *N,N*-Dimethylformamide |
| DMF-DMA | N,N-Dimethylformamide dimethyl acetal |
| DMSO | Dimethyl sulfoxide |
| EA/EtOAc | Ethyl acetate |
| HATU | O-(7-Azabenzotriazol-1-yl)-N,N,N',N'-tetramethyluronium |
| | hexafluorophosphate |
| HNO₃ | Nitric acid |
| H₂O | Water |
| K₂CO₃ | Potassium carbonate |
| LC-MS | Liquid chromatography-mass spectrometry |
| LiAlH₄ | Lithium aluminum hydride |
| LiOH | Lithium hydroxide |
| MeCN | Acetonitrile |
| MeOH | Methanol |
| MsCl | Methanesulfonyl chloride |
| MTBE | Methyl *tert*-butyl ether |
| NaH | Sodium hydride |
| NMR | Nuclear magnetic resonance |
| Pd/C | Palladium on carbon |
| PdCl₂(PPh₃)₂ | Bis(triphenylphosphine)palladium(II) chloride |
| Pd₂(dba)₃ | Tris(dibenzylideneacetone)dipalladium(O) |
| PE | Petroleum ether |
| TBDMSCl | *tert*-Butyldimethylchlorosilane |
| TEA | Triethylamine |
| THF | Tetrahydrofuran |
| TLC | Thin-layer chromatography |
| Xphos | 2-Dicyclohexylphosphino-2',4',6'-triisopropylbiphenyl |

### Example 1: synthesis of 7-((4-(4-(2,3-dichlorophenyl)piperazin-1-yl)but-1-yn-1-yl)oxy)-3,4-dihydroquinolin-2(1H)-one (compound 1)

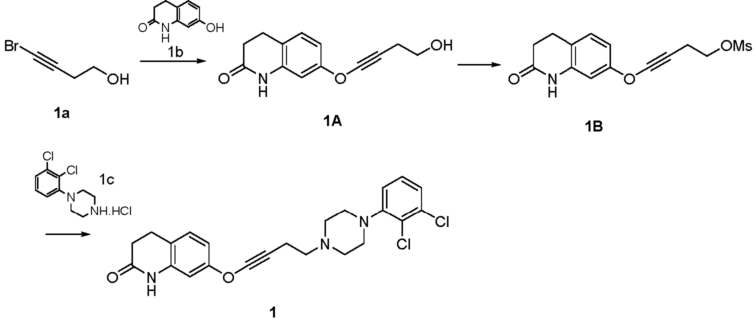

**Step 1: 1a** (5 g, 30.64 mmol) and K₂CO₃ (8.47 g, 61.28 mmol) were added to DMF (50 mL), followed by the addition of 3,4-dihydro-7-hydroxy-2(1H)-quinolinone (1b) (6.85 g, 45.96 mmol). The reaction solution was stirred at 100°C for 24 hours. After dilution with water, the mixture was extracted with ethyl acetate. The organic phase was dried, filtered, and concentrated by rotary evaporation to obtain a crude product, which was purified by normal-phase column chromatography (eluent: dichloromethane:methanol = 20:1). The target product was collected as a yellow liquid **1A** (6.2 g, yield: 79.9%).

**Step 2: 1A** (5.2 g, 22.49 mmol) and triethylamine (6.84 g, 67.47 mmol) were added to DCM (60 mL), and then MsCl (3.87 g, 33.7 mmol) was added dropwise at 0°C. The reaction solution was stirred at room temperature for 1 hour. The reaction solution was purified by normal-phase column chromatography (eluent: dichloromethane:ethyl acetate = 10:1). The target product was collected as a yellow solid **1B** (6.1 g, yield: 91.2%).

**Step 3: 1B** (6.1 g, 19.72 mmol) and K₂CO₃ (8.18 g, 59.16 mmol) were added to DMF (70 mL), followed by the addition of 1-(2,3-dichlorophenyl)piperazine hydrochloride (1c) (5.8 g, 21.69 mmol). The reaction solution was stirred at 50°C for 16 hours. After dilution with water, the mixture was extracted with ethyl acetate. The organic phase was dried, filtered, and concentrated by rotary evaporation to obtain a crude product, which was purified by normal-phase column chromatography (eluent: dichloromethane:methanol = 93:7) and then further purified by C-18 reversed-phase column chromatography (eluent: MeOH:H₂O (0.1% HCOOH) = 85:15). The target fraction was collected and lyophilized to obtain the title compound 1 (yellow solid) (12 mg, yield: 0.14%).

LCMS: 444 [M+H]⁺

¹H NMR (400 MHz, DMSO-*d*₆) δ 9.98 (s, 1H), 7.30 - 7.29 (m, 2H), 7.16 - 7.15 (m, 1H), 7.05 (d, 1H), 6.53 (d, 1H), 6.46 (s, 1H), 4.10 - 4.01 (m, 2H), 3.92 - 3.89 (m, 1H), 3.39 - 3.38 (m, 1H), 3.07 - 2.94 (m, 4H), 2.79 - 2.64 (m, 6H), 2.42 - 2.38 (m, 2H).

### Example 2: synthesis of 7-((6-(4-(2,3-dichlorophenyl)piperazin-1-yl)hexyl)oxy)-3,4-dihydroquinolin-2(1H)-one (compound 3)

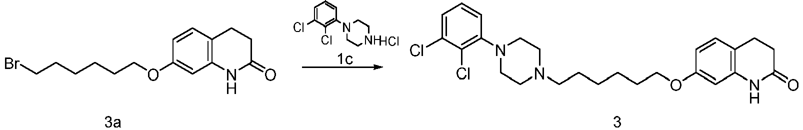

**3a** (0.32 g, 1.0 mmol, 1.0 eq.), 1-(2,3-dichlorophenyl)piperazine hydrochloride (1c) (0.27 g, 1.1 mmol, 1.1 eq.), K₂CO₃ (0.55 g, 4.0 mmol, 4.0 eq.), potassium iodide (0.33 g, 2.0 mmol, 2.0 eq.), and acetonitrile (15 mL) were added to a reaction flask, and the mixture was refluxed with heating for 4 hours. After filtration, the filter cake was washed with DMSO, and the organic phase was concentrated to obtain a crude product. The crude product was purified by C18 reversed-phase column chromatography (eluent: methanol:0.5% aqueous formic acid = 85:15), and the target fraction was collected and concentrated to dryness to obtain the title compound 3 (white solid) (135 mg, yield: 28.3%).

LCMS: 476 [M+H]⁺

¹H NMR (400 MHz, DMSO-*d*₆) δ 9.97 (s, 1H), 7.32-7.24 (m, 2H), 7.13 (dd, 1H), 7.02 (d, 1H), 6.47-6.41 (m, 2H), 3.87 (t, 2H), 2.96 (br, 4H), 2.76 (t, 2H), 2.41-2.32 (m, 4H), 1.72-1.63 (m, 2H), 1.46-1.32 (m, 6H). Note: four hydrogen signal peaks are overlapped by solvent signal peaks.

### Example 3: synthesis of 7-((5-(4-(2,3-dichlorophenyl)piperazin-1-yl)pentyl)oxy)-3,4-dihydroquinolin-2(1H)-one (compound 4)

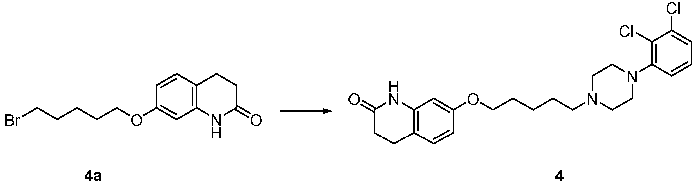

The title compound 4 (white solid) was prepared using the same synthetic route as in Example 2, except that **3a** in Example 2 was replaced with **4a.**

LCMS: 462 [M+H]⁺

¹H NMR (400 MHz, DMSO-*d*₆) δ 9.96 (s, 1H), 7.32-7.28 (m, 2H), 7.14 (dd, 1H), 7.03 (d, 1H), 6.48 (d, 1H), 6.42 (s, 1H), 3.88 (t, 2H), 2.97 (br, 4H), 2.76 (t, 2H), 2.51 (br, 2H), 2.41-2.34 (m, 6H), 1.75-1.66 (m, 2H), 1.54-1.38 (m, 4H).

### Example 4: synthesis of 7-(4-(4-(3-(trifluoromethyl)phenyl)piperazin-1-yl)butoxy)-3,4-dihydroquinolin-2(1H)-one (compound 6)

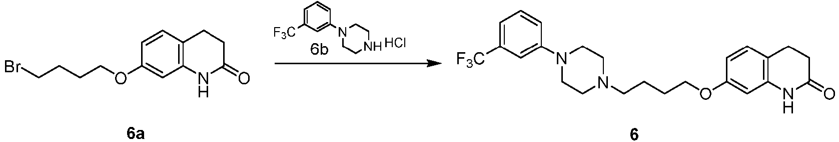

7-(4-Bromobutoxy)-3,4-dihydro-2(1H)-quinolinone (6a) (100 mg, 0.34 mmol, 1.0 eq.) was dissolved in acetonitrile (2 mL), potassium iodide (185 mg, 1.34 mmol, 4.0 eq.) was added, and the mixture was heated to 85°C for 1 hour. Then, 1-(3-trifluoromethylphenyl)piperazine hydrochloride (6b) (134 mg, 0.50 mmol, 1.5 eq.) and anhydrous potassium carbonate (111 mg, 0.67 mmol, 2.0 eq.) were added, and the reaction was continued at 85°C for 2 hours. Inorganic salts were filtered off, and the solid was washed with methanol (2×10 mL). The filtrate was concentrated under reduced pressure, and the residue was purified by 18C reversed-phase column chromatography (eluent: methanol:0.1% aqueous formic acid = 70:30). The target fraction was collected and concentrated. The residue was dissolved in methanol (0.5 mL), purified water (1 mL) was added, and methanol was removed under reduced pressure followed by lyophilization to obtain the title compound 6 (pale yellow flocculent solid, 80 mg, yield: 53%).

LCMS: 448 [M+H]⁺

¹H NMR (400 MHz, CD₃OD) δ 8.46 (s, 1H), 7.42 (t, 1H), 7.22 (m, 2H), 7.14 (d, 1H), 7.03 (d, 1H), 6.54 (d, 1H), 6.46 (s, 1H), 4.66 (t, 2H), 4.15 (br, 4H), 3.98 (br, 4H), 3.81 (t, 2H), 3.49 (t, 2H), 3.17 (t, 2H), 1.95 (br, 2H), 1.85-1.82 (m, 2H).

### Example 5: synthesis of 7-((5-(4-(3-(trifluoromethyl)phenyl)piperazin-1-yl)pentyl)oxy)-3,4-dihydroquinolin-2(1H)-one (compound 7)

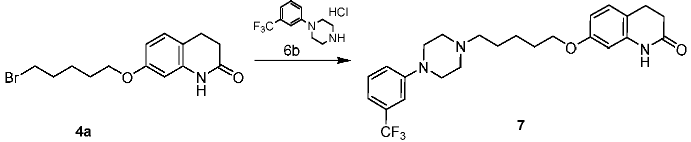

The title compound 7 (pale yellow solid) was prepared using the same synthetic route as in Example 4, except that 6a in Example 4 was replaced with 4a.

LCMS: 462 [M+H]⁺

¹H NMR (400 MHz, DMSO-*d*₆) δ 9.96 (s, 1H), 7.40 (t, 1H), 7.20 (d, 1H), 7.13 (s, 1H), 7.03 (t, 2H), 6.47 (dd, 1H), 6.41 (d, 1H), 3.88 (t, 2H), 3.21-3.18 (m, 4H), 2.76 (t, 2H), 2.43-2.36 (t, 2H), 2.33 (t, 2H), 1.74-1.66 (m, 2H), 1.49 (m, 2H), 1.45-1.35 (m, 2H). Note: four hydrogen signal peaks are overlapped by solvent signal peaks.

### Example 7: synthesis of 7-((5-(4-(2,3-dichlorophenyl)piperazin-1-yl)pentyl)oxy)quinolin-2(1H)-one (compound 9)

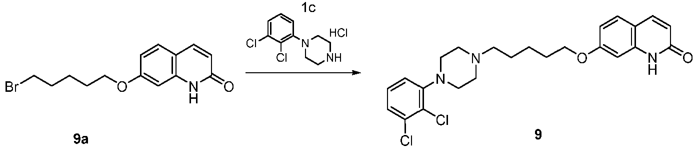

9a (100 mg, 0.32 mmol, 1.0 eq.) was dissolved in acetonitrile (5 mL), and 1-(2,3-dichlorophenyl)piperazine hydrochloride (1c) (129 mg, 0.48 mmol, 1.5 eq.), anhydrous potassium carbonate (178 mg, 1.29 mmol, 4.0 eq.), and potassium iodide (107 mg, 0.64 mmol, 2.0 eq.) were added. The mixture was heated to 85°C and reacted overnight. After filtration, the solid was washed with methanol (2×10 mL), and the filtrate was concentrated under reduced pressure. The residue was purified by 18C reversed-phase column chromatography (eluent: methanol:0.1% aqueous formic acid = 70:30). The target fraction was collected and concentrated, and the residue was purified with methanol (5 mL) to obtain the title compound 9 (white solid) (12 g, yield: 8%).

LCMS: 460 [M+H]⁺

¹H NMR (400 MHz, DMSO-*d*₆) δ 11.55 (s, 1H), 7.78 (d, 1H), 7.54 (d, 1H), 7.31-7.26 (m, 2H), 7.12 (dd, 1H), 6.78-6.77 (m, 2H), 6.28 (d, 1H), 4.00 (t, 2H), 2.96 (br, 4H), 2.35 (t, 2H), 1.80-1.72 (m, 2H), 1.56-1.38 (m, 4H). Note: four hydrogen signal peaks are overlapped by solvent signal peaks.

### Example 8: synthesis of 7-(4-(4-(2,3-dichlorophenyl)piperazin-1-yl)butoxy)quinolin-2(1H)-one (compound 10)

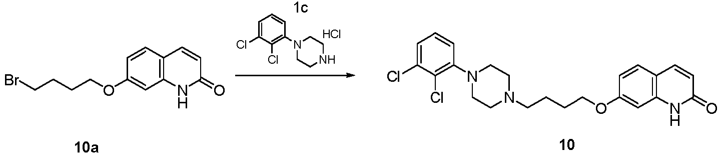

The title compound 10 (white solid) was prepared using the same synthetic route as in Example 7, except that 9a in Example 7 was replaced with 10a.

LCMS: 446 [M+H]⁺

¹H NMR (400 MHz, DMSO-*d*₆) δ 11.60 (s, 1H), 7.80 (d, 1H), 7.55 (d, 1H), 7.30 (s, 2H), 7.14 (s, 1H), 6.78 (s, 2H), 6.29 (d, 1H), 4.04 (t, 2H), 3.02 (br, 4H), 2.71 (br, 4H), 2.58 (br, 2H), 1.78-1.66 (m, 4H).

### Example 15: synthesis of 7-(3-(1-(2,3-dichlorophenyl)piperidin-4-yl)propoxy)-3,4-dihydroquinolin-2(1H)-one (compound 17)

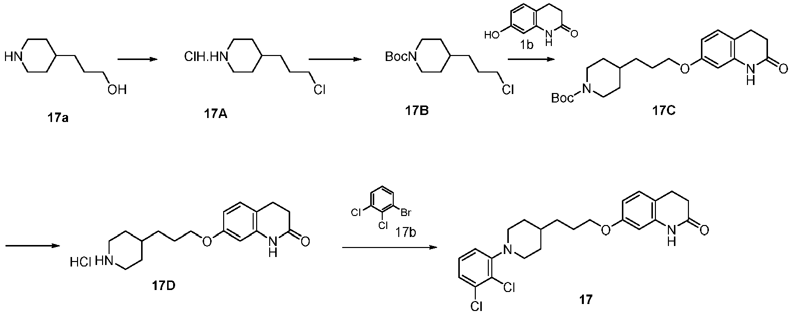

**Step 1: 17a** (500 mg, 3.49 mmol, 1.0 eq.) and DCM (80 mL) were added to a reaction flask, and thionyl chloride (630 mg, 5.25 mmol, 1.5 eq.) was added dropwise under an ice-water bath. The reaction solution was reacted at room temperature for 4 hours under nitrogen protection. LCMS monitoring showed complete consumption of the starting material, and concentration gave crude **17A** (550 mg, yield: 99%).

**Step 2: 17A** (550 mg, 3.49 mmol, 1.0 eq.), dioxane (10 mL), and triethylamine (1.05 mL, 10.5 mmol, 3.0 eq.) were added to a reaction flask, and Boc-anhydride (900 mg, 4.2 mmol, 1.2 eq.) was added dropwise to the reaction solution under an ice-water bath. After the addition, the reaction solution was reacted at room temperature for 4 hours. After LCMS monitoring showed almost complete consumption of the starting material, the mixture was concentrated under reduced pressure, and the residue was purified by column chromatography (petroleum ether:ethyl acetate = 5:1) to obtain **17B** (650 mg, yield: 80%).

**Step 3: 17B** (500 mg, 1.88 mmol, 1.0 eq.), acetonitrile (20 mL), potassium carbonate (380 mg, 2.74 mmol, 1.5 eq.), and 7-hydroxy-3,4-dihydroquinolin-2(1H)-one (**1b**) (360 mg, 2.32 mmol, 1.2 eq.) were added to a reaction flask. The reaction solution was reacted at 85°C for 4 hours. After LCMS monitoring showed complete consumption of the starting material, water (10 mL) was added, and the mixture was extracted with ethyl acetate. The organic phase was concentrated under reduced pressure, and the residue was purified by column chromatography (petroleum ether:ethyl acetate = 2:1) to obtain **17C** (600 mg, yield: 80%).

**Step 4: 17C** (600 mg, 1.32 mmol, 1.0 eq.) and ethyl acetate (10 mL) were added to a reaction flask, and a dioxane hydrochloride solution (4M, 10 mL) was added dropwise. The reaction solution was reacted at room temperature for 4 hours. After LCMS monitoring showed almost complete consumption of the starting material, the mixture was concentrated under reduced pressure, and the residue was purified with ethyl acetate, filtered, and the filter cake was dried to obtain **17D** (500 mg, yield: 90%).

**Step 5: 17D** (350 mg, 1.32 mmol, 1.0 eq.) and toluene (10 mL) were added to a reaction flask, followed by addition of BINAP (150 mg, 0.27 mmol, 0.2 eq.), potassium tert-butoxide (290 mg, 2.64 mmol, 2.0 eq.), and 1-bromo-2,3-dichlorobenzene (17b) (240 mg, 1.59 mmol, 1.2 eq.). Tris(dibenzylideneacetone)dipalladium (150 mg, 0.2 mmol, 0.15 eq.) was added to the reaction solution under nitrogen protection, and the solution was reacted at 100°C for 6 hours. After LCMS monitoring showed complete consumption of the starting material, the mixture was filtered, and the filtrate was concentrated under reduced pressure. The residue was purified by C18 reversed-phase preparative chromatography (eluent: methanol:0.1% aqueous formic acid = 70:30) to obtain a crude product, which was further separated by silica gel column to obtain the title compound 17 (30 mg, yield: 10%).

LCMS: 433 [M+H]⁺

¹H NMR (400 MHz, DMSO-*d*₆) δ 9.96 (s, 1H), 7.32-7.19 (m, 2H), 7.11 (d, 1H), 7.02 (d, 1H), 6.46 (d, 1H), 6.41 (s, 1H), 3.87 (t, 2H), 3.26-3.23 (m, 2H), 2.76 (t, 2H), 2.61 (t, 2H), 2.39 (t, 2H), 1.79-1.73 (m, 4H), 1.39-1.32 (m, 5H).

### Example 16: synthesis of 7-(4-(1-(2,3-dichlorophenyl)piperidin-4-yl)butoxy)-3,4-dihydroquinolin-2(1H)-one (compound 18)

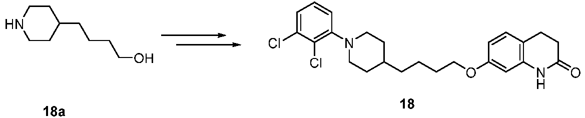

The title compound 18 was prepared using the same synthetic route as in Example 15, except that 17a in Example 15 was replaced with 18a.

LCMS: 447 [M+H]⁺

¹H NMR (400 MHz, CDCl₃) δ 7.67 (s, 1H), 7.16 (s, 2H), 7.06 (d, 1H), 6.54 (d, 1H), 6.31 (s, 1H), 3.94 (t, 2H), 3.38 (d, 2H), 2.90 (t, 2H), 2.62 (t, 2H), 1.80 (dd, 4H), 1.52 (s, 2H), 1.46-1.33 (m, 3H), 1.33-1.17 (m, 4H).

### Example 17: synthesis of 7-(4-(4-phenylpiperidin-1-yl)butoxy)-3,4-dihydroquinolin-2(1H)-one (compound 20)

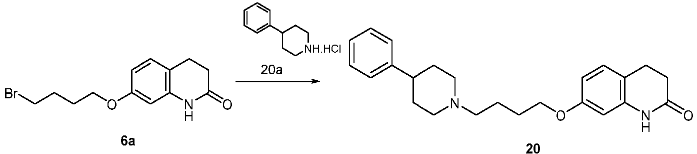

20a (500 mg, 1.65 mmol, 1.0 eq.), 7-(4-bromobutoxy)-3,4-dihydroquinolin-2(1H)-one (6a) (730 mg, 2.48 mmol, 1.5 eq.), potassium carbonate (700 mg, 4.95 mmol, 3.0 eq.), and MeCN (20 mL) were added to a reaction flask. The reaction solution was purged with nitrogen three times and reacted at 85°C for 12 hours. After TLC and LCMS monitoring showed complete consumption of the starting material, filtration gave a crude product, which was purified by C18 reversed-phase column chromatography. The target fraction was collected and concentrated to obtain the title compound 20 (white solid) (500 mg, yield: 70%).

LCMS: 379 [M+H]⁺

¹H NMR (400 MHz, CD₃OD) δ 8.46 (s, 1H), 7.34-7.21 (m, 5H), 7.07 (d, 1H), 6.56 (d, 1H), 6.47 (s, 1H), 4.02 (t, 2H), 3.69-3.66 (m, 2H), 3.23 (t, 2H), 3.10 (t, 2H), 2.88-2.86 (m, 3H), 2.53 (t, 2H), 2.11-1.94 (m, 6H), 1.90-1.85 (m, 2H).

### Example 18: synthesis of N-(4-(2,3-dichlorophenyl)cyclohexyl)-2-((2-oxo-1,2,3,4-tetrahydroquinolin-7-yl)oxy)acetamide (compound 21)

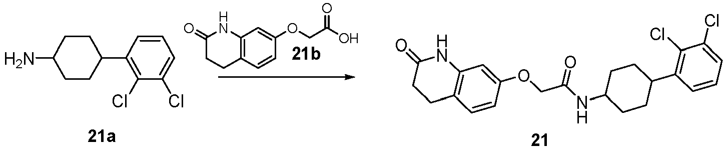

**21a** (550 mg, 2.25 mmol), **21b** (748 mg, 3.38 mmol), and DIPEA (874 mg, 6.76 mmol) were added to DMF (6 mL), followed by addition of HATU (1.28 g, 3.38 mmol). The reaction solution was stirred at room temperature overnight. After dilution with water, the mixture was extracted with ethyl acetate. The organic phase was dried, filtered, and concentrated, and the residue was purified by C18 reversed-phase column chromatography (eluent: methanol:0.1% aqueous formic acid = 70:30). The target product was collected and lyophilized to obtain the title compound 21 (white solid) (0.065 g, yield: 6.5%).

LCMS: 447 [M+H]⁺;

¹H NMR (400 MHz, DMSO-*d*₆) δ 10.1 (s, 1H), 7.95 (d, 1H), 7.47 (d, 1H), 7.39 (d, 1H), 7.35- 7.31 (m, 1H), 7.05 (d, 1H), 6.49 - 6.47 (m, 2H), 4.37 (s, 2H), 3.77 - 3.69 (m, 1H), 2.98 - 2.91 (m, 1H), 2.80 - 2.76 (m, 2H), 2.42 - 2.39 (m, 2H), 1.89 - 1.78 (m, 4H), 1.56 - 1.43 (m, 4H).

### Example 19: synthesis of 7-((5-(4-(2,3-dichlorophenyl)piperazin-1-yl)pentyl)oxy)-3,4-dihydroisoquinolin-1(ZH)-one (compound 23)

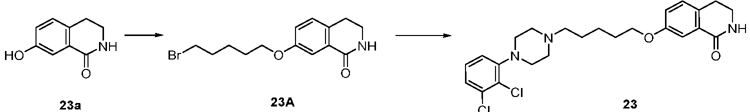

**Step 1:** 7-Hydroxy-3,4-dihydroisoquinolin-1(2H)-one (23a) (0.326 g, 2.0 mmol, 1.0 eq.), 1,5-dibromopentane (0.92 g, 4.0 mmol, 2.0 eq.), potassium carbonate (0.55 g, 2.0 mmol, 2.0 eq.), and DMF (8 mL) were added to a reaction flask, and the mixture was stirred at 85°C for 7 hours. After filtration, the filter cake was washed with methanol, and the filtrate was concentrated to obtain a crude product. The crude product was purified by C18 reversed-phase column chromatography (eluent: methanol:0.5% aqueous formic acid = 80:20), and the target fraction was collected and concentrated to obtain a pale yellow solid **23A** (540 mg, purity 60%, yield: 58.3%).

**Step 2:** The title compound 23 (off-white solid) was prepared using the same synthetic route as in Example 2, except that 3a in Example 2 was replaced with 23A.

LCMS: 462 [M+H]⁺

¹H NMR (400 MHz, DMSO-*d*₆) δ 7.92 (s, 1H), 7.33 (s, 1H), 7.31-7.28 (m, 2H), 7.19 (d, 1H), 7.16 (dd, 1H), 7.05 (dd, 1H), 3.98 (t, 2H), 3.33-3.31 (m, 2H), 2.99 (br, 4H), 2.80 (t, 2H), 2.63 (br, 4H), 2.44 (t, 2H), 1.77-1.67 (m, 2H), 1.55-1.51 (m, 2H), 1.46-1.42 (m, 2H).

### Example 20: synthesis of 6-(4-(4-(2,3-dichlorophenyl)piperazin-1-yl)butoxy)indolin-2-one (compound 24)

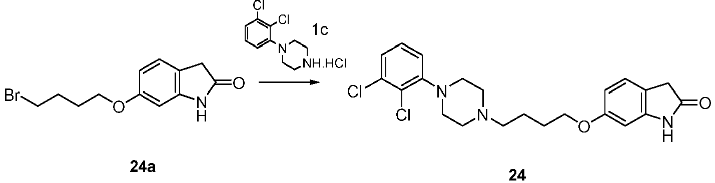

**24a** (100 mg, 0.37 mmol, 1.0 eq.) was dissolved in acetonitrile (6 mL), and 1-(2,3-dichlorophenyl)piperazine hydrochloride (1c) (130 mg, 0.45 mmol, 1.2 eq.), potassium carbonate (150 mg, 1.11 mmol, 3.0 eq.), and potassium iodide (10 mg, 0.0037 mmol, 0.1 eq.) were added. The reaction solution was reacted at 70°C for 4 hours. After LCMS monitoring showed almost complete consumption of the starting material, the reaction solution was filtered, concentrated, and purified by C18 reversed-phase column chromatography to obtain the title compound 24 (off-white solid, 20 mg, yield: 12%).

LCMS: 434 [M+H]⁺

¹H NMR (400 MHz, CDCl₃) δ 7.79 (s, 1H), 7.24-7.13 (m, 2H), 7.14-7.06 (m, 1H), 6.97 (d, 1H), 6.53 (d, 1H), 6.46 (s, 1H), 3.98 (t, 2H), 3.47 (s, 2H), 3.15 (s, 4H), 2.76 (s, 3H), 2.61 (d, 2H), 1.97-1.64 (m, 5H).

### Example 21: synthesis of 6-(4-(4-(3-(trifluoromethyl)phenyl)piperazin-1-yl)butoxy)indolin-2-one (compound 25)

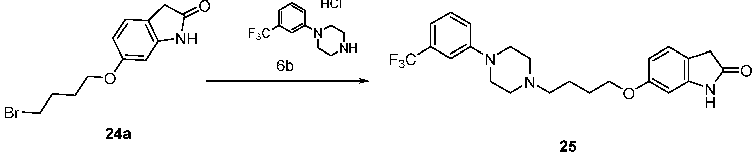

**24a** (100 mg, 0.37 mmol, 1.0 eq.) was dissolved in acetonitrile (6 mL), and 3-(trifluoromethyl)phenylpiperazine hydrochloride (6b) (130 mg, 0.45 mmol, 1.2 eq.), potassium carbonate (150 mg, 1.11 mmol, 3.0 eq.), and potassium iodide (10 mg, 0.0037 mmol, 0.1 eq.) were added. The reaction solution was reacted at 70°C for 4 hours. After LCMS monitoring showed almost complete consumption of the starting material, the reaction solution was filtered, and the filtrate was concentrated and purified by normal-phase column chromatography (eluent: DCM:MeOH = 10:1) to obtain the title compound **25** (off-white solid, 80 mg, yield: 50%).

LCMS: 434 [M+H]⁺

¹H NMR (400 MHz, CDCl₃) δ 8.10 (s, 1H), 7.34 (t, 1H), 7.12-7.06 (m, 4H), 6.54 (d, 1H), 6.47 (s, 1H), 3.98 (t, 2H), 3.48 (s, 2H), 3.28 (s, 4H), 2.67 (s, 4H), 2.52 (s, 2H), 1.96-1.80 (m, 2H), 1.79-1.65 (m, 2H).

### Example 22: synthesis of 6-((5-(4-(2,3-dichlorophenyl)piperazin-1-yl)pentyl)oxy)-2H-benzo[b][1,4]oxazin-3(4H)-one (compound 27)

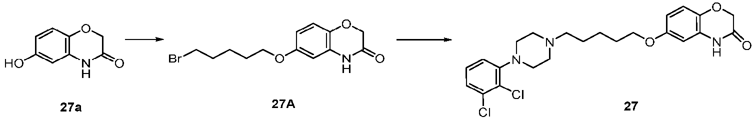

**Step 1:** 27a (0.33 g, 2.0 mmol, 1.0 eq.), 1,4-dibromobutane (0.69 g, 3.0 mmol, 1.5 eq.), potassium carbonate (0.55 g, 2.0 mmol, 2.0 eq.), DMF (10 mL), and H₂O (2 mL) were added to a reaction flask and stirred at 40°C for 5 hours. The reaction solution was filtered, the filter cake was washed with DMSO, and the filtrate was concentrated to obtain a crude product, which was purified by C18 reversed-phase column chromatography (eluent: methanol:0.5% aqueous formic acid = 80:20). The target fraction was collected and concentrated to obtain a white solid 27A (120 mg, yield: 19%).

**Step 2:** The title compound 27 (white solid) was prepared using the same synthetic route as in Example 2, except that 3a in Example 2 was replaced with 27A.

LCMS: 464 [M+H]⁺

¹H NMR (400 MHz, DMSO-*d*₆) δ 10.62 (s, 1H), 7.31 (d, 2H), 7.16 (t, 1H), 6.84 (d, 1H), 6.48-6.46 (m, 2H), 4.47 (s, 2H), 3.87 (t, 2H), 3.07 (br, 4H), 2.85 (br, 4H), 2.67 (br, 2H), 1.72-1.67 (m, 2H), 1.59-1.57 (m, 2H), 1.45-1.40 (m, 2H).

### Example 23: synthesis of 6-(3-(4-(2,3-dichlorophenyl)piperazin-1-yl)propoxy)benzo[d]thiazole (compound 28)

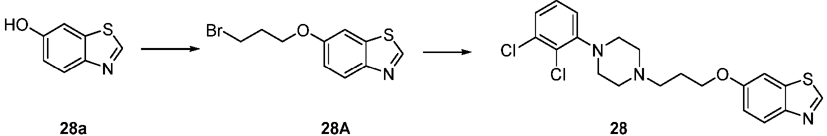

**Step 1:** 28a (200 mg, 1.32 mmol, 1.0 eq.) and N,N-dimethylformamide (4 mL)/water (0.4 mL) were added to a reaction flask, followed by addition of potassium carbonate (365 mg, 2.64 mmol, 2.0 eq.) and 1,3-dibromopropane (800 mg, 3.96 mmol, 3.0 eq.). The reaction solution was reacted at 45°C for 3 hours. After LCMS monitoring showed almost complete consumption of the starting material, water (10 mL) was added, and the mixture was extracted with ethyl acetate, dried, concentrated, and purified by silica gel column chromatography (petroleum ether:ethyl acetate = 1:1) to obtain 28A (250 mg, yield: 80%).

**Step 2:** 28A (350 mg, 1.32 mmol, 1.0 eq.) and N,N-dimethylformamide (10 mL) were added to a reaction flask, followed by addition of potassium carbonate (710 mg, 5.28 mmol, 4.0 eq.), potassium iodide (200 mg, 1.32 mmol, 1.0 eq.), and 1-(2,3-dichlorophenyl)piperazine hydrochloride (1c) (420 mg, 1.59 mmol, 1.2 eq.). The reaction solution was reacted at 85°C for 4 hours. After LCMS monitoring showed almost complete consumption of the starting material, the mixture was filtered, and the mother liquor was concentrated to obtain a crude product, which was purified by C18 reversed-phase preparative chromatography (eluent: methanol:0.1% aqueous formic acid = 70:30) to obtain the title compound 28 (150 mg, yield: 40%).

LCMS: 422 [M+H]⁺

¹H NMR (400 MHz, DMSO-*d*₆) δ 9.17 (s, 1H), 7.95 (d, 1H), 7.72 (d, 1H), 7.29-7.28 (m, 2H), 7.13-7.11 (m, 2H), 4.10 (t, 2H), 2.99 (br, 4H), 2.62-2.49 (m, 6H), 1.97-1.94 (m, 2H).

### Example 24: synthesis of 6-(4-(4-(2,3-dichlorophenyl)piperazin-1-yl)butoxy)benzo[d]thiazole (compound 29)

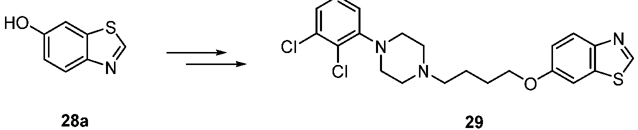

The title compound 29 was prepared using the same synthetic route as in Example 23, except that 1,3-dibromopropane in Step 1 of Example 23 was replaced with 1,4-dibromobutane, and the equivalent of potassium iodide in Step 2 was changed from 1.0 eq. to 0.1 eq.

LCMS: 436 [M+H]⁺

¹H NMR (400 MHz, DMSO-*d*₆) δ 9.17 (s, 1H), 7.94 (d, 1H), 7.71 (d, 1H), 7.29-7.28 (m, 2H), 7.14-7.10 (m, 2H), 4.07 (t, 2H), 2.98 (br, 4H), 2.61 (br, 4H), 1.82-1.75 (m, 2H), 1.67-1.62 (m, 2H). *Note: two hydrogen signal peaks are overlapped by solvent signal peaks.*

### Example 25: synthesis of 4-(4-(2,3-dichlorophenyl)piperazin-1-yl)-4-oxo-N-(5,5,8,8-tetramethyl-5,6,7,8-tetrahydronaphthalen-2 -yl)but-2-enamide (compound 30)

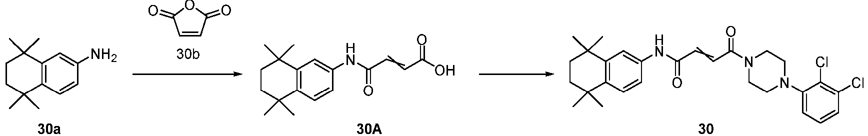

**Step 1: 30a** (500 mg, 2.46 mmol, 1.0 eq.) was added to toluene (5 mL), and then **30b** (242 mg, 2.46 mmol, 1.0 eq.) was added. The mixture was stirred at 25°C for 16 hours. The reaction solution was filtered, and the filter cake was dried to obtain a yellow solid **30A** (560 mg, yield: 75.5%).

**Step 2:** The title compound 30 (white solid) was prepared using the same synthetic route as in Example 18, except that 21a in Example 18 was replaced with 1-(2,3-dichlorophenyl)piperazine hydrochloride (1c), and 21b was replaced with 30A.

LCMS: 514 [M+H]⁺

H NMR (400 MHz, DMSO-*d*₆) δ 10.1 (s, 1H), 7.47-7.40 (m, 2H), 7.30-7.22 (m, 3H), 7.10-7.08 (m, 1H), 6.52 (d, 1H), 6.23 (d, 1H), 3.69-3.62 (m, 2H), 3.51-3.44 (m, 2H), 2.99-2.94 (m, 4H), 1.66-1.55 (m, 4H), 1.19 (s, 12H).

### Example 26: synthesis of 4-(4-(2,3-dichlorophenyl)piperazin-1-yl)-4-oxo-N-(5,5,8,8-tetramethyl-5,6,7,8-tetrahydronaphthalen-2 -yl)butanamide (compound 31)

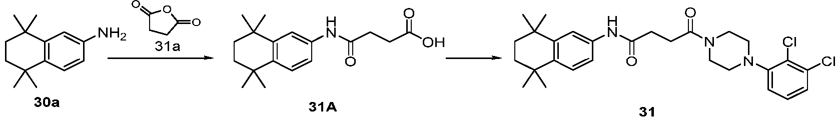

**Step 1: 30a** (500 mg, 2.46 mmol, 1.0 eq.) and triethylamine (636 mg, 4.92 mmol, 2.0 eq.) were added to DCM (10 mL), and then **31a** (271 mg, 2.71 mmol, 1.1 eq.) was added. The mixture was stirred at 25°C for 16 hours. The reaction solution was concentrated to obtain a yellow solid **31A** (740 mg, yield: 99.1%).

**Step 2:** The title compound 31 (white solid) was prepared using the same synthetic route as in Example 18, except that 21a in Example 18 was replaced with 1-(2,3-dichlorophenyl)piperazine hydrochloride (1c), and 21b was replaced with 31A.

LCMS: 518 [M+H]⁺

H NMR (400 MHz, DMSO-*d*₆) δ 9.81 (s, 1H), 7.56-7.54 (m, 1H), 7.39-7.33 (m, 3H), 7.25-7.15 (m, 2H), 3.68-3.64 (m, 4H), 3.03-2.96 (m, 4H), 2.72-2.68 (m, 2H), 2.60-2.57 (m, 2H), 1.68-1.62 (m, 4H), 1.24 (s, 12H).

### Example 27: synthesis of 1-(4-(2,3-dichlorophenyl)piperazin-1-yl)-4-((5,5,8,8-tetramethyl-5,6,7,8-tetrahydronaphthalen-2-yl)a mino)butan-1-one (compound 32)

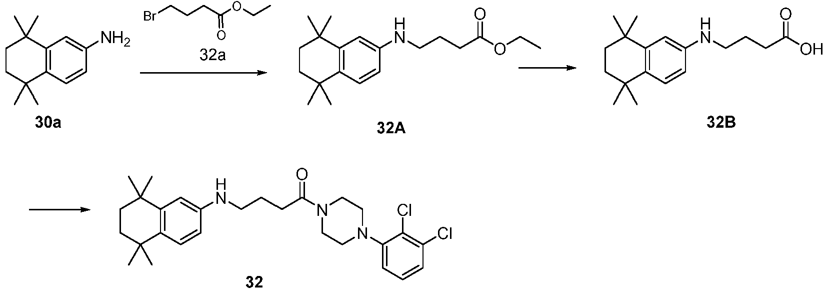

**Step 1: 30a** (1 g, 4.92 mmol, 1.0 eq.) and sodium carbonate (1.56 g, 14.75 mmol, 3.0 eq.) were added to acetonitrile (20 mL), and then **32a** (1.25 g, 6.39 mmol, 1.3 eq.) was added. The mixture was stirred at 85°C for 16 hours. The reaction solution was concentrated to obtain a crude product, which was purified by normal-phase column chromatography (eluent: petroleum ether:ethyl acetate = 9:1) to collect the target product as a yellow oil **32A** (350 mg, yield: 22.4%).

**Step 2: 32A** (350 mg, 1.10 mmol, 1.0 eq.) was dissolved in THF/MeOH/H₂O (6 mL/2 mL/2 mL), and then LiOH (66 mg, 2.76 mmol, 2.5 eq.) was added. The mixture was stirred at 25°C for 2 hours. The reaction solution was diluted with water, adjusted to pH 5-6 with 1M aqueous hydrochloric acid, extracted with ethyl acetate. The organic phase was dried, filtered, and concentrated to obtain a yellow solid **32B** (300 mg, yield: 94.0%).

**Step 3: 32B** (300 mg, 1.04 mmol, 1.0 eq.), 1-(2,3-dichlorophenyl)piperazine hydrochloride **(1c)** (416 mg, 1.55 mmol, 1.5 eq.), and DIPEA (402 mg, 3.11 mmol, 3.0 eq.) were added to DMF (5 mL), followed by addition of HATU (590 mg, 1.55 mmol, 1.5 eq.). The mixture was stirred at 25°C for 16 hours and purified by C-18 reversed-phase column chromatography (eluent: methanol:0.1% aqueous formic acid = 85:15). The target fraction was collected and lyophilized to obtain the title compound **32** (white solid, 94 mg, yield: 18.0%).

LCMS: 502 [M+H]⁺

¹H NMR (400 MHz, DMSO-*d*₆) δ 7.31-7.27 (m, 2H), 7.10-7.09 (m, 1H), 6.98-6.96 (m, 1H), 6.45 (s, 1H), 6.36-6.34 (m, 1H), 5.28-5.22 (m, 1H), 3.60-3.57 (m, 4H), 3.00-2.97 (m, 2H), 2.95-2.84 (m, 4H), 2.45-2.41 (m, 2H), 1.78-1.72 (m, 2H), 1.57-1.52 (m, 4H), 1.17 (s, 6H), 1.13 (s, 6H).

### Example 28: synthesis of 4-(4-(2,3-dichlorophenyl)piperazine-1-carbonyl)-N-(5,5,8,8-tetramethyl-5,6,7,8-tetrahydronaphthalen -2-yl)benzamide (compound 33)

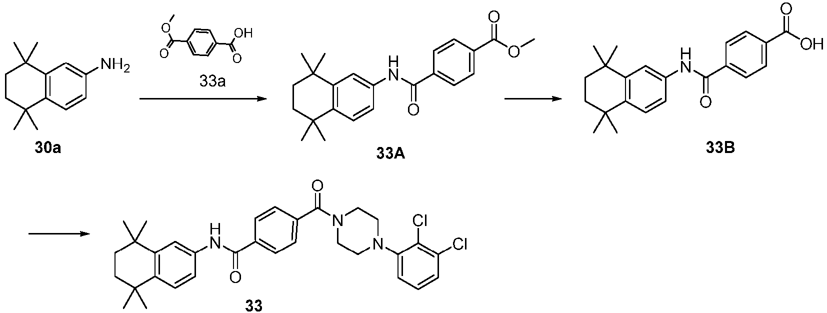

**Step 1:** Compound **33A** (yellow solid) was prepared using the same synthetic route as in Example 18, except **that** 21a in Example 18 was replaced with 30a and 21b was replaced with 33a.

**Step 2:** Compound **33B** was prepared using the same synthetic route as in Step 2 of Example 27, except that **32A** in Example 27 was replaced with **33A.**

**Step 3:** The title compound 33 (white solid) was prepared using the same synthetic route as in Example 18, except that 21a in Example 18 was replaced with 1c and 21b was replaced with 33B.

LCMS: 564 [M+H]⁺

¹H NMR (400 MHz, DMSO-*d*₆) δ 10.16 (s, 1H), 8.02-7.80 (m, 2H), 7.70-7.65 (m, 1H), 7.59-7.57 (m, 3H), 7.33-7.26 (m, 3H), 7.18-7.15 (m, 1H), 3.90-3.72 (m, 2H), 3.55-3.41 (m, 2H), 3.06-2.97 (m, 4H), 1.68-1.58 (m, 4H), 1.23 (s, 6H), 1.22 (s, 6H).

### Example 29: synthesis of 7-(2-(4-(benzo[d]thiazol-2-yl)piperazin-1-yl)ethoxy)-3,4-dihydroquinolin-2(1H)-one (compound 34)

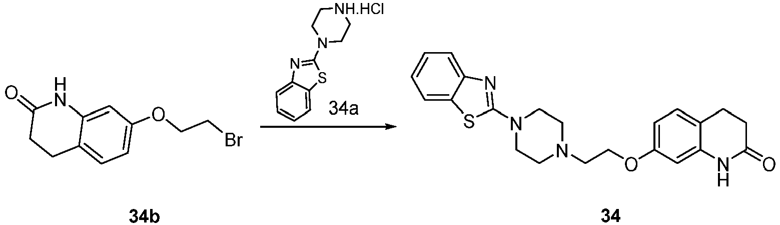

The title compound 34 was prepared using the same synthetic route as in Step 2 of Example 23, except that 28A in Step 2 of Example 23 was replaced with 34b and 1c was replaced with 34a.

LCMS: 408 [M+H]⁺

¹H NMR (400 MHz, DMSO-*d*₆) δ 9.99 (s, 1H), 7.75 (d, 1H), 7.44 (d, 1H), 7.26 (t, 1H), 7.06-7.03 (m, 2H), 6.50 (d, 1H), 6.44 (s, 1H), 4.03 (t, 2H), 3.56-3.53 (m, 4H), 2.81-2.73 (m, 4H), 2.62-2.58 (m, 4H), 2.40 (t, 2H).

### Example 30: synthesis of 7-(2-(4-(benzothiazol-2-yl)piperazin-1-yl)ethoxy)quinolin-2(1H)-one (compound 35)

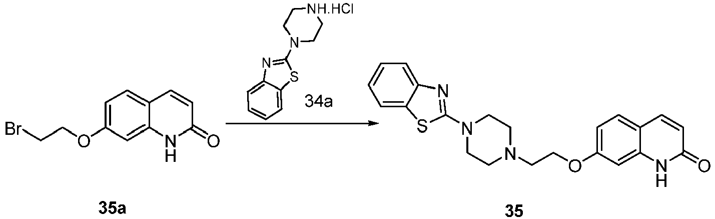

The title compound 35 was prepared using the same synthetic route as in Step 2 of Example 23, except that 28A in Step 2 of Example 23 was replaced with 35a and 1c was replaced with 34a.

LCMS: 407 [M+H]⁺

¹H NMR (400 MHz, DMSO-*d*₆) δ 11.59 (s, 1H), 7.81-7.74 (m, 2H), 7.55 (d, 1H), 7.44 (d, 1H), 7.26 (t, 1H), 7.06 (t, 1H), 6.82-6.80 (m, 2H), 6.30 (d, 1H), 4.15 (t, 2H), 3.57-3.55 (m, 4H), 2.80 (d, 2H), 2.65-2.63 (m, 4H).

### Example 31: synthesis of 7-(4-(4-(benzo[d]thiazol-2-yl)piperazin-1-yl)butoxy)-3,4-dihydroquinolin-2(1H)-one (compound 36)

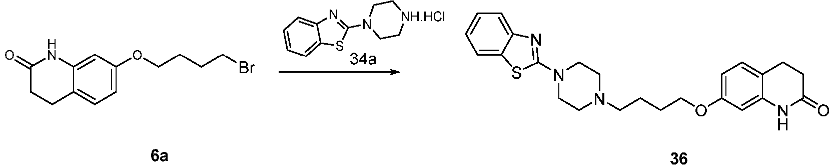

34a (100 mg, 0.39 mmol, 1.0 eq.), acetonitrile (4 mL), potassium carbonate (165.6 mg, 1.2 mmol, 3.0 eq.), potassium iodide (10 mg, 0.039 mmol, 0.1 eq.), and 7-(4-bromobutoxy)-3,4-dihydro-2(1H)-quinolinone (6a) (175 mg, 0.59 mmol, 1.5 eq.) were added to a reaction flask. The reaction solution was purged with nitrogen three times and reacted at 85°C for 4 hours. After TLC monitoring showed complete consumption of the starting material, the reaction solution was filtered and concentrated, and the crude product was purified by C18 reversed-phase column chromatography. The target fraction was collected and concentrated to obtain the title compound 36 (white solid) (20 mg, yield: 10%).

LCMS: 437 [M+H]⁺

¹H NMR (400 MHz, DMSO-*d*₆) δ 9.97 (s, 1H), 7.75 (d, 1H), 7.44 (d, 1H), 7.26 (t, 1H), 7.06-7.02 (m, 2H), 6.47 (d, 1H), 6.42 (s, 1H), 3.91 (t, 2H), 3.54 (br, 4H), 2.76 (t, 2H), 2.46-2.34 (m, 4H), 1.79-1.65 (m, 2H), 1.64-1.52 (m, 2H). Note: four hydrogen signal peaks are overlapped by solvent signal peaks.

### Example 32: synthesis of 7-(4-(4-(benzo[d]thiazol-2-yl)piperazin-1-yl)butoxy)quinolin-2(1H)-one (compound 37)

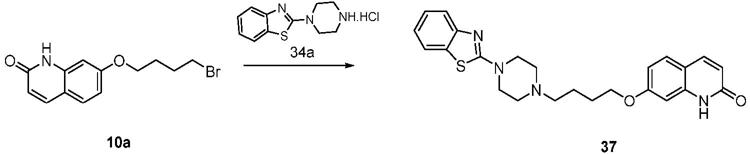

The title compound 37 (white solid) was prepared using the same synthetic route as in Example 31, except that 6a in Example 31 was replaced with 10a.

LCMS: 435 [M+H]⁺

¹H NMR (400 MHz, DMSO-*d*₆) δ 11.57 (s, 1H), 7.80 (d, 1H), 7.76 (d, 1H), 7.54 (d, 1H), 7.44 (d, 1H), 7.26 (t, 1H), 7.06 (t, 1H), 6.79-6.78 (m, 2H), 6.28 (d, 1H), 4.03 (t, 2H), 3.55 (t, 4H), 2.39 (t, 2H), 1.79-1.74 (m, 2H), 1.65-1.58 (m, 2H). Note: four hydrogen signal peaks are overlapped by solvent signal peaks.

### Example 33: synthesis of 7-(3-(4-(benzo[d]thiazol-2-yl)piperazin-1-yl)propoxy)-3,4-dihydroquinolin-2(1H)-one (compound 38)

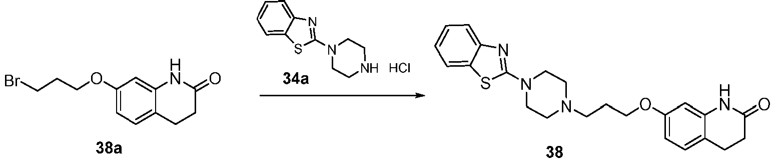

**38a** (0.14 g, 0.5 mmol, 1.0 eq.), **34a** (0.15 g, 0.6 mmol, 1.2 eq.), potassium carbonate (0.13 g, 1.0 mmol, 2.0 eq.), and acetonitrile (15 mL) were added to a reaction flask and stirred at 85°C for 6 hours. The mixture was purified by silica gel column chromatography (eluent: dichloromethane:methanol = 85:15), and the target fraction was collected and concentrated to obtain the title compound 38 (white solid) (50 mg, yield: 23.7%).

LCMS: 423 [M+H]⁺

¹H NMR (400 MHz, DMSO-*d*₆) δ 9.96 (s, 1H), 7.75 (d, 1H), 7.45 (d, 1H), 7.27 (t, 1H), 7.08-7.03 (m, 2H), 6.48 (d, 1H), 6.44 (s, 1H), 3.95 (t, 2H), 3.55 (br, 4H), 2.76 (t, 2H), 2.53-2.45 (m, 6H), 2.41 (t, 2H), 1.93- 1.84 (m, 2H).

### Example 34: synthesis of 7-(3-(4-(benzo[d]thiazol-2-yl)piperazin-1-yl)propoxy)quinolin-2(1H)-one (compound 39)

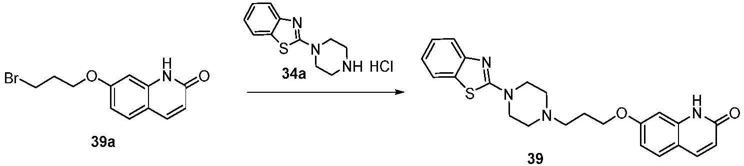

The title compound 39 (white solid) was prepared using the same synthetic route as in Example 33, except that 38a in Example 33 was replaced with 39a.

LCMS: 421 [M+H]⁺

¹H NMR (400 MHz, DMSO-*d*₆) δ 11.83 (s, 1H), 7.88-7.82 (m, 2H), 7.58 (t, 2H), 7.36 (t, 1H), 7.17 (t, 1H), 6.89- 6.80 (m, 2H), 6.33 (d, 1H), 4.24-4.20 (m, 2H), 4.14 (t, 2H),3.79 (t, 2H), 3.68-3.65 (m, 2H), 3.38-3.16 (m, 4H), 2.36-2.26 (m, 2H).

### Example 35: synthesis of 7-(3-(4-(2-methyl-10H-benzo[b]thieno[2,3-e][1,4]diazepin-4-yl)piperazin-1-yl)propoxy)-3,4-dihydroqu inolin-2(1H)-one (compound 40)

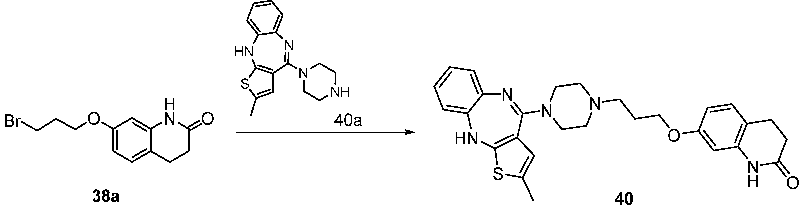

The title compound 40 (off-white solid) was prepared using the same synthetic route as in Example 20, except that 24a in Example 20 was replaced with 38a and 1-(2,3-dichlorophenyl)piperazine hydrochloride (1c) was replaced with 40a.

LCMS: 502 [M+H]⁺

¹H NMR (400 MHz, DMSO-*d*₆) δ 9.96 (s, 1H), 7.62 (s, 1H), 7.03 (d, 1H), 6.92-6.74 (m, 3H), 6.68 (d, 1H), 6.47 (d, 1H), 6.45 (d, 1H), 6.35 (s, 1H), 3.94 (t, 2H), 3.41-3.16 (m, 4H), 2.77 (t, 2H), 2.48-2.32 (m, 8H), 2.26 (s, 3H), 1.89 (s, 2H).

### Example 36: synthesis of 7-(4-(4-(2-methyl-10H-benzo[b]thieno[2,3-e][1,4]diazepin-4-yl)piperazin-1-yl)-4-oxobutoxy)-3,4-dihyd roquinolin-2(1H)-one (compound 41)

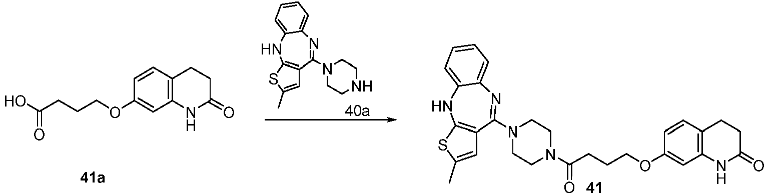

41a (200 mg, 0.80 mmol, 1.0 eq.) and N,N-dimethylformamide (6 mL) were added to a reaction flask, and then HATU (380 mg, 0.96 mmol, 1.2 eq.) was added. The reaction solution was stirred at room temperature for 30 minutes, then 40a (240 mg, 0.80 mmol, 1.0 eq.) and N,N-diisopropylethylamine (309 mg, 2.4 mmol, 3.0 eq.) were added, and the mixture was reacted at room temperature for 4 hours. After LCMS monitoring showed complete consumption of the starting material, the mixture was filtered, concentrated, and purified by C18 reversed-phase column chromatography, followed by normal-phase column chromatography to obtain the title compound 41 (off-white solid) (100 mg, yield: 24%).

LCMS: 530 [M+H]⁺

¹H NMR (400 MHz, DMSO-*d*₆) δ 9.96 (s, 1H), 7.63 (s, 1H), 7.03 (d, 1H), 6.89-6.73 (m, 3H), 6.68 (d, 1H), 6.48 (d, 1H), 6.42 (s, 1H), 6.38 (s, 1H), 3.92 (t, 2H), 3.52 (s, 4H), 3.32 (s, 2H), 3.29-3.20 (m, 2H), 2.76 (t, 2H), 2.49-2.44 (m, 2H), 2.39 (t, 2H), 2.27 (s, 3H), 2.00-1.84 (m, 2H).

### Example 37: synthesis of 7-(4-(4-(2-methyl-10H-benzo[b]thieno[2,3-e][1,4]diazepin-4-yl)piperazin-1-yl)butoxy)-3,4-dihydroqui nolin-2(1H)-one (compound 42)

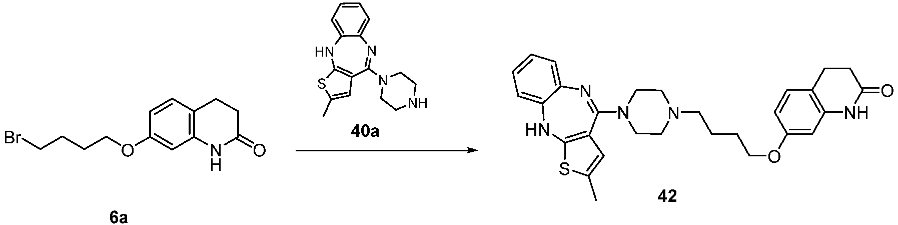

The title compound 42 (off-white solid) was prepared using the same synthetic route as in Example 7, except that 9a in Example 7 was replaced with 6a and 1-(2,3-dichlorophenyl)piperazine hydrochloride (1c) was replaced with 40a.

LCMS: 516 [M+H]⁺

¹H NMR (400 MHz, DMSO-*d*₆) δ 9.96 (s, 1H), 7.58 (s, 1H), 7.04 (d, 1H), 6.88-6.72 (m, 3H), 6.68 (d, 1H), 6.48 (d, 1H), 6.42 (s, 1H), 6.32 (s, 1H), 3.90 (t, 2H), 3.31-3.27 (m, 4H), 2.76 (t, 2H), 2.41-2.33 (m, 8H), 2.26 (s, 3H), 1.76-1.66 (m, 2H), 1.62-1.52 (m, 2H).

### Example 38: synthesis of 7-(2-(4-(1-propionylindolin-5-yl)piperazin-1-yl)ethoxy)-3,4-dihydroquinolin-2(1H)-one (compound 43)

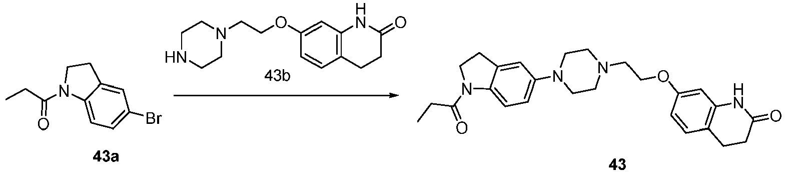

43a (506 mg, 2.0 mmol, 1.0 eq.), dioxane (10 mL), 43b (684 mg, 2.2 mmol, 1.1 eq.), and sodium tert-butoxide (576 mg, 6.0 mmol, 3.0 eq.) were added to a reaction flask sequentially. The reaction solution was purged with nitrogen three times, then tris(dibenzylideneacetone)dipalladium(0) (115 mg, 0.2 mmol, 0.1 eq.) was added, and the mixture was reacted at 100°C under nitrogen protection for 12 hours. After LCMS monitoring showed complete consumption of the starting material, the reaction solution was filtered and concentrated, and purified by C18 reversed-phase column chromatography followed by normal-phase column chromatography (DCM:MeOH = 10:1) to obtain the title compound 43 (179 mg, yield: 20%).

LCMS: 449 [M+H]⁺

¹H NMR (400 MHz, DMSO-*d*₆) δ 8.02 (d, 1H), 7.51 (s, 1H), 7.04 (d, 1H), 6.84 (s, 1H), 6.78 (d, 1H), 6.55 (d, 1H), 6.44 (s, 1H), 4.14 (t, 2H), 4.05 (t, 2H), 3.23-3.11 (m, 6H), 2.93 (t, 2H), 2.88-2.78 (m, 6H), 2.54 (t, 2H), 2.47 (q, 2H), 1.18 (t, 3H).

### Example 39: synthesis of 6-(4-(4-(benzo[d]thiazol-2-yl)piperazin-1-yl)butoxy)indolin-2-one (compound 44)

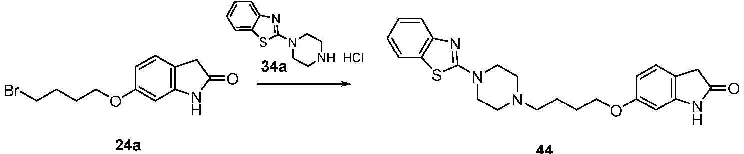

**24a** (100 mg, 0.37 mmol, 1.0 eq.) was dissolved in acetonitrile (6 mL) in a reaction flask, and then 34a (130 mg, 0.45 mmol, 1.2 eq.), potassium carbonate (150 mg, 1.11 mmol, 3.0 eq.), and potassium iodide (10 mg, 0.0037 mmol, 0.1 eq.) were added. The reaction solution was reacted at 60°C for 4 hours. After LCMS monitoring showed complete consumption of the starting material, the reaction solution was filtered, and the filtrate was concentrated and purified by normal-phase column chromatography (eluent: DCM:MeOH = 10:1) to obtain the title compound 44 (off-white solid, 80 mg, yield: 51%).

LCMS: 423 [M+H]⁺

¹H NMR (400 MHz, CDCl₃) δ 7.79 (s, 1H), 7.61 (d, 1H), 7.56 (d, 1H), 7.30 (t, 1H), 7.10-7.08 (m, 2H), 6.52 (d, *J=* 8.2 Hz, 1H), 6.44 (s, 1H), 3.97 (t, 2H), 3.72 (s, 3H), 3.46 (s, 2H), 2.80-2.45 (m, 5H), 1.91-1.69 (m, 4H), 1.61 (s, 2H).

### Example 40: synthesis of 2-((2-(4-(3,5-bis(trifluoromethyl)phenyl)piperazin-1-yl)-2-oxoethyl)thio)-N-(m-tolyl)acetamide (compound 45)

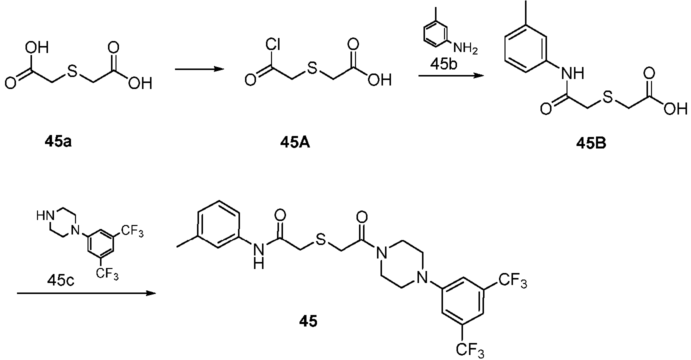

**Step 1:** 2,2'-Thiodiacetic acid (45a) (1.00 g, 6.667 mmol, 1.0 eq.), DCM (10 mL), and DMF (49 mg, 0.67 mmol, 0.1 eq.) were added to a reaction flask, and oxalyl chloride (847 mg, 6.67 mmol, 1.0 eq.) was added dropwise under an ice bath. After the dropwise addition, the reaction solution was stirred at room temperature for 2 hours and directly concentrated under reduced pressure to obtain 1.1 g of crude 45A.

**Step 2:** 45A (1.10 g, 6.55 mmol, 1.0 eq.), DCM (5 mL), TEA (2.54 g, 19.65 mmol, 3 eq.), and m-toluidine (45b) (701 mg, 6.55 mmol, 1 eq.) were added to a reaction flask and stirred at room temperature for 2 hours. The reaction solution was purified by column chromatography (eluent: dichloromethane:methanol = 9:1), and the target fraction was collected and concentrated under reduced pressure to obtain a white solid 45B (1.0 g, yield: 63%).

**Step 3:** 45B (100 mg, 0.42 mmol, 1.0 eq.), DCM (5 mL), and HATU (191 mg, 0.50 mmol, 1.2 eq.) were added to a reaction flask and stirred at room temperature for 30 minutes, then DIPEA (162 mg, 1.25 mmol, 3 eq.) and 1-(3,5-bis(trifluoromethyl)phenyl)piperazine (45c) (126 mg, 0.42 mmol, 1.0 eq.) were added. The reaction solution was stirred at room temperature overnight, concentrated, and purified by column chromatography (eluent: methanol:0.1% aqueous formic acid = 65:35). The target fraction was collected and concentrated under reduced pressure to obtain the title compound 45 (white solid) (114 mg, yield: 52%).

LCMS: 520 [M+H]⁺

¹H NMR (400 MHz, DMSO-d) δ 9.97 (s, 1H), 7.43 (s, 2H), 7.38 (s, 1H), 7.33-7.32 (m, 2H), 7.15 (t,1H), 6.85 (d, 1H), 3.67-3.57 (m, 6H), 3.44-3.38 (m, 4H), 3.37-3.33 (m, 2H), 2.24 (s, 3H).

### Example 41: synthesis of 2-((2-(4-(2,3-dichlorophenyl)piperazin-1-yl)-2-oxoethyl)thio)-N-(m-tolyl)acetamide (compound 46)

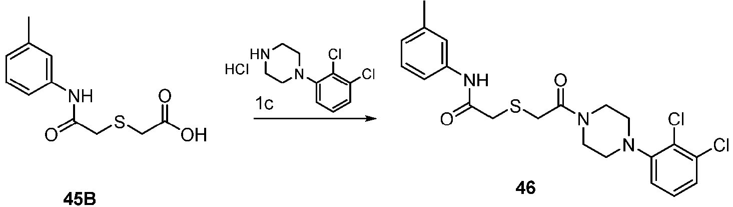

The title compound 46 (white solid) was prepared using the same synthetic route as in Step 3 of Example 40, except that 1-(3,5-bis(trifluoromethyl)phenyl)piperazine (45c) in Step 3 of Example 40 was replaced with 1-(2,3-dichlorophenyl)piperazine hydrochloride (1c).

LCMS: 452 [M+H]⁺

¹H NMR (400 MHz, DMSO-*d*₆) δ 10.03 (s, 1H), 7.42 (s, 1H), 7.36-7.28 (m, 3H), 7.17 (t, 1H), 7.10 (dd, 1H), 6.86 (d, 1H), 3.65-3.62 (m, 6H), 3.40 (s, 2H), 2.99 (t, 2H), 2.92 (t, 2H), 2.26 (s, 3H).

### Example 42: synthesis of N-(3-amino-4-methoxyphenyl)-2-(4-(2,3-dichlorophenyl)piperazin-1-yl)acetamide (compound 47)

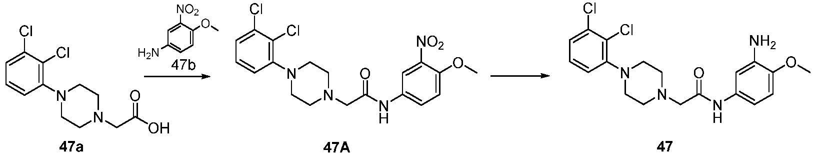

**Step 1:** 47a (1.5 g, 0.0052 mol, 1 eq.), 4-methoxy-3-nitroaniline (47b) (0.7 g, 0.0052 mol, 1 eq.), HATU (3.8 g, 0.01 mol, 2 eq.), and dichloromethane (30 mL) were added to a 250 mL three-necked flask and stirred at room temperature for 4 hours. After the reaction was complete, the mixture was separated by silica gel column chromatography (dichloromethane:methanol = 10:1) and concentrated under reduced pressure to obtain an off-white solid 47A (1.6 g, yield: 70.2%).

**Step 2:** 47A (1.6 g, 0.0037 mol, 1 eq.), Pd/C (0.5 g), and methanol (50 mL) were added to a 250 mL single-necked flask, purged with a hydrogen balloon three times, and stirred at room temperature for 16 hours. After the reaction was complete, the mixture was separated by silica gel column chromatography (dichloromethane:methanol = 10:1), concentrated under reduced pressure to obtain a crude product, which was purified by preparative chromatography and lyophilized to obtain the title compound 47 (off-white solid) (0.15 g, yield: 9.9%).

LCMS: 409 [M+1]⁺

¹H NMR (400 MHz, DMSO): δ 9.34 (s, 1H), 7.31 (m, 2H),7.17 (m, 1H), 6.99 (d, 1H), 6.74 (m, 2H), 3.71 (s, 3H), 3.14 (s, 2H), 3.04 (m, 4H), 2.69 (m, 4H).

### Example 43: synthesis of 2-(4-(2,3-dichlorophenyl)piperazin-1-yl)-N-(3-(dimethylamino)-4-methoxyphenyl)acetamide (compound 48)

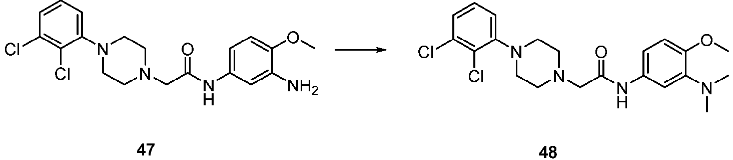

47 (80 mg, 0.20 mmol, 1.0 eq.) and methanol (6 mL) were added to a reaction flask, and then paraformaldehyde (6.6 mg, 0.22 mmol, 1.1 eq.) was added. The reaction was stirred at room temperature under nitrogen for 4 hours, then sodium cyanoborohydride (63 mg, 1.0 mmol, 5.0 eq.) was added, and the reaction solution was stirred at room temperature overnight. After the reaction was complete, the reaction solution was filtered, concentrated, and purified by C18 reversed-phase column chromatography to obtain the title compound 48 (white solid) (42 mg, yield: 50%).

LCMS: 437 [M+H]⁺

¹H NMR (400 MHz, DMSO-*d*₆) δ 9.54 (s, 1H), 7.43-7.26 (m, 2H), 7.27-7.13 (m, 3H), 6.88 (d, 1H), 3.78 (s, 3H), 3.24-3.14 (m, 2H), 3.13-3.04 (m, 4H), 2.78-2.73 (m, 4H), 2.71 (s, 6H).

### Example 44: synthesis of N-(3-amino-4-methoxyphenyl)-3-(4-(2,3-dichlorophenyl)piperazin-1-yl)propanamide (compound 49)

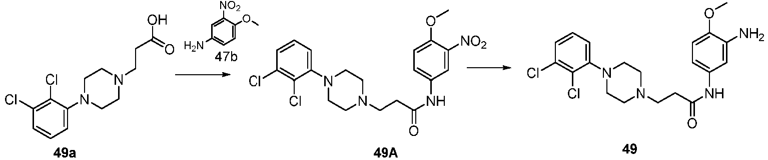

**Step 1:** 49a (135 mg, 0.47 mmol, 1.0 eq.) and N,N-dimethylformamide (6 mL) were added to a reaction flask, and then HATU (214 mg, 0.56 mmol, 1.2 eq.) was added. The reaction was stirred at room temperature under nitrogen for 1 hour, then 4-methoxy-3-nitroaniline (47b) (94 mg, 0.56 mmol, 1.0 eq.) and N,N-diisopropylethylamine (182 mg, 1.41 mmol, 3.0 eq.) were added, and the reaction was stirred at room temperature under nitrogen for 6 hours. After the reaction was complete, the reaction solution was directly purified by C18 column chromatography to obtain a white solid 49A (103 mg, yield: 50%).

**Step 2:** 49A (103 mg, 0.235 mmol, 1.0 eq.) and acetic acid (6 mL) were added to a reaction flask, and then iron powder (658 mg, 1.17 mmol, 5.0 eq.) was added. The reaction was stirred at 60°C under nitrogen for 4 hours. After the reaction was complete, the reaction solution was filtered, concentrated, and separated by column chromatography (dichloromethane:methanol = 10:1) to obtain the title compound 49 (gray solid) (80 mg, yield: 85%).

LCMS: 423 [M+H]⁺

¹H NMR (400 MHz, DMSO-*d*₆) δ 9.64 (s, 1H), 7.36-7.23 (m, 2H), 7.20-7.09 (m, 1H), 6.94 (d, 1H), 6.77-6.61 (m, 2H), 4.70 (s, 1H), 3.70 (s, 3H), 3.07-2.93 (m, 4H), 2.67 (t, 2H), 2.63-2.54 (m, 4H), 2.43 (t, 2H).

### Example 45: synthesis of 3-(4-(2,3-dichlorophenyl)piperazin-1-yl)-N-(4-methoxy-3-(methylamino)phenyl)propanamide (compound 50)

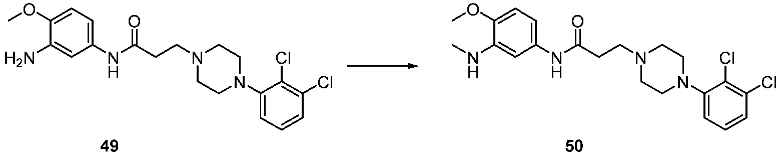

The title compound 50 (white solid) was prepared using the same synthetic route (during work-up, the reaction solution was filtered, concentrated, and purified by C18 reversed-phase column chromatography and normal-phase column chromatography) as in Example 43, except that compound 47 in Example 43 was replaced with compound 49.

LCMS: 437 [M+H]⁺

¹H NMR (400 MHz, DMSO-*d*₆) δ 9.72 (s, 1H), 7.33-7.23 (m, 2H), 7.13 (s, 1H), 6.79 (d, 1H), 6.72 (s, 1H), 6.66 (d, 1H), 5.00 (s, 1H), 3.70 (s, 3H), 3.06-2.88 (m, 4H), 2.69-2.66 (s, 3H), 2.62-2.52 (m, 4H), 2.44 (t, 2H).

### Example 46: synthesis of N⁴-(2-(4-(2,3-dichlorophenyl)piperazin-1-yl)ethyl)pyrimidin-2,4-diamine (compound 51)

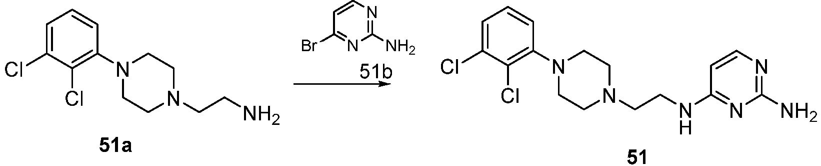

**51a** (0.9 g, 0.0029 mol, 1 eq.), 4-bromo-2-aminopyrimidine (51b) (0.56 g, 0.0032 mol, 1.1 eq.), cesium carbonate (8.5 g, 0.0261 mol, 9 eq.), Pd₂(dba)₃ (0.53 g, 0.00058 mol, 0.2 eq.), Xphos (0.41 g, 0.00087 mol, 0.3 eq.), and 1,4-dioxane (30 mL) were added to a 100 mL single-necked flask and purged with nitrogen three times. The mixture was heated to 100°C under nitrogen protection for 3 hours. After the reaction was complete, the mixture was filtered, and the filtrate was evaporated to dryness. The crude product was purified with methanol and filtered to obtain a yellow solid, which was purified by initial column chromatography (eluent: DCM:MeOH = 100:1~10:1) and then by column chromatography (eluent: methanol:0.5% aqueous formic acid = 80:20) to obtain the title compound 51 (yellow oil) (0.01 g, yield: 9.4%).

LCMS: 367 [M+1]⁺

¹H NMR (400 MHz, CD₃OD): δ 7.55-7.54 (d, 1H), 7.25-7.23 (m, 2H), 7.10-7.09 (d, 1H), 6.10-6.08 (d, 1H), 3.70 (s, 2H), 3.12-3.09 (m, 4H), 2.88-2.69 (m, 6H).

### Example 47: synthesis of 4-(((2-(4-(2,3-dichlorophenyl)piperazin-1-yl)ethyl)amino)methyl)benzene-1,2-diol (compound 52)

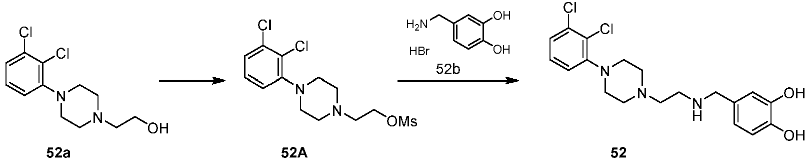

**Step 1:** Compound **52A** (off-white solid) was prepared using the same synthetic route as in Step 2 of Example 1, except that 1A in Step 2 of Example 1 was replaced with 52a.

**Step 2: 52A** (540 mg, 1.53 mmol, 1.0 eq.) was dissolved in dichloromethane (5 mL), and triethylamine (1.1 mL, 7.64 mmol, 5.0 eq.) and 3,4-dihydroxybenzylamine hydrochloride (52b) (673 mg, 3.06 mmol, 2.0 eq.) were added. The reaction was allowed to proceed at room temperature overnight, concentrated under reduced pressure to obtain a crude product, and purified by reversed-phase column chromatography (eluent: methanol:0.1% aqueous formic acid = 80:20). The target fraction was collected, concentrated, and dried to obtain the title compound 52 (yellow oily liquid) (92 mg, yield: 15%).

LCMS: 396 [M+H]⁺

¹H NMR (400 MHz, CD₃OD) δ 7.26-7.17 (m, 2H), 7.09 (dd, 1H), 6.99 (s, 1H), 6.88-6.81 (m, 2H), 4.12 (s, 2H), 3.17 (t, 2H), 3.05 (br, 4H), 2.76 (t, 2H), 2.68 (br, 4H).

### Example 48: synthesis of N-(4-(4-(3,5-bis(trifluoromethyl)phenyl)piperazin-1-yl)butyl)-4-chlorobenzamide (compound 53)

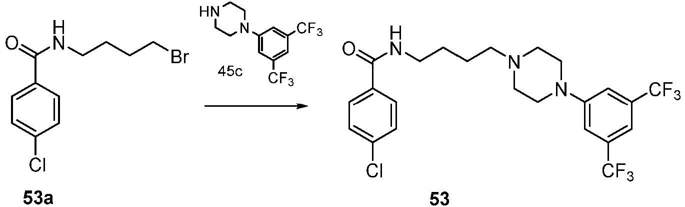

Compound **53a** (145 mg, 0.50 mmol, 1.0 eq.) was dissolved in acetonitrile (10 mL) in a reaction flask, and then potassium carbonate (207 mg, 1.50 mmol, 3.0 eq.), 1-(3,5-bis(trifluoromethyl)phenyl)piperazine hydrochloride **(45c)** (180 mg, 0.6 mmol, 1.2 eq.), and potassium iodide (10 mg, 0.05 mmol, 0.1 eq.) were added. The reaction solution was reacted at 85°C for 8 hours. After LCMS monitoring showed complete consumption of the starting material, the reaction solution was filtered, concentrated, and purified by C18 reversed-phase column chromatography to obtain the title compound **53** (off-white solid, 127 mg, yield: 50%).

LCMS: 508 [M+H]⁺

¹H NMR (400 MHz, DMSO-*d*₆) δ 8.53 (t, 1H), 7.85 (d, 2H), 7.52 (d, 2H), 7.43 (s, 2H), 7.28 (s, 1H), 3.34-3.23 (m, 10H), 2.35 (t, 2H), 1.61-1.44 (m, 4H).

### Example 49: synthesis of (E)-1-(4-(2,3-dichlorophenyl)piperazin-1-yl)-3-(3,4,5-trihydroxyphenyl)prop-2-en-1-one (compound 55)

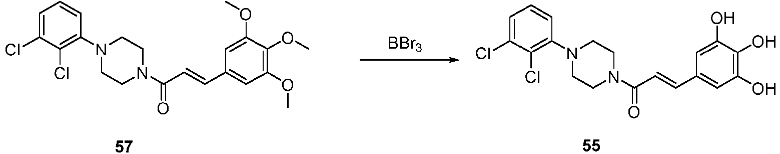

57 (0.4 g, 0.88 mmol, 1.0 eq.) and dichloromethane (30 mL) were added to a reaction flask, and boron tribromide (17% dichloromethane solution, 4.0 mL, 3.0 mmol, 3.5 eq.) was added dropwise under an ice bath. After the dropwise addition, the mixture was stirred at room temperature overnight. The reaction was quenched by adding a saturated sodium carbonate solution, extracted with 50 mL of dichloromethane, and a solid precipitated. The solid was dissolved in methanol and purified by C18 reversed-phase column chromatography (eluent: methanol:0.5% aqueous formic acid = 65:35). The target fraction was collected and concentrated to obtain the title compound 55 (white solid) (117 mg, yield: 32.5%).

LCMS: 409 [M+H]⁺

¹H NMR (400 MHz, DMSO-*d*₆) δ 7.33-7.31 (m, 2H), 7.28 (d, 1H), 7.17-7.14 (m, 1H), 6.89 (d, 1H), 6.62 (s, 2H), 3.81-3.72 (m, 4H), 2.98 (br, 4H).

### Example 50: synthesis of (E)-1-(4-(2,3-dichlorophenyl)piperazin-1-yl)-4-(3,4-dimethoxyphenyl)but-3-en-1-one (compound 59)

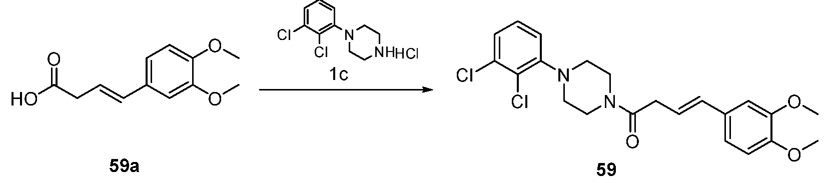

The title compound 59 (off-white solid) was prepared using the same synthetic route as in Step 3 of Example 40, except that 1-(3,5-bis(trifluoromethyl)phenyl)piperazine (45c) in Step 3 of Example 40 was replaced with 1-(2,3-dichlorophenyl)piperazine hydrochloride (1c), and 45B was replaced with 59a.

LCMS: 435 [M+H]⁺

¹H NMR (400 MHz, DMSO-*d*₆) δ 7.32-7.29 (m, 2H), 7.14 (dd, 1H), 7.02 (s, 1H), 6.88 (s, 2H), 6.39 (d, 1H), 6.26-6.16 (m, 1H), 3.76 (s, 3H), 3.73 (s, 3H), 3.64 (br, 4H), 3.33 (d, 2H), 2.98-2.94 (m, 4H).

### Example 51: synthesis of (E)-1-(2,3-dichlorophenyl)-4-(3-(3,4-dimethoxyphenyl)allyl)piperazine (compound 60)

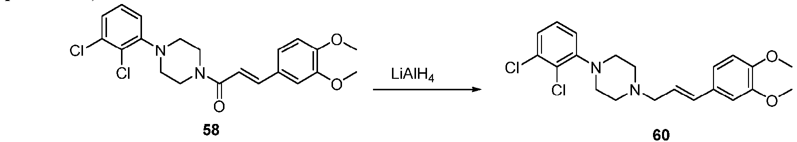

**58** (1.1 g, 2.6 mmol, 1.0 eq.) and THF (30 mL) were added to a reaction flask, and LiAlH₄ (2.5 mol/L THF solution, 1.5 mL, 3.9 mmol, 1.5 eq.) was added dropwise under an ice bath. After the dropwise addition, the mixture was stirred at room temperature for 0.5 hour. Saturated sodium sulfate solution was added dropwise, and the mixture was filtered. The filter cake was washed with DMSO, and the mother liquor was concentrated to obtain a crude product, which was purified by C18 reversed-phase column chromatography (eluent: methanol:0.5% aqueous formic acid = 80:20). The target fraction was collected and concentrated to obtain the title compound 60 (oily liquid) (0.54 g, yield: 51%).

LCMS: 407 [M+H]⁺

¹H NMR (400 MHz, DMSO-*d*₆) δ 7.32-7.22 (m, 2H), 7.14 (dd, 1H), 7.08 (s, 1H), 6.94-6.86 (m, 2H), 6.48 (d, 1H), 6.25-6.16 (m, 1H), 3.77 (s, 3H), 3.73 (s, 3H), 3.17 (d, 2H), 3.00 (br, 4H), 2.61 (br, 4H).

### Example 52: synthesis of (E)-4-(3-(4-(2,3-dichlorophenyl)piperazin-1-yl)prop-1-en-1-yl)benzene-1,2-diol (compound 61)

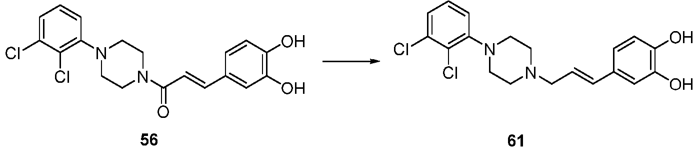

The title compound 61 (off-white solid) was prepared using the same synthetic route as in Example 51, except that 58 in Example 51 was replaced with 56.

LCMS: 379 [M+1]⁺

¹H NMR (400 MHz, DMSO): δ 7.30 (m, 2H), 7.14 (m, 1H), 6.82 (s, 1H), 6.68 (m, 2H), 6.38 (d, 1H), 5.99 (m, 1H), 3.14 (d, 2H), 2.99 (s, 4H), 2.58 (s, 4H).

### Example 53: synthesis of (E)-5-(3-(4-(2,3-dichlorophenyl)piperazin-1-yl)prop-1-en-1-yl)-2-(2-hydroxypropoxy)phenol (compound 62)

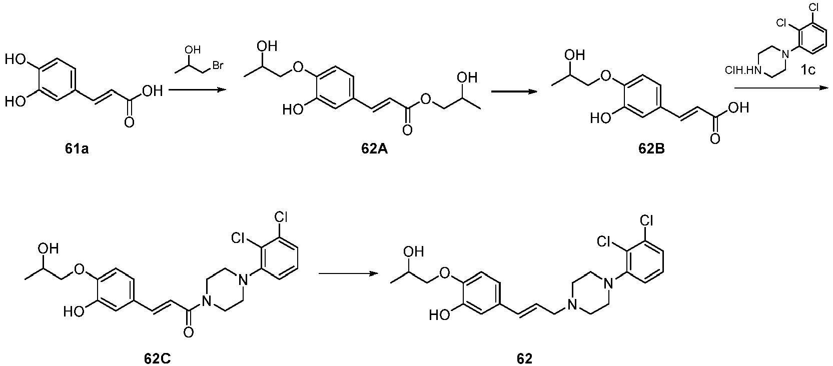

**Step 1:** Compound **62A** (yellow oil) was prepared using the same synthetic route as in Step 1 of Example 1, except that 1a in Example 1 was replaced with 1-bromo-2-propanol and 1b was replaced with 61a.

**Step 2: 62A** (1.5 g, 5.06 mmol) was added to a mixed solvent of THF/H₂O (40 mL, 3:1), and then sodium hydroxide (405 mg, 10.1 mmol) was added. The reaction solution was stirred at room temperature for 20 hours, adjusted to pH 5-6 with 1M HCl aqueous solution, and extracted with ethyl acetate. The organic phase was dried, filtered, and concentrated to obtain a black solid 62B (700 mg, yield: 58.0%).

**Step 3:** Compound 62C (brown solid) was prepared using the same synthetic route as in Example 18, except that 21b in Example 18 was replaced with 62B and 21a was replaced with 1c.

**Step 4: 62C** (420 mg, 0.93 mmol) was added to THF (5 mL), and then LiAlH₄ (1.1 mL, 2.5 M in THF) was slowly added dropwise at 0°C. The reaction solution was stirred at 0°C for 2 hours, quenched with water, filtered, and concentrated to obtain a crude product, which was purified by C-18 reversed-phase column chromatography (eluent: (H₂O (0.1% NH₄HCO₃):MeOH = 20:80). The target fraction was collected and lyophilized to obtain the title compound 62 (white solid, 76 mg, yield: 16.9%).

LCMS: 437 [M+H]⁺

¹H NMR (400 MHz, DMSO-*d*₆) δ 8.69 (s, 1H),7.30 - 7.29 (m, 2H), 7.15 - 7.13 (m, 1H), 6.90 (s, 1H), 6.84 - 6.78 (m,2H), 6.44 - 6.40 (m, 1H), 6.11 - 6.05 (m, 1H), 4.95 (s, 1H), 3.99 - 3.91 (m, 1H), 3.86 - 3.82 (m, 1H), 3.70 - 3.64 (m, 1H), 3.17 (d, 2H), 3.05 - 2.94 (m, 4H), 2.69 - 2.57 (m, 4H), 1.14 - 1.11 (m, 3H).

### Example 54: synthesis of 4-(2,3-dichlorophenyl)-N-(3,4-dihydroxyphenethyl)piperazine-1-carboxamide (compound 63)

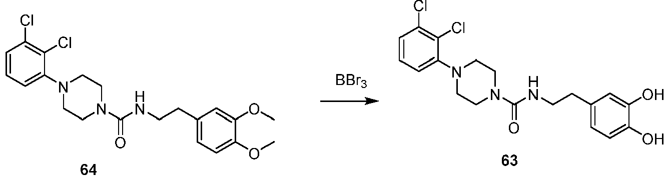

The title compound 63 (white solid) was prepared using the same synthetic route as in Example 49, except that 57 in Example 49 was replaced with 64.

LCMS: 410 [M+H]⁺

¹H NMR (400 MHz, DMSO-*d*₆) δ 8.72 (s, 1H), 8.59 (s, 1H), 7.31 (d, 2H), 7.14 (t, 1H), 6.65-6.59 (m, 2H), 6.57 (s, 1H), 6.42 (d, 1H), 3.43 (br, 4H), 3.15 (q, 2H), 2.90 (br, 4H), 2.52 (t, 2H).

### Example 55: synthesis of 4-(2,3-dichlorophenyl)-N-(3,4-dimethoxyphenethyl)piperazine-1-carboxamide (compound 64)

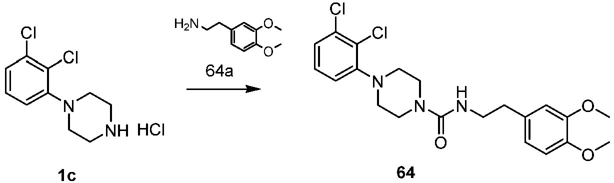

Triphosgene (89 mg, 0.30 mmol, 0.4 eq.) was dissolved in dichloromethane (2 mL), a solution of 3,4-dimethoxyphenethylamine (64a) (163 mg, 0.90 mmol, 1.2 eq.) and triethylamine (0.3 mL, 2.24 mmol, 3.0 eq.) in dichloromethane (2 mL) was added dropwise, and the reaction was allowed to proceed at room temperature for 1 hour. Then, 1-(2,3-dichlorophenyl)piperazine hydrochloride (1c) (200 mg, 0.75 mmol, 1.0 eq.) was added, and the reaction was stirred at room temperature overnight. The mixture was concentrated under reduced pressure, and the crude product was purified by 18C reversed-phase column chromatography (eluent: methanol:0.1% aqueous formic acid = 70:30). The target fraction was collected and concentrated to obtain the title compound 64 (yellow solid) (80 mg, yield: 24%).

LCMS: 438 [M+H]⁺

¹H NMR (400 MHz, CD₃OD) δ 7.19-7.18 (m, 2H), 6.98 (t, 1H), 6.82-6.81 (m, 2H), 6.74 (d, 1H), 3.79 (s, 3H), 3.75 (s, 3H), 3.50 (br, 4H), 3.37 (t, 2H), 2.94-2.87 (m, 4H), 2.74 (t, 2H).

### Example 56: synthesis of 4-(2,3-dichlorophenyl)-N-(2-((3,4-dimethoxyphenyl)amino)-2-oxoethyl)piperazine-1-carboxamide (compound 65)

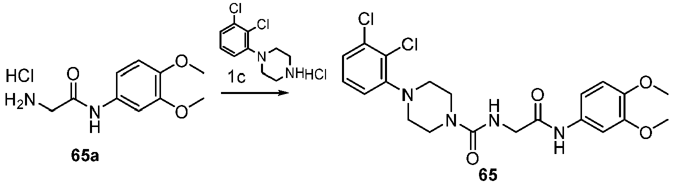

1-(2,3-Dichlorophenyl)piperazine hydrochloride (1c) (0.5 g, 1.86 mmol, 1.0 eq.), pyridine (0.32 g, 4.1 mmol, 2.2 eq.), triphosgene (0.2 g, 0.69 mmol, 0.37 eq.), and dichloromethane (20 mL) were added to a reaction flask and stirred at room temperature for 3 hours. 65a (0.5 g, 2.04 mmol, 1.1 eq.) was dissolved in dichloromethane (10 mL), and DIPEA (1.4 g, 11.2 mmol, 6.0 eq.) was added dropwise under an ice bath. After the dropwise addition, the mixture was stirred for 15 minutes, the above reaction solution was added dropwise, and then the mixture was stirred at room temperature overnight. The mixture was washed with 20 mL of water, and extracted with 50 mL of dichloromethane. The layers were separated, the organic phase was dried over anhydrous sodium sulfate, and purified by silica gel column chromatography (eluent: dichloromethane:methanol = 85:15). The target fraction was collected and concentrated to obtain the title compound 65 (white solid) (0.55 g, yield: 63.8%).

LCMS: 467 [M+H]⁺

¹H NMR (400 MHz, DMSO-*d*₆) δ 9.72 (s, 1H), 7.32-7.31 (m, 3H), 7.16 (t, 1H), 7.07 (d, 1H), 6.96 (t, 1H), 6.87 (d, 1H), 3.77 (d, 2H), 3.73 (s, 3H), 3.71 (s, 3H), 3.50 (br, 4H), 2.95 (br, 4H).

### Example 57: synthesis of 4-(2,3-dichlorophenyl)-N-(2-((3,4-dihydroxyphenyl)amino)-2-oxoethyl)piperazine-1-carboxamide (compound 66)

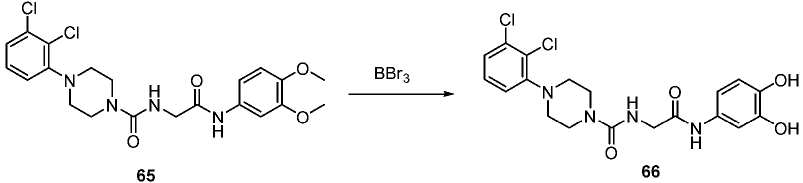

The title compound 66 (off-white solid) was prepared using the same synthetic route as in Example 49, except that 57 in Example 49 was replaced with 65.

LCMS: 439 [M+H]⁺

¹H NMR (400 MHz, DMSO-*d*₆) δ 9.48 (s, 1H), 8.90 (s, 1H), 8.55 (s, 1H), 7.31 (d, 2H), 7.15 (t, 1H), 7.11 (d, 1H), 6.93 (t, 1H), 6.79 (dd, 1H), 6.61 (d, 1H), 3.73 (d, 2H), 3.49 (br, 4H), 2.95-2.93 (m, 4H).

### Example 58: synthesis of 4-(2,3-dichlorophenyl)-N-(2-((3-hydroxyphenyl)amino)-2-oxoethyl)piperazine-1-carboxamide (compound 67)

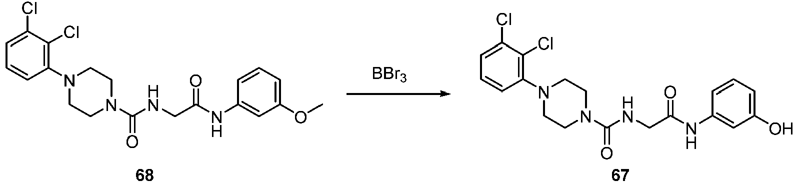

The title compound 67 (off-white solid) was prepared using the same synthetic route as in Example 49, except that 57 in Example 49 was replaced with 68.

LCMS: 423 [M+H]⁺

¹H NMR (400 MHz, DMSO-*d*₆) δ 9.74 (s, 1H), 9.35 (s, 1H), 7.31 (d, 2H), 7.17-7.15 (m, 2H), 7.05 (t, 1H), 7.00-6.94 (m, 2H), 6.42 (d, 1H), 3.77 (d, 2H), 3.49 (br, 4H), 3.00-2.89 (m, 4H).

### Example 59: synthesis of 4-(2,3-dichlorophenyl)-N-(2-((3-methoxyphenyl)amino)-2-oxoethyl)piperazine-1-carboxamide (compound 68)

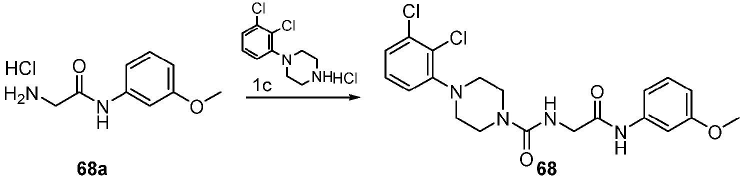

The title compound 68 (white solid) was prepared using the same synthetic route as in Example 56, except that 65a in Example 56 was replaced with 68a.

LCMS: 437 [M+H]⁺

¹H NMR (400 MHz, DMSO-*d*₆) δ 9.86 (s, 1H), 7.32-7.31 (m, 3H), 7.22-7.15 (m, 2H), 7.11 (d, 1H), 6.99 (t, 1H), 6.62 (dd, 1H), 3.79 (d, 2H), 3.71 (s, 3H), 3.50 (t, 4H), 2.96 (t, 4H).

### Example 60: synthesis of 1-(2,3-dichlorophenyl)-4-(3-(5-methoxypyridin-3-yl)prop-2-yn-1-yl)piperazine (compound 69)

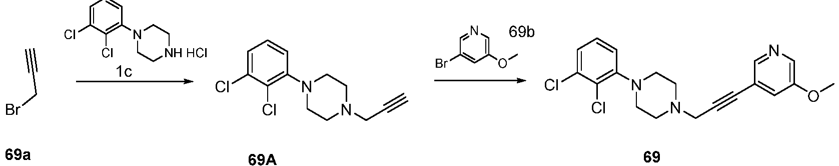

**Step 1:** 1-(2,3-Dichlorophenyl)piperazine hydrochloride 1C (1.08 g, 4.69 mmol, 1.0 eq.), propargyl bromide (830 mg, 7.04 mmol, 1.5 eq.), potassium carbonate (2.9 g, 14.1 mmol, 3.0 eq.), and MeCN (20 mL) were added to a reaction flask. The reaction solution was purged with nitrogen three times, and reacted at room temperature for 12 hours. After TLC and LCMS monitoring showed complete consumption of the starting material, the mixture was filtered, and the crude product was concentrated and purified by normal-phase column chromatography to obtain compound 69A (colorless liquid, 1 g, yield: 70%).

**Step 2:** 69A (500 mg, 1.86 mmol, 1.0 eq.), anhydrous acetonitrile (10 mL), Cs₂CO₃ (606 mg, 1.86 mmol, 1.0 eq.), 3-bromo-5-methoxypyridine (69b) (490.2 mg, 2.79 mmol, 1.5 eq.), and triethylenediamine (208.7 mg, 1.86 mmol, 1.0 eq.) were added to a reaction flask. The reaction solution was purged with nitrogen three times, and tris(dibenzylideneacetone)dipalladium(0) (110 mg, 0.19 mmol, 0.1 eq.) was added. The mixture was stirred at 50°C for 16 hours. After TLC monitoring showed complete consumption of the starting material, the mixture was filtered, and the crude product was concentrated and purified by C18 reversed-phase preparative chromatography. The target fraction was collected and concentrated to obtain the title compound 69 (white solid) (220 mg, yield: 30%).

LCMS: 376 [M+H]⁺

¹H NMR (400 MHz, CDCl₃) δ 8.30 (s, 1H), 8.25 (d, 1H), 7.24 (s, 1H), 7.20-7.09 (m, 2H), 6.98 (dd, 1H), 3.85 (s, 3H), 3.62 (s, 2H), 3.15 (br, 4H), 2.86 (br, 4H).

### Example 61: synthesis of 5-(3-(4-(2,3-dichlorophenyl)piperazin-1-yl)prop-1-yn-1-yl)pyridin-3-ol (compound 70)

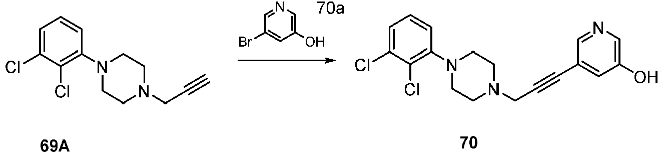

The title compound 70 (gray solid) was prepared using the same synthetic route as in Step 2 of Example 60, except that 3-bromo-5-methoxypyridine (69b) in Step 2 of Example 60 was replaced with 3-hydroxy-5-bromopyridine (70a).

LCMS: 362 [M+H]⁺

¹H NMR (400 MHz, CD₃OD) δ 8.34 (s, 1H), 8.07 (d, 2H), 7.26-7.22 (m, 3H), 7.11 (dd, 1H), 3.65 (s, 2H), 3.13 (br, 4H), 2.87 (br, 4H).

### Example 62: synthesis of 1-(2,3-dichlorophenyl)-4-(4-(5-methoxypyridin-3-yl)but-3-yn-1-yl)piperazine (compound 71)

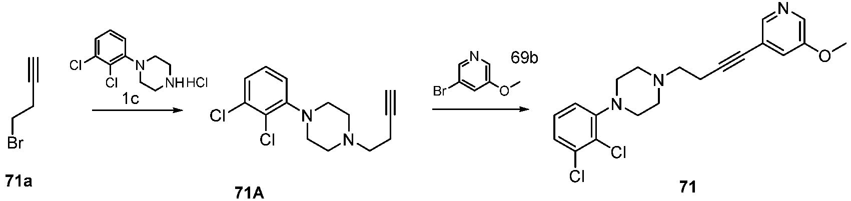

**Step 1:** Yellow solid 71A was prepared using the same synthetic route as in Example 7, except that 9a in Example 7 was replaced with 71a.

**Step 2: 71A** (200 mg, 0.71 mmol, 1.0 eq.) was dissolved in acetonitrile (3 mL), and 3-bromo-5-methoxypyridine (186 mg, 0.99 mmol, 1.4 eq.), cesium carbonate (690 mg, 2.12 mmol, 3.0 eq.), triethylenediamine (8 mg, 0.07 mmol, 0.1 eq.), and Pd₂(dba)₃ (32 mg, 0.04 mmol, 0.05 eq.) were added. The mixture was heated to 50°C under nitrogen protection overnight, diluted with water (50 mL), and extracted with ethyl acetate (3×50 mL). The organic phases were combined, washed with saturated brine (50 mL), dried, and concentrated. The residue was purified by silica gel column chromatography (eluent: petroleum ether:ethyl acetate = 2:1). The target fraction was collected and concentrated to obtain a crude product, which was purified by preparative TLC (dichloromethane:methanol = 20:1) to obtain the title compound 71 (yellow solid) (15 mg, yield: 5%).

LCMS: 390 [M+H]⁺

¹H NMR (400 MHz, DMSO-*d*₆) δ 8.23 (d, 1H), 8.17 (s, 1H), 7.38 (s, 1H), 7.30-7.28 (m, 2H), 7.14 (dd, 1H), 3.81 (s, 3H), 2.98 (br, 4H), 2.66-2.63 (m, 8H).

### Example 63: synthesis of 1-(4-(2,3-dichlorophenyl)piperazin-1-yl)-3-(5-methoxypyridin-3-yl)prop-2-yn-1-one (compound 72)

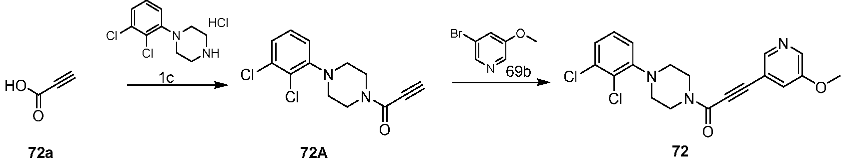

**Step 1:** Propiolic acid (72a) (100 mg, 1.43 mmol, 1.0 eq.) was dissolved in dichloromethane (3 mL), and 1-(2,3-dichlorophenyl)piperazine hydrochloride (1c) (458 mg, 1.71 mmol, 1.2 eq.), HATU (1.1 g, 2.86 mmol, 2.0 eq.), and DIPEA (0.7 mL, 4.28 mmol, 3.0 eq.) were added. The reaction was allowed to proceed at room temperature overnight, concentrated under reduced pressure, and purified by silica gel column chromatography (eluent: petroleum ether:ethyl acetate = 5:1). The target fraction was collected and concentrated to obtain **72A** as a white solid (380 mg, yield: 94%).

**Step 2:** 3-Bromo-5-methoxypyridine (69b) (280 mg, 1.49 mmol, 1.0 eq.) was dissolved in N,N-dimethylformamide (5 mL), and then cuprous iodide (57 mg, 0.30 mmol, 0.2 eq.), **72A** (590 mg, 2.08 mmol, 1.4 eq.), cesium carbonate (485 mg, 1.49 mmol, 1.0 eq.), and PdCl₂(PPh₃)₂ (52 mg, 0.07 mmol, 0.05 eq.) were added. The mixture was heated to 70°C under nitrogen protection overnight, diluted with water (50 mL), and extracted with ethyl acetate (3×50 mL). The organic phases were combined, washed with saturated brine (50 mL), dried, and concentrated. The residue was purified by silica gel column chromatography (eluent: petroleum ether:ethyl acetate = 2:1). The target fraction was collected and concentrated to obtain a crude product, which was purified by preparative TLC (petroleum ether:ethyl acetate = 1:1) to obtain the title compound 72 (brown oily liquid) (20 mg, yield: 3%).

LCMS: 390 [M+H]⁺

¹H NMR (400 MHz, CDCl₃) δ 8.37 (s, 1H), 8.33 (s, 1H), 7.35 (s, 1H), 7.21-7.14 (m, 2H), 6.93 (d, 1H), 4.00 (t, 2H), 3.11 (t, 2H), 3.06 (t, 2H). Note: five hydrogen signal peaks are overlapped by solvent signal peaks.

### Example 64: synthesis of (E)-2-(4-(3-(3,4-dimethoxyphenyl)allyl)piperazin-1-yl)benzo[d]thiazole (compound 73)

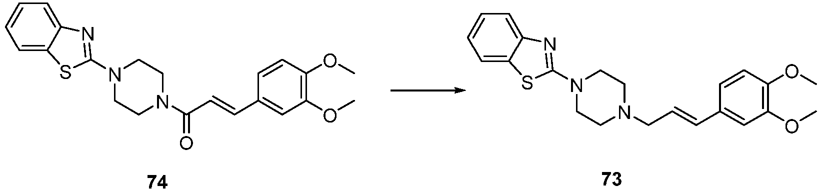

74 (200 mg, 0.49 mmol, 1.0 eq.) and tetrahydrofuran (5 mL) were added to a reaction flask, and a solution of lithium aluminum hydride in tetrahydrofuran (0.3 mL, 0.74 mmol, 1.5 eq.) was added under nitrogen protection in an ice-water bath. The reaction was warmed to room temperature and stirred under nitrogen for 0.5 hours, quenched with a saturated sodium sulfate aqueous solution, filtered and concentrated. The crude product was separated by C18 column chromatography, and then purified with methanol to obtain the title compound 73 (white solid) (26 mg, yield: 13%).

LCMS: 396 [M+H]⁺

¹H NMR (400 MHz, DMSO-*d*₆) δ 7.75 (d, 1H), 7.44 (d, 1H), 7.25 (t, 1H), 7.07-7.03 (m, 2H), 6.93 (dd, 2H), 6.43 (d, 1H), 6.23-6.16 (m, 1H), 3.76 (s, 3H), 3.72 (s, 3H), 3.55 (br, 4H), 3.13 (d, 2H), 2.53 (br, 4H).

### Example 65: synthesis of (E)-1-(4-(benzo[d]thiazol-2-yl)piperazin-1-yl)-3-(3,4-dimethoxyphenyl)prop-2-en-1-one (compound 74)

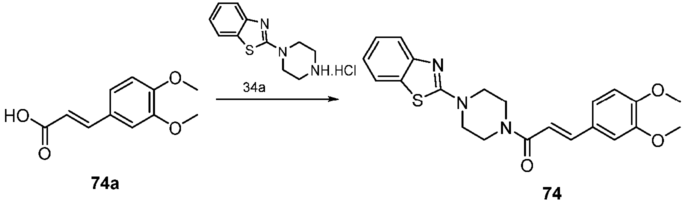

3,4-Dimethoxycinnamic acid (74a) (1.0 g, 4.81 mmol, 1.0 eq.) and DCM (30 mL) were added to a reaction flask, and then HATU (2.74 g, 7.21 mmol, 1.5 eq.) was added. The reaction was stirred at room temperature under nitrogen for 1 hour, then 34a (1.3 g, 5.04 mmol, 1.05 eq.) and DIPEA (1.86 g, 14.23 mmol, 3.0 eq.) were added, and the reaction was stirred at room temperature under nitrogen for 6 hours until complete. The mixture was filtered, and the crude product was purified with methanol to obtain the title compound 74 (white solid) (1.2 g, yield: 61%).

LCMS: 410 [M+H]⁺

¹H NMR (400 MHz, DMSO-*d*₆) δ 7.78 (d, 1H), 7.51-7.47 (m, 2H), 7.38 (s, 1H), 7.28 (t, 1H), 7.24 (d, 1H), 7.19 (d, 1H), 7.08 (t, 1H), 6.98 (d, 1H), 3.89 (br, 2H), 3.82 (s, 3H), 3.78-3.75 (m, 5H), 3.63 (br, 4H).

### Example 66: synthesis of 4-(5-(4-(2,3-dichlorophenyl)piperazin-1-yl)pentyl)-6-methyl-3,4-dihydro-2H-pyrido[3,2-b][1,4]oxazin e (compound 75)

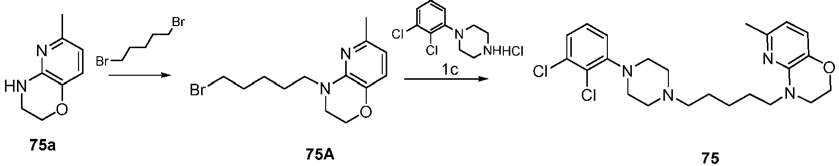

**Step** 1: 75a (0.3 g, 2.0 mmol, 1.0 eq.), 1,5-dibromopentane (0.68 g, 3.0 mmol, 1.5 eq.), and DMF (10 mL) were added to a reaction flask, and NaH (0.16 g, 4.0 mmol, 2.0 eq.) was added in portions under an ice bath. After the addition, the mixture was stirred at room temperature for 2 hours, quenched with water under an ice bath, and purified by silica gel column chromatography (eluent: dichloromethane:methanol = 95:5). The target fraction was collected and concentrated to obtain a pale yellow oily liquid **75A** (0.24 g, yield: 80.2%).

**Step 2:** The title compound 75 (colorless oily liquid) (127 mg, yield: 35.3%) was prepared using the same synthetic route as in Example 2, except that 3a in Example 2 was replaced with 75A.

LCMS: 449 [M+H]⁺

¹H NMR (400 MHz, DMSO-*d*₆) δ 7.30-7.29 (m, 2H), 7.12 (t, 1H), 6.76 (d, 1H), 6.26 (d, 1H), 4.10 (t, 2H), 3.52 (t, 2H), 3.40 (t, 2H), 2.98 (br, 4H), 2.61 (br, 4H), 2.43 (t, 2H), 2.20 (s, 3H), 1.59-1.46 (m, 4H), 1.35-1.20 (m, 2H).

### Example 67: synthesis of N-(2-(4-(5-chloro-2-methylphenyl)piperazin-1-yl)ethyl)-3-(6-methyl-2,3-dihydro-4H-pyrido[3,2-b][1,4 ]oxazin-4-yl)propanamide (compound 76)

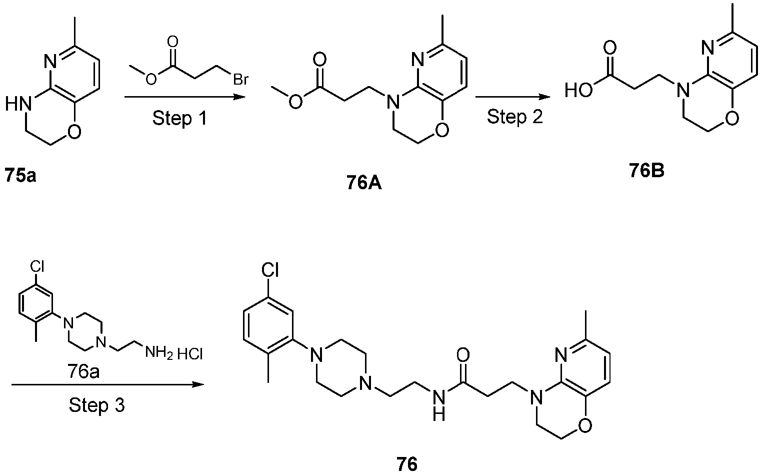

**Steps 1-2:** 75a (0.3 g, 2.0 mmol, 1.0 eq.), methyl 3-bromopropionate (0.4 g, 2.4 mmol, 1.2 eq.), and DMF (10 mL) were added to a reaction flask, and NaH (0.2 g, 5.0 mmol, 2.5 eq., 60%) was added in portions under an ice bath. After the addition, the mixture was warmed to 25°C and reacted for 4 hours. The reaction was quenched with water, and purified by silica gel column chromatography (eluent: DCM:MeOH = 60:40). The target fraction was collected and concentrated to obtain an off-white solid **76B** (0.35 g, yield: 78.7%).

**Step 3: 76B** (175 mg, 0.78 mmol, 1.0 eq.), HATU (358 mg, 0.94 mmol, 1.2 eq.), and DMF (8 mL) were added to a reaction flask and stirred at 25°C for 30 minutes, then **76a** (274 mg, 0.94 mmol, 1.2 eq.) and DIPEA (252 mg, 1.95 mmol, 2.5 eq.) were added, and the mixture was stirred at 25°C for 2 hours. The mixture was concentrated, and the crude product was purified by C18 reversed-phase column chromatography (eluent: 0.5% aqueous formic acid:acetonitrile = 40:60) to obtain the title compound 76 (off-white solid) (45 mg, yield: 12.6%).

LCMS: 458 [M+H]⁺

¹H NMR (400 MHz, DMSO-*d*₆) δ 9.41 (s, 1H), 7.34-722 (m, 2H), 7.10-7.07 (m, 2H), 6.61 (s, 1H), 4.22 (t, 2H), 3.88 (t, 2H), 3.74- 3.58(m, 10H), 3.47 (q, 2H), 2.94 (t, 2H), 2.61 (t, 2H), 2.41 (s, 3H), 2.24 (s, 3H).

### Example 68: synthesis of 4-(5-(4-(5-chloro-2-methylphenyl)piperazin-1-yl)pentyl)-6-methyl-3,4-dihydro-2H-pyrido[3,2-b][1,4]o xazine (compound 77)

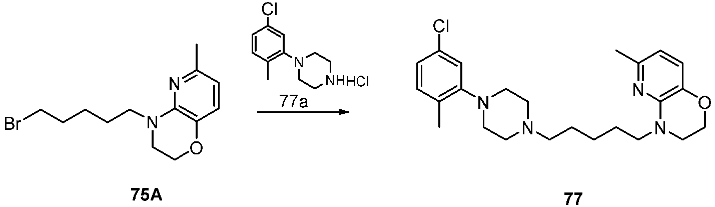

The title compound 77 (colorless oily liquid) was prepared using the same synthetic route as in Example 2, except that 3a in Example 2 was replaced with 75A and 1c was replaced with 77a.

LCMS: 429 [M+H]⁺

¹H NMR (400 MHz, CDCl₃) δ 7.09 (d, 1H), 6.99-6.97 (d, 2H), 6.79 (d, 1H), 6.29 (d, 1H), 4.17 (t, 2H), 3.62 (t, 2H), 3.43 (t, 2H), 3.07 (s, 4H), 2.94 (br, 4H), 2.76-2.68 (m, 2H), 2.32 (s, 3H), 2.24 (s, 3H), 1.80-1.71 (m, 2H), 1.68-1.62 (m, 2H), 1.43-1.38 (m, 2H).

### Example 69: synthesis of 5,6-dimethoxy-1-(4-(4-(3-(trifluoromethyl)phenyl)piperazin-1-yl)butyl)indol-2-one (compound 78)

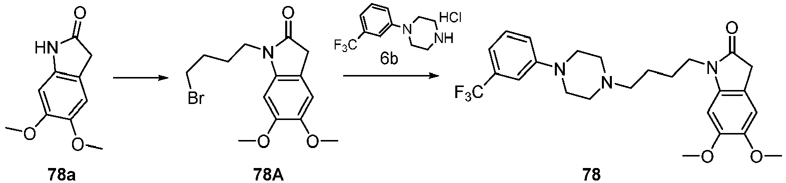

**Step 1:** 78a (1.0 g, 5.0 mmol, 1.0 eq.) and acetonitrile (30 mL) were added to a reaction flask, followed by addition of potassium carbonate (1.38 g, 10.0 mmol, 2.0 eq.), and 1,4-dibromobutane (3.34 g, 15.0 mmol, 3.0 eq.), and the reaction solution was reacted at 95°C for 8 hours. After LCMS monitoring showed complete consumption of the starting material, the mixture was filtered, concentrated, and purified by silica gel column chromatography to obtain an off-white solid 78A (500 mg, yield: 31%).

**Step 2:** The title compound 78 (off-white solid) was prepared using the same synthetic route as in Example 20, except that 24a in Example 20 was replaced with 78A and 1c was replaced with 6b.

LCMS: 478 [M+H]⁺

¹H NMR (400 MHz, DMSO-*d*₆) δ 7.39 (t, 1H), 7.19 (d, 1H), 7.12 (s, 1H), 7.04 (d, 1H), 6.96 (s, 1H), 6.74 (s, 1H), 3.77 (s, 3H), 3.68 (s, 3H), 3.65 (t, 2H), 3.44 (s, 2H), 3.24-3.14 (m, 4H), 2.53-2.43 (m, 4H), 2.34 (t, 2H), 1.66-1.53 (m, 2H), 1.49-1.38 (m, 2H).

### Example 70: synthesis of 1-(4-(4-(2,3-dichlorophenyl)piperazin-1-yl)butyl)-5,6-dimethoxyindolin-2-one (compound 79)

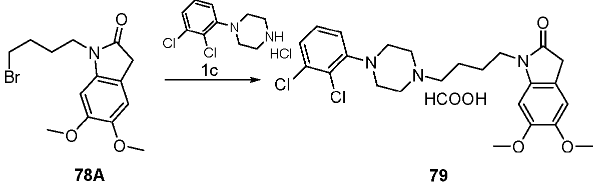

The title compound 79 (off-white solid) was prepared using the same synthetic route as in Example 20, except that 24a in Example 20 was replaced with 78A.

LCMS: 478 [M+H]⁺

¹H NMR (400 MHz, DMSO-*d*₆) δ 7.30-7.24 (m, 2H), 7.12 (dd, 1H), 6.96 (s, 1H), 6.74 (s, 1H), 3.78 (s, 3H), 3.68 (s, 3H), 3.65 (t, 2H), 3.44 (s, 2H), 2.99-2.90 (m, 4H), 2.54-2.50 (m, 4H), 2.37 (t, 2H), 1.64-1.55 (m, 2H), 1.51-1.41 (m, 2H).

### Example 71: synthesis of ethyl 2-((4-(4-(benzo[d]thiazol-2-yl)piperazin-1-yl)butanamido)methyl)-5-methylthiazole-4-carboxylate (compound 80)

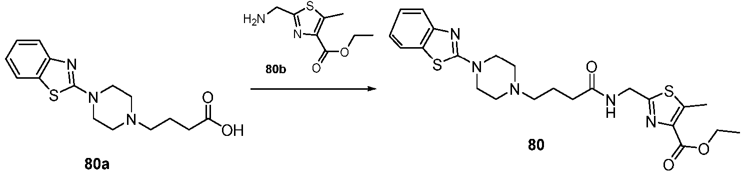

The title compound 80 (white solid) was prepared using the same synthetic route as in Step 3 of Example 67, except that 76a in Step 3 of Example 67 was replaced with 80b, and 76B was replaced with 80a.

LCMS: 488 [M+H]⁺

¹H NMR (400 MHz, DMSO-*d*₆) δ 8.78 (t, 1H), 7.77 (d, 1H), 7.46 (d, 1H), 7.28 (t, 1H), 7.08 (t, 1H), 4.44 (d, 2H), 4.25 (q, 2H), 3.62 (br, 4H), 3.34 (br, 2H), 2.77 (br, 4H), 2.64 (s, 3H), 2.22 (t, 2H), 1.83-1.72 (m, 2H), 1.27 (t, 3H).

### Example 72: synthesis of 4-(4-(benzo[d]thiazol-2-yl)piperazin-1-yl)-N-((4-(hydroxymethyl)-5-methylthiazol-2-yl)methyl)butana mide (compound 81)

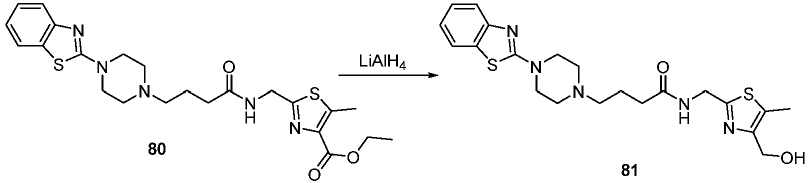

Compound **80** (0.276 g, 0.566 mmol, 1.0 eq.) and THF (10 mL) were added to a reaction flask, and LiAlH₄ (0.9 mL, 2.264 mmol, 4.0 eq., 2.5 mol/L in THF) was added dropwise under an ice bath. After the addition, the reaction was stirred for 1 hour. The reaction was quenched with saturated Na₂SO₄ solution, the solvent was evaporated, and the residue was dissolved in 4 mL of DMSO, and purified by C18 reversed-phase column chromatography (eluent: 0.5% aqueous formic acid:MeOH = 25:75). The target fraction was collected and concentrated to obtain the title compound 81 (white solid, 90 mg, yield: 35.7%).

LCMS: 446 [M+H]⁺

¹H NMR (400 MHz, DMSO-*d*₆) δ 8.65 (t, 1H), 7.75 (d, 1H), 7.44 (d, 1H), 7.27 (t, 1H), 7.06 (t, 1H), 4.41-4.39 (m, 4H), 3.54 (t, 4H), 2.53-2.50 (m, 4H), 2.40-2.30 (m, 5H), 2.18 (t, 2H), 1.76-1.67 (m, 2H).

### Example 73: synthesis of ethyl 2-((4-(4-(2,3-dichlorophenyl)piperazin-1-yl)butanamido)methyl)-5-methylthiazole-4-carboxylate (compound 82)

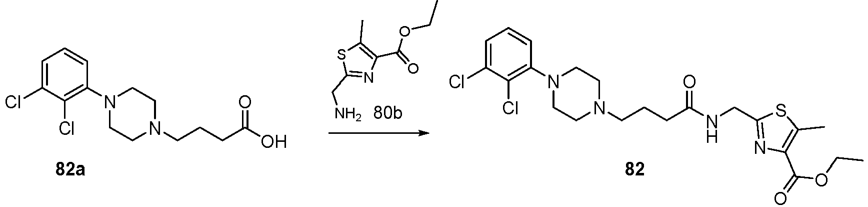

The title compound 82 (white solid) was prepared using the same synthetic route as in Step 3 of Example 67, except that 76a in Step 3 of Example 67 was replaced with 80b, and 76B was replaced with 82a.

LCMS: 499 [M+H]⁺

¹H NMR (400 MHz, DMSO-*d*₆) δ 8.83 (t, 1H), 7.38-7.29 (m, 2H), 7.20 (dd 1H), 4.46 (d, 2H), 4.26 (q, 2H), 3.32 (br, 4H), 3.14 (br, 4H), 2.92 (br, 2H), 2.65 (s, 3H), 2.25 (t, 2H), 1.90-1.82 (m, 2H), 1.28 (t, 3H).

### Example 74: synthesis of 4-(4-(2,3-dichlorophenyl)piperazin-1-yl)-N-((4-(hydroxymethyl)-5-methylthiazol-2-yl)methyl)butana mide (compound 83)

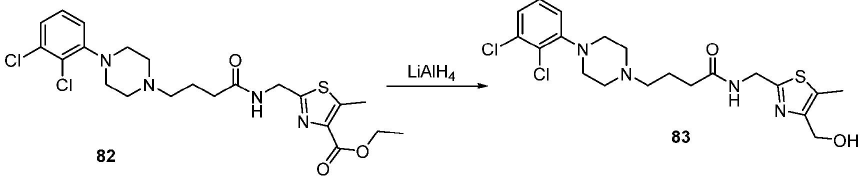

The title compound 83 (pale yellow oily liquid) was prepared using the same synthetic route as in Example 51, except that 58 in Example 51 was replaced with 82.

LCMS: 457 [M+H]⁺

¹H NMR (400 MHz, DMSO-*d*₆) δ 8.68 (t, 1H), 7.33-7.28 (m, 2H), 7.15 (dd, 1H), 4.42-4.41 (m, 4H), 2.99 (br, 4H), 2.55 (br, 2H), 2.40-2.35 (m, 5H), 2.19 (t, 2H), 1.76-1.71 (m, 2H). Note: two hydrogen signal peaks are overlapped by solvent signal peaks.

### Example 75: synthesis of N-(2-(4-(2,3-dichlorophenyl)piperazin-1-yl)ethyl)-2-(3-oxo-3,4-dihydro-2H-benzo[b][1,4]oxazin-2-yl)a cetamide (compound 85)

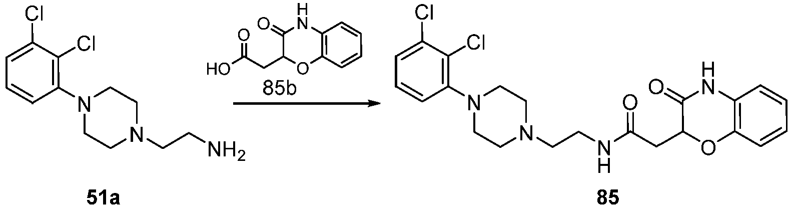

The title compound 85 (white solid) was prepared using the same synthetic route as in Step 3 of Example 67, except that 76a in Step 3 of Example 67 was replaced with 51a, and 76B was replaced with 85b.

LCMS: 463 [M+H]⁺

¹H NMR (400 MHz, DMSO-*d*₆) δ 10.71 (s, 1H), 7.96 (t, 1H), 7.35-7.30 (m, 2H), 7.16 (dd, 1H), 6.96-6.90 (m, 4H), 4.93 (dd, 1H), 3.26 (q, 2H), 3.01 (br, 4H), 2.77-2.66 (m, 2H), 2.61 (br, 4H), 2.47 (t, 2H).

### Example 76: synthesis of (S)-N⁶-(3-(4-(benzo[d]thiazol-2-yl)piperazin-1-yl)propyl)-4,5,6,7-tetrahydrobenzo[d]thiazole-2,6-diam ine (compound 86)

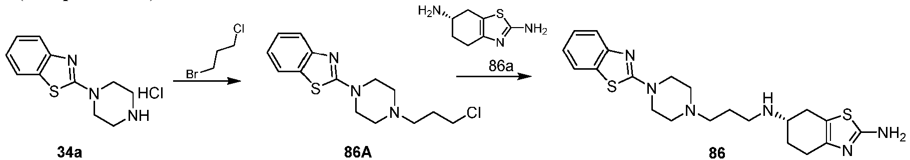

**Step 1:** 2-(Piperazin-1-yl)benzo[d]thiazole hydrochloride (34a) (200 mg, 0.78 mmol, 1.0 eq.) was dissolved in acetone (3 mL), and a solution of sodium hydroxide (66 mg, 1.64 mmol, 2.1 eq.) and water (0.3 mL) was added. The mixture was reacted at room temperature for 1 hour, then 1-bromo-3-chloropropane (123 mg, 0.78 mmol, 1.0 eq.) was added, and the reaction was stirred at room temperature overnight. Acetone was removed under reduced pressure, and the residue was purified by silica gel column chromatography (eluent: petroleum ether:ethyl acetate = 1:4) to obtain white solid **86A** (60 mg, yield: 26%).

**Step 2: 86A** (60 mg, 0.20 mmol, 1.0 eq.) was dissolved in acetonitrile (1 mL), and (S)-2,6-diamino-4,5,6,7-tetrahydrobenzo[d]thiazole (86a) (41 mg, 0.24 mmol, 1.2 eq.), N,N-diisopropylethylamine (105 mg, 0.81 mmol, 4.0 eq.), and potassium iodide (34 mg, 0.20 mmol, 1.0 eq.) were added. The reaction was heated to 85°C and allowed to proceed overnight. The mixture was concentrated under reduced pressure, and the residue was purified by C18 reversed-phase column chromatography (eluent: methanol:0.1% aqueous ammonia = 90:10) and then by C18 reversed-phase column chromatography (eluent: methanol:0.1% aqueous formic acid = 70:30) to obtain the title compound 86 (pale yellow viscous solid) (18 mg, yield: 19%).

LCMS: 215 [M/2+H]⁺

¹H NMR (400 MHz, CD₃OD) δ 7.65 (d, 1H), 7.48 (d, 1H), 7.30 (t, 1H), 7.10 (t, 1H), 3.65-3.50 (m, 5H), 3.27 (t, 2H), 3.08 (dd, 1H), 2.79 (dd, 1H), 2.71-2.56 (m, 8H), 2.27-2.19 (m, 1H), 2.13-2.04 (m, 1H), 2.02-2.91 (m, 2H).

### Example 77: synthesis of (S)-N-(2-amino-4,5,6,7-tetrahydrobenzo[d]thiazol-6-yl)-3-(4-(benzo[d]thiazol-2-yl)piperazin-1-yl)pro panamide (compound 87)

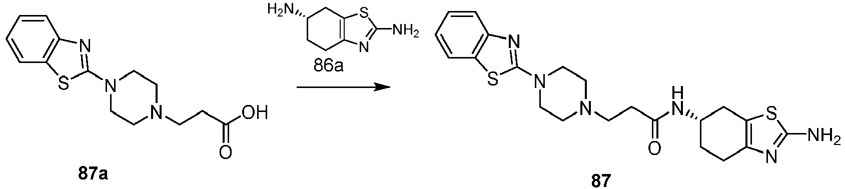

The title compound 87 (white solid) was prepared using the same synthetic route as in Step 3 of Example 40, except that 45B in Step 3 of Example 40 was replaced with 87a, and 1-(3,5-bis(trifluoromethyl)phenyl)piperazine (45c) was replaced with 86a.

LCMS: 443 [M+H]⁺

¹H NMR (400 MHz, DMSO-*d*₆) δ 8.09 (d, 1H), 7.75 (d, 1H), 7.44 (d, 1H), 7.26 (t, 1H), 7.06 (t, 1H), 6.63 (s, 2H), 4.06-3.96 (m, 1H), 3.54-3.46 (m, 4H), 2.73 (dd, 1H), 2.58 (t, 2H), 2.53-2.49 (m, 4H), 2.47-2.39 (m, 2H), 2.35 (dd, 1H), 2.27 (t, 2H), 1.84-1.76 (m, 1H), 1.74-1.63 (m, 1H).

### Example 78: synthesis of (S)-N⁶-(3-(4-(2,3-dichlorophenyl)piperazin-1-yl)propyl)-4,5,6,7-tetrahydrobenzo[d]thiazole-2,6-diami ne (compound 88)

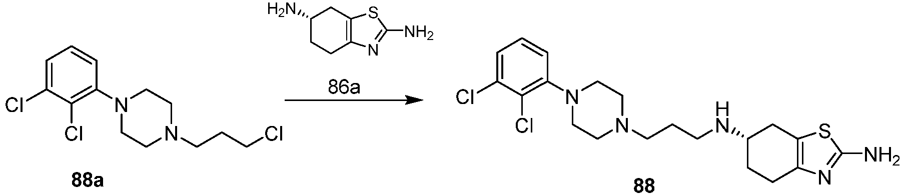

The title compound 88 (white solid) was prepared using the same synthetic route as in Step 2 of Example 76, except that 86A in Step 2 of Example 76 was replaced with 88a.

LCMS: 221 [M/2+H]⁺

¹H NMR (400 MHz, DMSO-*d*₆) δ 7.33-7.24 (m, 2H), 7.13 (dd, 1H), 6.57 (s, 2H), 2.94 (br, 4H), 2.83-2.76 (m, 1H), 2.74-2.68 (m, 1H), 2.60 (t, 2H), 2.50 (br, 4H), 2.47-2.29 (m, 5H), 2.24-2.18 (m, 1H), 1.90-1.83 (m, 1H), 1.60-1.45 (m, 3H).

### Example 79: synthesis of (S)-N-(2-amino-4,5,6,7-tetrahydrobenzo[d]thiazol-6-yl)-3-(4-(2,3-dichlorophenyl)piperazin-1-yl)prop anamide (compound 89)

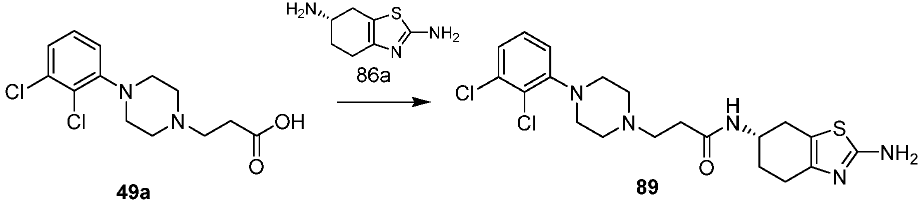

The title compound 89 (pale yellow solid) was prepared using the same synthetic route as in Step 3 of Example 40, except that 45B in Step 3 of Example 40 was replaced with 49a, and 1-(3,5-bis(trifluoromethyl)phenyl)piperazine (45c) was replaced with 86a.

LCMS: 454 [M+H]⁺

¹H NMR (400 MHz, CDCl₃) δ 9.04 (br, 1H), 7.19-7.14 (m, 2H), 7.04-7.00 (m, 1H), 4.72-4.50 (m, 3H), 2.97-2.90 (m, 2H), 2.73-2.47 (m, 10H), 2.44 (t, 2H), 2.08-1.98 (m, 1H), 1.92-1.82 (m, 1H).

### Example 80: synthesis of 3-((3-(4-(2,3-dichlorophenyl)piperazin-1-yl)propoxy)methyl)-2-methylimidazo[5,1-b]thiazole (compound 90)

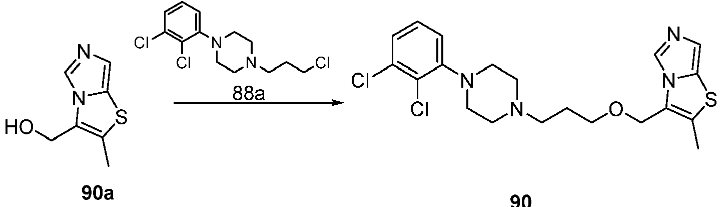

**90a** (200 mg, 1.19 mmol, 1.0 eq.) and DMF (2 mL) were added to a reaction flask, and the mixture was cooled to 0°C under nitrogen protection. 1-(3-Chloropropyl)-4-(2,3-dichlorophenyl)piperazine (88a) (364 mg, 1.19 mmol, 1.0 eq.) and potassium iodide (10 mg) were added under an ice bath, and the mixture was stirred at room temperature for 2 hours. The reaction was quenched with water, and extracted with 20 mL of EA. The organic phase was dried over anhydrous sodium sulfate, and concentrated under reduced pressure to obtain a crude product. The crude product was purified by column chromatography (eluent: DCM:1% ammonia methanol solution = 80:20) to obtain the title compound 90 (pale yellow solid) (98 mg, yield: 19%).

LCMS: 439 [M+H]⁺

¹H NMR (400 MHz, DMSO-*d*₆) δ 8.05 (s, 1H), 7.30-7.25 (m, 2H), 7.11 (d, 1H), 6.99 (s, 1H), 4.64 (s, 2H), 3.50 (t, 2H), 3.00-2.86 (m, 4H), 2.47-2.42 (m, 4H), 2.37-2.31 (m, 5H), 1.73-1.65 (m, 2H).

### Example 84: synthesis of 7-(4-((1,2,3,4-tetrahydroacridin-9-yl)amino)butoxy)-3,4-dihydroquinolin-2(1H)-one (compound 95)

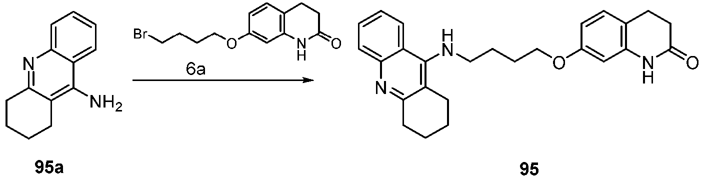

95a (600 mg, 3.0 mmol, 1.0 eq.) and N,N-dimethylformamide (10 mL) were added to a reaction flask, and sodium hydride (144 mg, 3.6 mmol, 1.2 eq.) was added. The reaction solution was reacted at 0°C for 30 minutes, then 7-(4-bromobutoxy)-3,4-dihydroquinolin-2 (1H)-one (6a) (900 mg, 3.0 mmol, 1.0 eq.) was added thereto, and the reaction was continued at room temperature for 1 hour. After LC-MS monitoring showed 40% of the starting material remained, the reaction was quenched with ice water, extracted with dichloromethane, dried, concentrated, and purified by normal-phase column chromatography (eluent: dichloromethane:methanol = 10:1) to obtain the title compound 95 (gray solid) (40 mg, yield: 10%).

LCMS: 416 [M+H]⁺

¹H NMR (400 MHz, CD₃OD) δ 8.39 (d, 1H), 7.83 (d, 1H), 7.74 (d, 1H), 7.52 (d, 1H), 7.01 (d, 1H), 6.43 (d, 1H), 6.35 (s, 1H), 4.07 (t, 2H), 4.02 (t, 2H), 2.97 (br, 2H), 2.87 (t, 2H), 2.67 (br, 2H), 2.54 (t, 2H), 2.07-2.00 (m, 2H), 1.96-1.88 (m, 6H).

### Example 85: synthesis of N-(2-((2,3-dichlorobenzyl)amino)ethyl)-2-((2-oxo-1,2,3,4-tetrahydroquinolin-7-yl)oxy)acetamide (compound 96)

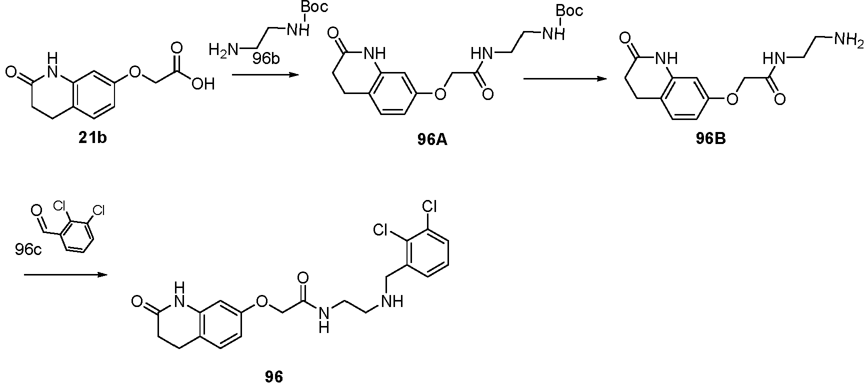

**Step 1:** White solid 96A (510 mg, yield: 62.0%) was prepared using the same synthetic route as in Example 18, except that 21a in Example 18 was replaced with 96b.

**Step 2: 96A** (500 mg, 1.38 mmol) was added to 4M HCl/1,4-dioxane (5 mL) and stirred at room temperature for 1 hour. The reaction solution was concentrated to obtain white solid **96B** (400 mg, yield: 97.0%).

**Step 3: 96B** (300 mg, 1.00 mmol), **96c** (176 mg, 1.00 mmol), and triethylamine (102 mg, 1.00 mmol) were added to MeOH (5 mL), stirred at room temperature for 0.5 hours, then NaBH₃CN (189 mg, 3.00 mmol) was added, and the reaction solution was stirred at 0°C for 16 hours. The reaction was quenched with a saturated sodium bicarbonate aqueous solution, and extracted with ethyl acetate. The organic phase was dried, filtered, and concentrated to obtain a crude product, which was purified by C18 reversed-phase column chromatography (eluent: methanol:0.1% aqueous formic acid = 75:25) to obtain the title compound 96 (white solid) (86 mg, yield: 20.3%).

LCMS: 422 [M+H]⁺

¹H NMR (400 MHz, DMSO-*d*₆) δ 10.06 (s, 1H), 8.17 (s, 1H), 8.06 - 8.03 (m, 1H), 7.54 - 7.47 (m, 2H), 7.36 - 7.32 (m, 1H), 7.04 (d, 1H), 6.49 - 6.48 (m, 2H), 4.39 (s, 2H), 3.8 (s, 2H), 3.28 - 3.24 (m, 2H), 2.77 (t, 2H), 2.44 (t, 2H), 2.40 (t, 2H).

### Example 86: synthesis of 7-(2-((2-((2,3-dichlorobenzyl)(methyl)amino)ethyl)amino)ethoxy)-3,4-dihydroquinolin-2(1H)-one (compound 97)

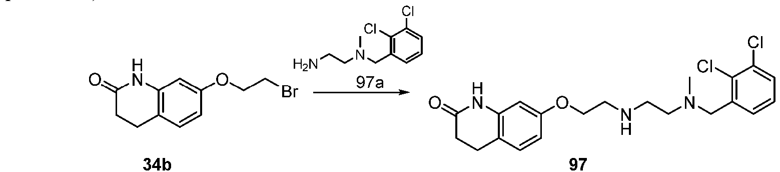

34b (2.7 g, 0.01 mol, 1 eq.), 97a (2.33 g, 0.01 mol, 1 eq.), potassium carbonate (2.76 g, 0.02 mol, 2 eq.), and acetonitrile (50 mL) were added to a 250 mL three-necked flask and refluxed at 90°C for 5 hours. After the reaction was complete, the mixture was separated by silica gel column chromatography (dichloromethane:methanol = 10:1), concentrated under reduced pressure to obtain a crude product, which was purified by preparative chromatography to obtain the title compound 97 (0.05 g, yield: 1.2%) as a white solid.

LCMS: 422 [M+1]⁺

¹H NMR (400 MHz, CD₃OD): δ 8.46 (s, 1H), 7.47 (m, 2H), 7.28 (t, 1H),7.06 (d, 1H), 6.59 (dd, 1H), 6.52 (d, 1H),4.26 (t, 2H), 3.74 (s, 2H), 3.47 (t, 2H), 3.32 (t, 2H), 2.88 (m, 4H), 2.50 (t, 2H), 2.28 (s, 3H).

### Example 87: synthesis of 7-(2-((2-((2,3-dichlorobenzyl)oxy)ethyl)(methyl)amino)ethoxy)-3,4-dihydroquinolin-2(1H)-one (compound 98)

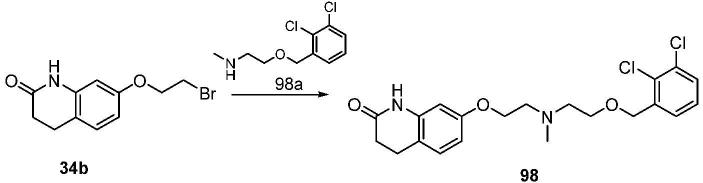

The title compound 98 (white solid) was prepared using the same synthetic route as in Example 86, except that 97a in Example 86 was replaced with 98a.

LCMS: 423 [M+1]⁺

¹H NMR (400 MHz, CD₃OD): δ 8.47 (s, 1H), 7.48 (t, 2H), 7.29 (t, 1H),7.08 (d, 1H), 6.60 (dd, 1H), 6.49 (d, 1H),4.67 (s, 2H), 4.28 (t, 2H), 3.90 (t, 2H), 3.47 (t, 2H), 3.37 (m, 2H), 2.87 (m, 5H), 2.54(t, 2H).

### Example 88: synthesis of 1-(3,4-dichlorophenylethyl)-3-(2-((2-oxo-1,2,3,4-tetrahydroquinolin-7-yl)oxy)ethyl)urea (compound 99)

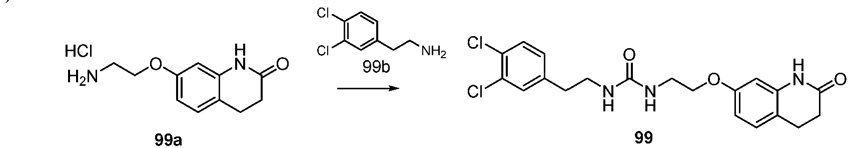

3,4-Dichlorophenethylamine (99b) (0.19 g, 1.0 mmol, 1.0 eq.), triphosgene (0.11 g, 0.37 mmol, 0.37 eq.), and dichloromethane (10 mL) were added to a reaction flask, and pyridine (0.173 g, 2.2 mmol, 2.2 eq.) was added dropwise under an ice bath. After the addition, the mixture was warmed to room temperature and stirred for 2 hours. **99a** (0.24 g, 1.0 mmol, 1.0 eq.) was dissolved in dichloromethane (5 mL), DIPEA (0.775 g, 6.0 mmol, 6.0 eq.) was added under an ice bath, and the above solution was added dropwise. After the dropwise addition, the mixture was stirred at room temperature for 2 hours, filtered, and the filter cake was purified with dichloromethane. The mixture was filtered and dried to obtain the title compound 99 (white solid) (0.16 g, yield: 37.9%).

LCMS: 422 [M+H]⁺

¹H NMR (400 MHz, DMSO-*d₆*) δ 9.99 (s, 1H), 7.51-7.45 (m, 2H), 7.17 (d, 1H), 7.03 (d, 1H), 6.48 (d, 1H), 6.42 (d, 1H), 6.08 (t, 1H), 5.96 (t, 1H), 3.83 (t, 2H), 3.30 (t, 2H), 3.22 (q, 2H), 2.77 (t, 2H), 2.67 (t, 2H), 2.40 (t, 2H).

### Example 89: synthesis of 7-(2-((4-(trifluoromethyl)benzyl)amino)ethoxy)-3,4-dihydroquinolin-2(1H)-one (compound 100)

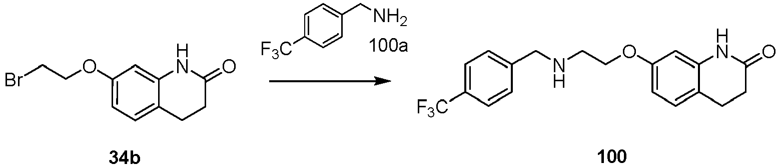

The title compound 100 (pale yellow solid) was prepared using the same synthetic route as in Example 7, except that 9a in Example 7 was replaced with 34b, and 1c was replaced with 100a.

LCMS: 365 [M+H]⁺

¹H NMR (400 MHz, CD₃OD) δ 7.68-7.62 (m, 4H), 7.08 (d, 1H), 6.59 (d, 1H), 6.51 (s, 1H), 4.14 (t, 2H), 4.08 (s, 2H), 3.13 (t, 2H), 2.87 (t, 2H), 2.53 (t, 2H).

### Example 90: synthesis of 7-(2-(((6-(trifluoromethyl)pyridin-3-yl)methyl)amino)ethoxy)-3,4-dihydroquinolin-2(1H)-one (compound 101)

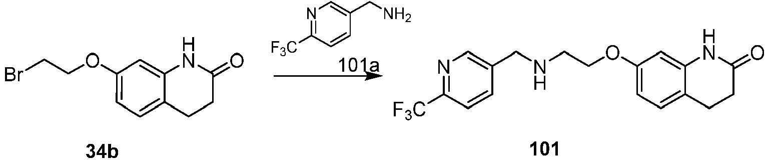

The title compound 101 (off-white solid) was prepared using the same synthetic route as in Example 7, except that 9a in Example 7 was replaced with 34b, and 1c was replaced with 101a.

LCMS: 366 [M+H]⁺

¹H NMR (400 MHz, CD₃OD) δ 8.83 (s, 1H), 8.18 (d, 1H), 7.88 (d, 1H), 7.10 (d, 1H), 6.62 (d, 1H), 6.52 (s, 1H), 4.32 (s, 2H), 4.22 (t, 2H), 3.37 (t, 2H), 2.88 (t, 2H), 2.54 (t, 2H).

### Example 91: synthesis of 7-(2-((2-(2-(2,3-dichlorophenyl)cyclopropyl)ethyl)amino)ethoxy)-3,4-dihydroquinolin-2(1H)-one (compound 102)

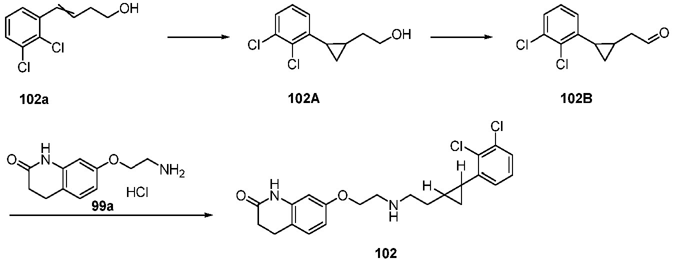

**Step 1:** Diiodomethane (2.68 g, 0.01 mol, 1.1 eq.) and dichloromethane (100 mL) were added to a 250 mL three-necked flask, cooled to -20°C, and diethylzinc (10 mL, 0.01 mol, 1.1 eq.) was added. After stirring for 30 minutes, the mixture was cooled to 0°C, and then trifluoroacetic acid (2.2 g, 0.02 mol, 2.2 eq.) was slowly added. The mixture was stirred at 0-10°C for 1 hour, then 102a (2.0 g, 0.009 mol, 1 eq.) was added and stirred at room temperature for 15 hours. The reaction was quenched with water (100 mL), extracted with dichloromethane, and the organic phase was purified by column chromatography (dichloromethane:methanol = 20:1), concentrated under reduced pressure to obtain white solid 102A (1.3 g, yield: 62.5%).

**Step 2:** 102A (1.3 g, 0.0056 mol, 1 eq.) and dichloromethane (50 mL) were added to a 250 mL three-necked flask, cooled to 0°C, and Dess-Martin periodinane (3.6 g, 0.0084 mol, 1.5 eq.) was added. The mixture was stirred at 0-10°C for 2 hours. After the reaction was complete, the reaction was quenched with water (100 mL), and extracted with dichloromethane. The organic phase was purified by column chromatography (ethyl acetate:petroleum ether = 1:5), and the resulting product was concentrated under reduced pressure to obtain white solid 102B (1.1 g, yield: 84.6%).

**Step 3:** 102B (0.46 g, 0.002 mol, 1 eq.), 99a (0.41 g, 0.002 mol, 1 eq.), sodium triacetoxyborohydride (2.1 g, 0.01 mol, 5 eq.), and dichloromethane (50 mL) were added to a 250 mL three-necked flask, cooled to 0°C, and reacted for 3 hours. After the reaction was complete, the reaction was quenched with saturated sodium bicarbonate solution, extracted with dichloromethane twice, the organic phase was dried over anhydrous sodium sulfate, concentrated, and purified by preparative liquid chromatography to obtain the title compound 102 (off-white solid) (0.04 g, yield: 4.8%).

LCMS: 419 [M+1]⁺

¹H NMR (400 MHz, CD₃OD): δ 7.33 (dd, 1H), 7.18 (t, 1H), 7.07 (d, 1H), 6.96 (dd, 1H), 6.57 (dd, 1H), 6.48 (d, 1H), 4.12 (t, 2H), 3.14 (t, 2H), 2.97 (m, 2H), 2.88 (t, 2H), 2.55 (t, 2H), 2.05 (m, 1H), 1.84 (m, 2H), 1.05 (m, 2H), 0.92 (d, 1H).

### Example 92: synthesis of 7-(2-(5-chloroindolin-1-yl)ethoxy)-3,4-dihydroquinolin-2(1H)-one (compound 103)

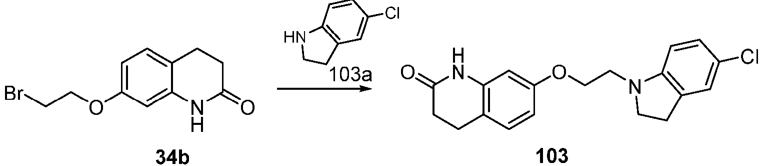

The title compound 101 (off-white solid) was prepared using the same synthetic route as in Example 86, except that 97a in Example 86 was replaced with 103a.

LCMS: 343 [M+1]⁺

¹H NMR (400 MHz, DMSO-*d₆*): δ 9.99 (s, 1H), 7.06-7.01 (m, 2H), 6.99 (d, 1H), 6.56-6.42 (m, 3H), 4.08 (t, 2H), 3.47-3.40 (m, 4H), 2.92 (t, 2H), 2.77 (t, 2H), 2.42 (t, 2H).

### Example 93: synthesis of N-(4-fluorobenzo[d]thiazol-2-yl)-4-((2-oxo-1,2,3,4-tetrahydroquinolin-7-yl)oxy)butanamide (compound 104)

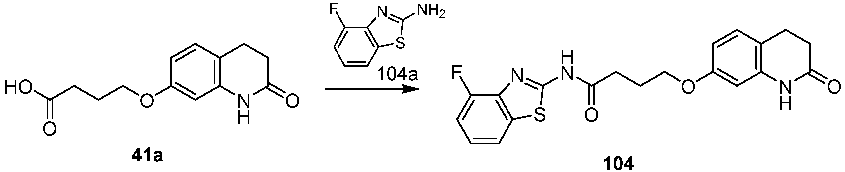

The title compound 104 (white solid) was prepared using the same synthetic route as in Step 3 of Example 40, except that 1-(3,5-bis(trifluoromethyl)phenyl)piperazine (45c) in Step 3 of Example 40 was replaced with 104a, and 45B was replaced with 41a.

LCMS: 400 [M+H]⁺

¹H NMR (400 MHz, DMSO-*d₆*) δ 12.61 (s, 1H), 9.96 (s, 1H), 7.79 (d, 1H), 7.32-7.22 (m, 2H), 7.01 (d, 1H), 6.45 (dd, 1H), 6.41 (d, 1H), 3.93 (t, 2H), 2.75 (t, 2H), 2.65 (t, 2H), 2.38 (t, 2H), 2.07-1.99 (m, 2H).

### Example 94: synthesis of 7-(4-((4-fluorobenzo[d]thiazol-2-yl)amino)butoxy)-3,4-dihydroquinolin-2(1H)-one (compound 105)

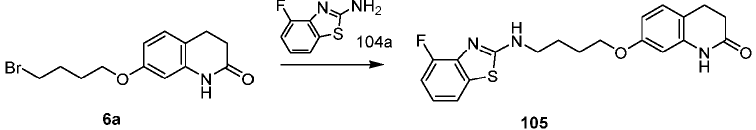

The title compound 105 (white solid) was prepared using the same synthetic route as in Example 7, except that 9a in Example 7 was replaced with 6a, and 1c was replaced with 104a.

LCMS: 386 [M+H]⁺

¹H NMR (400 MHz, DMSO-*d₆*) δ 9.97 (s, 1H), 8.25 (t, 1H), 7.49 (d, 1H), 7.10-6.97 (m, 3H), 6.48 (dd, 1H), 6.43 (d, 1H), 3.92 (t, 2H), 3.46-3.39 (m, 2H), 2.77 (t, 2H), 2.40 (t, 2H), 1.81-1.69 (m, 4H).

### Example 95: synthesis of 7-(2-((8-hydroxy-1,2,3,4-tetrahydronaphthalen-2-yl)amino)ethoxy)-3,4-dihydroquinolin-2(1H)-one (compound 107)

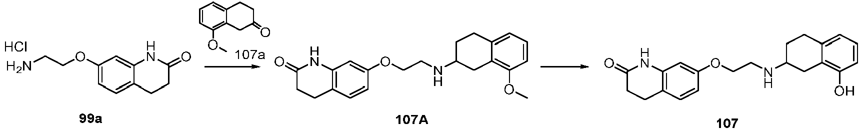

**Step 1: 99a** (1.1 g, 2.27 mmol, 1.0 eq), **107a** (1.04 g, 5.89 mmol, 1.3 eq), and TEA (459 mg, 4.53 mmol, 1.0 eq) were added to DCM (50 mL), followed by addition of NaBH(OAc)₃ (4.8 g, 22.66 mmol, 5.0 eq). The reaction solution was stirred at room temperature for 16 hours, diluted with water, adjusted to pH 7-8 with saturated aqueous NaHCO₃, and concentrated to remove DCM. The aqueous phase was extracted with ethyl acetate, the organic phase was dried, filtered, and concentrated to obtain a crude product, which was purified by normal-phase column chromatography (eluent: dichloromethane:methanol = 10:1). The target fraction was collected and lyophilized to obtain yellow solid **107A** (1.2 g, yield: 72.3%).

**Step 2: 107A** (1.2 g, 3.46 mmol, 1.0 eq) was added to DCM (50 mL), and a 2 M solution of boron tribromide in DCM (10 mL, 20.79 mmol, 10.0 eq) was added dropwise at 0°C. The reaction solution was stirred under nitrogen protection at 0°C for 4 hours, quenched with saturated aqueous NaHCO₃ to adjust pH to 7-8, and concentrated to remove dichloromethane. The aqueous phase was extracted with ethyl acetate, the organic phase was dried, filtered, and concentrated to obtain a crude product, which was purified by normal-phase column chromatography (eluent: dichloromethane:methanol = 10:1). The target fraction was collected and lyophilized to obtain the title compound 107 (yellow solid) (152 mg, yield: 13.2%).

LCMS: 353 [M+H]⁺

1H NMR (400 MHz, DMSO-*d*₆) δ 10.0 (s, 1H), 9.19 (s, 1H), 7.03 (d, 1H), 6.88 - 6.84 (m, 1H), 6.57 - 6.45 (m, 4H), 3.99 - 3.97 (m, 2H), 3.01 - 2.62 (m, 9H), 2.42 - 2.38 (m, 2H), 2.22 - 2.16 (m, 1H), 1.98 - 1.95 (m, 1H), 1.49 - 1.39 (m, 1H).

### Example 96: synthesis of 7-(2-((7-hydroxy-1,2,3,4-tetrahydronaphthalen-2-yl)amino)ethoxy)-3,4-dihydroquinolin-2(1H)-one (compound 108)

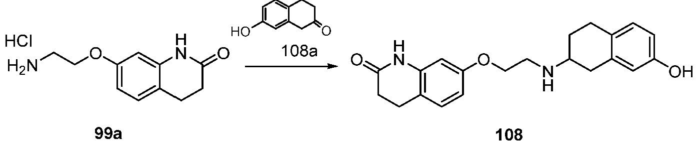

The title compound 108 (white solid) was prepared using the same synthetic route as in Step 1 of Example 95, except that 107a in Step 1 of Example 95 was replaced with 108a.

LCMS: 353 [M+H]⁺

H NMR (400 MHz, DMSO-*d*₆) δ 10.03 (s, 1H), 8.30 (s, 1H), 7.04 (d, 1H), 6.84 (d, 1H), 6.52 - 6.46 (m, 5H), 4.07 - 4.05 (m, 2H), 3.17 - 3.14 (m, 3H), 2.79 - 2.75 (m, 1H), 2.62 - 2.49 (m, 5H), 2.42 - 2.38 (m, 2H), 2.09 - 2.06 (m, 1H), 1.59 - 1.54 (m, 1H).

### Example 97: synthesis of 7-(3-(imidazo[1,2-a]pyridin-3-ylamino)propoxy)-3,4-dihydroquinolin-2(1H)-one (compound 109)

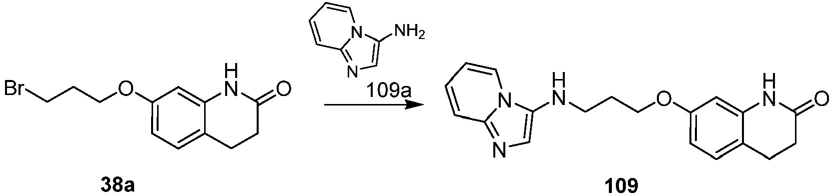

The title compound 109 (yellow oily liquid) was prepared using the same synthetic route as in Example 7, except that 9a in Example 7 was replaced with 38a, and 1c was replaced with 109a.

LCMS: 337 [M+H]⁺

¹H NMR (400 MHz, CD₃OD) δ 8.50 (d, 2H), 7.95 (d, 1H), 7.76 (d, 1H), 7.40 (t, 1H), 7.34 (s, 1H), 7.00 (d, 1H), 6.40 (d, 1H), 6.31 (s, 1H), 4.60 (t, 2H), 3.96 (t, 2H), 2.82 (t, 2H), 2.50 (t, 2H), 2.43-2.32 (m, 2H).

### Example 98: synthesis of 7-(4-(imidazo[1,2-a]pyridin-3-ylamino)butoxy)-3,4-dihydroquinolin-2(1H)-one (compound 110)

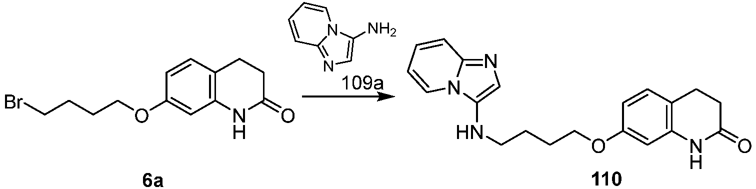

The title compound 110 (white solid) was prepared using the same synthetic route as in Example 7, except that 9a in Example 7 was replaced with 6a, and 1c was replaced with 109a.

LCMS: 351 [M+H]⁺

¹H NMR (400 MHz, DMSO-*d*₆) δ 10.04 (s, 1H), 8.57 (d, 1H), 8.13 (d, 1H), 7.83 (t, 1H), 7.45-7.30 (m, 2H), 7.02 (d, 1H), 6.52-6.34 (m, 2H), 4.42 (t, 2H), 3.90 (t, 2H), 2.76 (t, 2H), 2.44-2.33 (m, 2H), 2.03-1.84 (m, 2H), 1.76-1.58 (m, 2H).

### Example 99: synthesis of 7-((5-(imidazo[1,2-a]pyridin-3-ylamino)pentyl)oxy)-3,4-dihydroquinolin-2(1H)-one (compound 111)

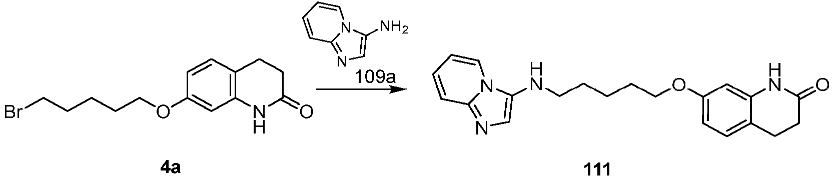

The title compound 111 (pale yellow oily liquid) was prepared using the same synthetic route as in Example 2, except that 3a in Example 2 was replaced with 4a, and 1c was replaced with 109a.

LCMS: 365 [M+H]⁺

¹H NMR (400 MHz, DMSO-*d*₆) δ 10.13 (s, 1H), 8.64 (d, 1H), 8.12 (d, 1H), 7.78 (t, 1H), 7.46-7.36 (m, 2H), 7.00 (d, 1H), 6.42-6.41 (m, 2H), 6.33 (br, 1H), 4.37 (t, 2H), 3.84 (t, 2H), 2.75 (t, 2H), 2.38 (t, 2H), 1.90-1.79 (m, 2H), 1.77-1.63 (m, 2H), 1.41-1.32 (m, 2H).

### Example 100: synthesis of 6-((2-((2-oxo-1,2,3,4-tetrahydroquinolin-7-yl)oxy)ethyl)amino)benzo[d]oxazol-2(3H)-one (compound 112)

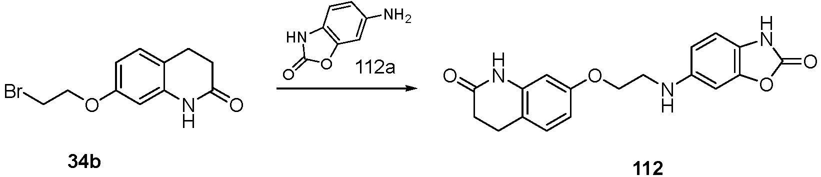

34b (0.54 g, 0.002 mol, 1 eq.), 6-amino-2-benzoxazolone (112a) (0.3 g, 0.002 mol, 1 eq.), potassium carbonate (0.52 g, 0.04 mol, 2.0 eq.), and DMF (15 mL) were added to a 250 mL three-necked flask, heated to 90°C, and reacted for 4 hours. After the reaction was complete, potassium carbonate was filtered off, and the filtrate was separated by column chromatography (dichloromethane:methanol = 10:1), concentrated under reduced pressure to obtain a crude product, which was purified by preparative liquid chromatography to obtain the title compound 112 (off-white solid) (0.074 g, yield: 10.9%).

LCMS: 340 [M+1]⁺

¹H NMR (400 MHz, DMSO-*d₆*): δ 9.95(s, 1H), 7.03 (d, 1H), 6.97 (d, 1H), 6.62 (d,1H), 4.47 (dd, 1H), 6.38 (d,1H), 6.29 (dd, 1H), 5.06 (s, 2H), 4.17 (t, 2H), 4.08 (t, 2H), 2.77 (t, 2H), 2.40 (t, 2H).

### Example 101: synthesis of N-(4-((2-oxo-1,2,3,4-tetrahydroquinolin-7-yl)oxy)butyl)-4H-thieno[3,2-b]pyrrole-5-carboxamide (compound 113)

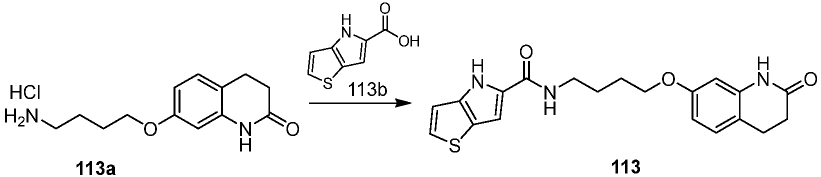

The title compound 113 (pale yellow solid) was prepared using the same synthetic route as in Example 36, except that 41a in Example 36 was replaced with 113a, and 40a was replaced with 113b.

LCMS: 384 [M+H]⁺

¹H NMR (400 MHz, DMSO-*d*₆) δ 11.65 (s, 1H), 9.96 (s, 1H), 8.22 (t, 1H), 7.34 (d, 1H), 7.04 (s, 1H), 7.01 (d, 1H), 6.93 (d, 1H), 6.46 (dd, 1H), 6.41 (d, 1H), 3.91 (t, 2H), 3.30 (q, 2H), 2.75 (t, 2H), 2.38 (t, 2H), 1.74-1.69 (m, 2H), 1.66-1.60 (m, 2H).

### Example 102: synthesis of N-((4-methyl-2-oxo-1,2,3,4-tetrahydroquinazolin-4-yl)methyl)-2-((2-oxo-1,2,3,4-tetrahydroquinolin-6-yl)oxy)acetamide (compound 114)

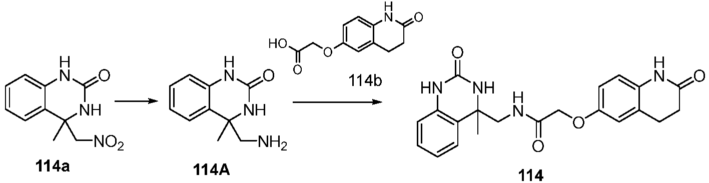

**Step 1:** 114a (0.4 g, 0.02 mol, 1.0 eq.), palladium/carbon (0.4 g, 1.0 eq.), and ethyl acetate (50 mL) were added to a 250 mL single-necked flask, purged with hydrogen balloon three times, and stirred at 10-20°C for 16 hours. After the reaction was complete, palladium/carbon was filtered off, and the filtrate was concentrated under reduced pressure to obtain white solid 114A (0.38 g, yield: 100%).

**Step 2:** 114A (0.44 g, 0.002 mol, 1 eq.), 114b (0.38 g, 0.002 mol, 1 eq.), HATU (0.76 g, 0.002 mol, 1 eq.), DIPEA (0.52 g, 0.004 mol, 2 eq.), and DMF (10 mL) were added to a 250 mL three-necked flask, cooled to 0°C, and reacted for 3 hours. After the reaction was complete, the mixture was washed with 100 mL of water, and extracted twice with dichloromethane. The organic phase was dried over anhydrous sodium sulfate, concentrated, and purified by preparative liquid chromatography to obtain the title compound 114 (off-white solid) (0.035 g, yield: 4.4%).

LCMS: 395 [M+1]⁺

¹H NMR (400 MHz, DMSO-*d₆*): δ 9.92(s, 1H), 9.17 (s, 1H), 7.63 (t, 1H), 7.13 (m, 2H), 6.85 (m,2H), 6.74 (m, 3H), 6.64 (dd, 1H), 4.36 (s, 2H), 3.46 (m,1H), 3.34 (m, 1H), 2.82 (t, 2H), 2.40 (t, 2H), 2.37 (s, 3H).

### Example 103: synthesis of 6-chloro-4-(3-((2-oxo-1,2,3,4-tetrahydroquinolin-6-yl)oxy)propyl)-3,4-dihydroquinoxalin-2(1H)-one (compound 115)

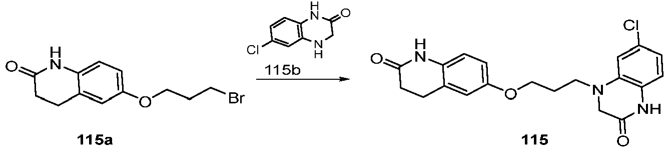

**115a** (300 mg, 1.64 mmol) and K₂CO₃ (681 mg, 4.92 mmol) were added to DMF (5 mL), followed by addition of **115b** (1.03 g, 3.6 mmol). The mixture was stirred at 50°C for 16 hours. The reaction solution was diluted with water, and extracted with ethyl acetate. The organic phase was dried over anhydrous sodium sulfate, filtered, and concentrated to obtain a crude product. The crude product was purified by normal-phase column chromatography (eluent: petroleum ether:ethyl acetate = 1:1) and then by C-18 reversed-phase column chromatography (eluent: methanol:0.1% aqueous formic acid = 80:20). The target fraction was collected and lyophilized to obtain the title compound 115 (white solid) (127 mg, yield: 20%).

LCMS: 386 [M+H]⁺

H NMR (400 MHz, DMSO-*d*₆) δ 9.91 (s, 1H), 7.00 (d, 1H), 6.75 - 6.67 (m, 5H), 6.32 (s, 1H), 3.99 - 3.97 (m, 4H), 3.79 (s, 2H), 2.82 - 2.79 (m, 2H), 2.40 - 2.36 (m, 2H), 1.96-1.85 (m, 2H).

### Example 104: synthesis of N-(2-((8-hydroxy-1,2,3,4-tetrahydronaphthalen-2-yl)amino)ethyl)-2-(3-oxo-3,4-dihydro-2H-benzo[b][ 1,4]oxazin-2-yl)acetamide (compound 130)

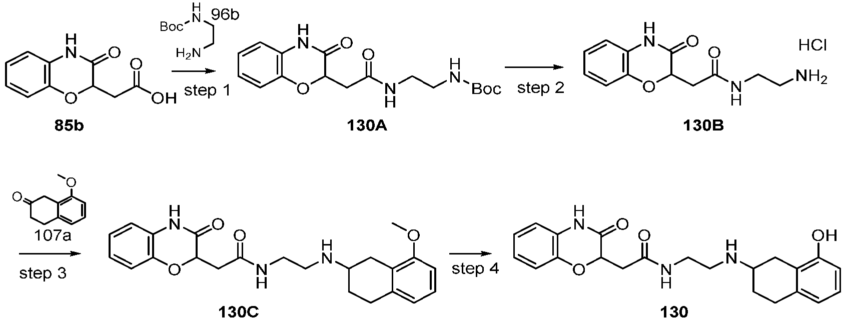

**Step 1: 85b** (1 g, 4.83 mmol), **96b** (1.16 g, 7.24 mmol), and DIEA (1.47 g, 14.5 mmol) were added to DCM (20 mL), followed by addition of HATU (2.7 g, 7.25 mmol). The mixture was stirred at room temperature for 3 hours. The reaction solution was filtered, and the filter cake was concentrated to obtain white solid 130A (770 mg, yield: 45.7%).

**Step 2: 130A** (770 mg, 2.20 mmol) was added to 4M HCl/1,4-dioxane (10 mL) and stirred at room temperature for 1 hour. The reaction solution was filtered, and the filter cake was concentrated to obtain gray solid **130B** (600 mg, yield: 95.3%).

**Steps 3-4:** The title compound 130 (white solid) was prepared using the same synthetic route as in Example 95, except that 99a in Example 95 was replaced with 130B.

LCMS: 396 [M+H]⁺

H NMR (400 MHz, DMSO-*d*₆) δ 10.7 (s, 1H), 8.38 - 8.32 (m, 2H), 6.94 - 6.87 (m, 5H), 6.59 (d, 1H), 6.51 (d, 1H), 4.91 - 4.88 (m, 1H), 3.38 - 3.23 (m, 2H), 3.10 - 3.00 (m, 2H), 2.94 - 2.84 (m, 2H), 2.78 - 2.61 (m, 4H), 2.49 - 2.45 (m, 1H), 2.35 - 2.28 (m, 1H), 2.06 - 2.03 (m, 1H), 1.56 - 1.52 (m, 1H).

### Example 105: synthesis of N-(2-((7-hydroxy-1,2,3,4-tetrahydronaphthalen-2-yl)amino)ethyl)-2-(3-oxo-3,4-dihydro-2H-benzo[b][ 1,4]oxazin-2-yl)acetamide (compound 131)

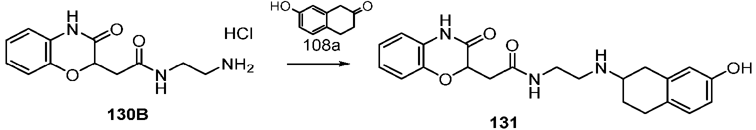

The title compound 131 (white solid) was prepared using the same synthetic route as in Example 95, except that 99a in Example 95 was replaced with 130B, and 107a was replaced with 108a.

LCMS: 396 [M+H]⁺

H NMR (400 MHz, DMSO-*d*₆) δ 10.71 (s, 1H), 8.28 (s, 2H), 6.95 - 6.83 (m, 5H), 6.51 - 6.49 (m, 1H), 6.45 (s, 1H), 4.91 - 4.88 (m, 1H), 3.33 - 3.22 (m, 2H), 3.04 - 2.54 (m, 10H), 2.04 - 2.01 (m, 1H), 1.57 - 1.46 (m, 1H).

### Example 106: synthesis of 5-(5,6-dimethoxy-2-oxoindolin-3-yl)-N-(4-isopropylbenzyl)pentanamide (compound 132)

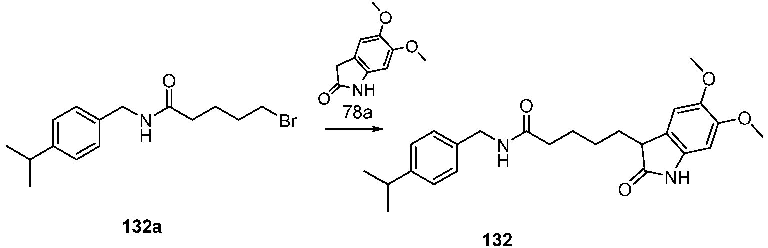

The title compound 132 (off-white solid) was prepared using the same synthetic route as in Example 7, except that 9a in Example 7 was replaced with 132a, and 1c was replaced with 78a.

LCMS: 425 [M+H]⁺

¹H NMR (400 MHz, DMSO-*d*₆) δ 10.57 (t, 1H),10.11 (s, 1H), 7.25 (s, 4H), 6.80 (s, 1H), 6.52 (s, 1H), 4.61 (d, 2H), 4.48 (t, 1H), 3.72 (s, 3H), 3.71 (s, 3H), 3.47-3.40 (m, 2H), 2.89-2.86 (m, 1H), 2.80-2.70 (m, 2H), 1.64-1.60 (m, 4H), 1.18-1.23 (d, 6H).

### Example 107: synthesis of (R)-7-(4-(3-((2,3-dichlorophenyl)(methyl)amino)pyrrolidin-1-yl)butoxy)-3,4-dihydroquinolin-2(1H)-o ne (compound 133)

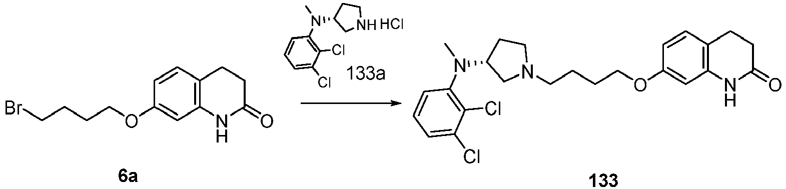

**133a** (0.38 g, 0.0013 mol, 1 eq.) was dissolved in DMF (5 mL), anhydrous potassium carbonate (0.54 g, 0.0039 mol, 3 eq.) was added with stirring, and the mixture was heated to 60°C. 7-(4-Bromobutoxy)-3,4-dihydroquinolin-2(1H)-one (6a) (0.39 g, 0.0013 mol, 1 eq.) was added at 60°C, and the reaction was maintained for 3 hours. After the reaction was complete, the mixture was cooled to room temperature, filtered, and the filter cake was washed with DMF. The filtrate was collected, added with a large amount of water, and extracted with ethyl acetate. The ethyl acetate phase was concentrated, and the crude product was preliminarily purified by column chromatography (eluent: dichloromethane:methanol = 1:1), followed by reversed-phase column chromatography (eluent: methanol:0.5% aqueous formic acid = 80:20). The resulting product fraction was lyophilized to obtain the title compound 133 (off-white solid) (0.04 g, yield: 6.7%).

LCMS: 462 [M+1]⁺

¹H NMR (400 MHz, CD₃OD): δ 8.43 (s, 1H), 7.33-7.24 (m, 3H), 7.07-7.05 (d, 1H), 6.55-6.53 (dd, 1H), 6.45-6.44 (d, 1H), 4.19-4.13 (m, 1H), 4.00-3.97 (t, 2H), 3.58-3.53 (m, 1H), 3.51-3.38 (m, 3H), 3.28-3.24 (t, 2H), 2.88-2.84 (t, 2H), 2.72 (s, 3H), 2.55-2.51 (t, 2H), 2.27-2.19 (m, 1H), 2.13-2.04 (m, 1H), 1.90-1.84 (m, 4H).

### Example 108: synthesis of 7-(2-(1-(4-(4-methoxybenzyl)-4H-thieno[3,2-b]pyrrole-5-carbonyl)piperidin-4-yl)ethoxy)-3,4-dihydro quinolin-2(1H)-one (compound 134)

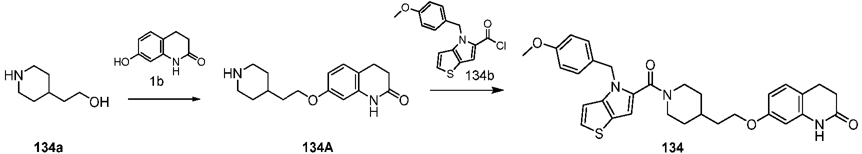

**Step 1:** 3,4-Dihydro-7-hydroxy-2(1H)-quinolinone (1b) (100 mg, 0.61 mmol, 1.0 eq.) was dissolved in anhydrous tetrahydrofuran (3 mL), and 4-piperidinylethanol (134a) (158 mg, 1.23 mmol, 2.0 eq.), diethyl azodicarboxylate (213 mg, 1.23 mmol, 2.0 eq.), and triphenylphosphine (321 mg, 1.23 mmol, 2.0 eq.) were added. The mixture was heated to 75°C under nitrogen protection overnight, concentrated under reduced pressure, and purified by reversed-phase column chromatography (eluent: methanol:0.1% aqueous formic acid = 70:30) to obtain pale yellow oily liquid **134A** (130 mg, yield: 77%).

**Step 2: 134A** (124 mg, 0.45 mmol, 1.0 eq.) was dissolved in dichloromethane (2 mL), added with triethylamine (137 mg, 1.35 mmol, 3.0 eq.), and the reaction was allowed to proceed at room temperature for 5 minutes. A solution of **134b** (138 mg, 0.45 mmol, 1.0 eq.) in dichloromethane (2 mL) was added dropwise, and the reaction was stirred at room temperature overnight. The mixture was concentrated under reduced pressure, and the residue was purified by reversed-phase column chromatography (eluent: methanol:0.1% aqueous formic acid = 80:20) to obtain the title compound 134 (white solid) (14 mg, yield: 6%).

LCMS: 544 [M+H]⁺

¹H NMR (400 MHz, CDCl₃) δ 8.25 (s, 1H), 7.17 (d, 1H), 7.09 (d, 2H), 7.05 (d, 1H), 6.91 (d, 1H), 6.79 (d, 2H), 6.54-6.46 (m, 2H), 6.33 (s, 1H), 5.40 (s, 2H), 4.40 (br, 2H), 3.94 (t, 2H), 3.75 (s, 3H), 2.90 (t, 2H), 2.87-2.73 (m, 2H), 2.62 (t, 2H), 1.80-1.55 (m, 5H), 0.90 (br, 2H).

### Example 109: synthesis of 7-(((3R,5R)-1-((R)-3-amino-3-(2,3-dichlorophenyl)propyl)-5-methylpiperidin-3-yl)methoxy)-3,4-dihy droquinolin-2(1H)-one (compound 135)

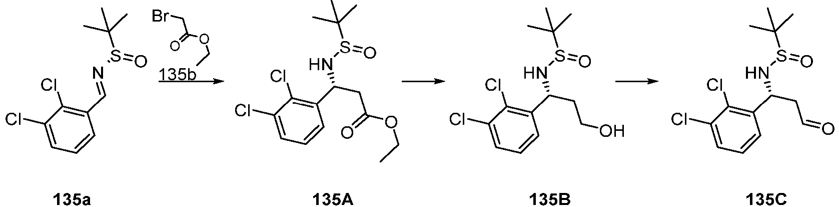

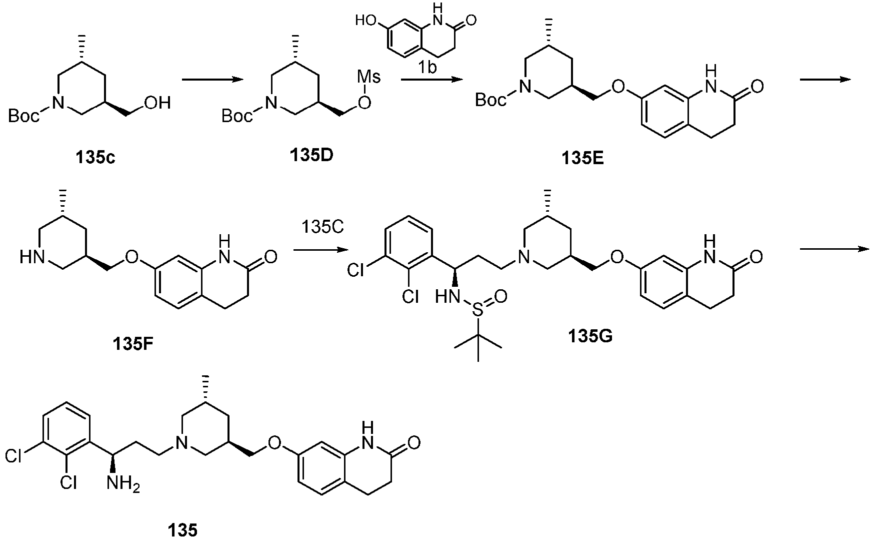

**Step 1:** Zinc powder (0.65 g, 0.01 mol, 1.0 eq.) and anhydrous THF (30 mL) were added to a 100 mL three-necked flask, and purged with nitrogen three times. Ethyl bromoacetate (135b) (1.67 g, 0.01 mol, 1.0 eq.) was added to the system via syringe, followed by addition of a THF (3 mL) solution of iodine. The mixture was heated to reflux, and a solution of 135a (1.78 g, 0.006 mol, 0.6 eq.) in anhydrous THF (15 mL) was added dropwise via syringe under reflux. After the dropwise addition, the reaction was maintained under reflux for 6 hours. After the reaction was complete, the reaction was quenched with water, and the crude product was purified by column chromatography (eluent: dichloromethane:methanol = 70:30). The target fraction was collected and concentrated to remove the solvent, thereby obtaining pale yellow gummy solid **135A** (1.26 g, yield: 34.3%).

**Step 2: 135A** (0.93 g, 0.00253 mol, 1.0 eq.) and anhydrous THF (10 mL) were added to a 100 mL three-necked flask, and was cooled in an ice-salt bath under nitrogen protection. After the mixture was cooled to below 0°C, a THF solution of lithium aluminum hydride (6.3 mL, 0.00253 mol, 1.0 eq.) was added dropwise via syringe. Obvious exothermic reaction occurs with the generation of a large amount of bubbles. The rate of the dropwise addition was controlled to regulate the temperature of the system. After the dropwise addition, the reaction was allowed to proceed for 1 hour. After the reaction was complete, the solid was filtered off, and the filtrate was concentrated to obtain oily substance **135B** (0.7 g, yield: 85.4%).

**Step 3: 135B** (0.7 g, 0.00216 mol, 1.0 eq.) and dichloromethane (10 mL) were added to a 100 mL single-necked flask, a Dess-Martin reagent (0.92 g, 0.00216 mol, 1.0 eq.) was added with stirring, and the reaction was allowed to proceed at room temperature for 1 hour. After the reaction was complete, the reaction solution was washed twice with saturated sodium bicarbonate solution. The layers were separated, and the DCM layer was dried over anhydrous sodium sulfate, filtered, and the filter cake was washed with DCM. The filtrate was concentrated to remove the solvent, thereby obtaining yellow oil **135C,** and the crude product was used directly in the next step.

**Step 4: 135c** (1.6 g, 0.007 mol, 1.0 eq.), triethylamine (2.08 g, 0.021 mol, 3 eq.), and DCM (10 mL) were added to a 100 mL three-necked flask, and the mixture was kept in an ice bath under nitrogen protection. After the system was cooled to 0°C , a DCM (2 mL) solution of methanesulfonyl chloride (0.96 g, 0.0084 mol, 1.2 eq.) was added dropwise via syringe, and the mixture was stirred overnight after the dropwise addition. After the reaction was complete, the solid was filtered off, and the filtrate was concentrated to obtain **135D,** the crude product was used directly in the next step.

**Step 5:** The crude **135D** from the previous step was dissolved in DMF (20 mL), and 7-hydroxy-3,4-dihydroquinolin-2(1H)-one (1b) (1.14 g, 0.007 mol, 1.0 eq.) and anhydrous potassium carbonate (2.9 g, 0.021 mol, 3 eq.) were added to the system. The mixture was reacted at 100°C for 2 hours. After the reaction was complete, the mixture was cooled to room temperature, added with a large amount of water, extracted with EA, concentrated, and the concentrate was preliminarily purified by column chromatography (eluent: dichloromethane:methanol = 80:20). The target fraction was collected and concentrated to obtain crude **135E** as a yellow solid (2.9 g, yield: 100%).

**Step 6: 135E** (2.9 g, 0.00774 mol, 1.0 eq.) and hydrochloric acid in dioxane solution (15 mL, 4 mol/L) were added to a 100 mL single-necked flask, and the reaction was allowed to proceed at room temperature for 1 hour. The mixture was filtered, the filter cake was washed with DCM, and dried to obtain white solid **135F** (0.93 g, yield: 38.8%).

**Step 7: 135F** (0.38 g, 0.00124 mol, 0.5 eq.), DCM (10 mL), and triethylamine (0.125 g, 0.00125 mol, 0.5 eq.) were added to a 100 mL single-necked flask, and the solid was dissolved with stirring. Then, **135C** (0.8 g, 0.00248 mol, 1.0 eq.) and acetic acid (0.177 g, 0.00496 mol, 2.0 eq.) were added. After stirring for 1 hour, sodium triacetoxyborohydride (1.05 g, 0.00496 mol, 2.0 eq.) was added, and the reaction was carried out at room temperature for 4 hours. After the reaction was complete, the mixture was filtered, the filter cake was washed with DCM, and the filtrate was washed with saturated sodium bicarbonate solution and water. The phases were separated, the organic phase was dried over anhydrous sodium sulfate, and the solvent was removed. The crude product was preliminarily purified by column chromatography (eluent: dichloromethane:methanol = 1:1). The target fraction was collected and concentrated to obtain off-white solid **135G** (0.57 g, yield: 91.9%).

**Step 8: 135G** (0.26 g, 0.000448 mol, 1.0 eq.) and EA (5 mL) were added to a 100 mL single-necked flask, and the solid was dissolved with stirring. A solution of hydrochloric acid in dioxane (0.6 mL) was added dropwise. After reacting at room temperature for 30 minutes, the mixture was filtered, the filter cake was washed with EA, and purified by preparative chromatography (eluent: methanol:0.5% aqueous formic acid = 80:20) to obtain the title compound 135 (white solid) (0.02 g, yield: 9.5%).

LCMS: 476 [M+1]⁺

¹H NMR (400 MHz, CD₃OD): δ 7.63-7.54 (m, 2H), 7.51-7.39 (m, 1H), 7.08-7.06 (m, 1H), 6.60-6.54 (m, 1H), 6.49-6.45 (m, 1H), 4.02-3.78 (m, 2H), 3.50 (d, 1H), 3.34 (s, 1H), 3.05 (m, 1H), 2.88-2.84 (t, 3H), 2.80-2.73 (m, 1H), 2.55-2.51 (t, 2H), 2.44-2.14 (m, 4H), 2.03-1.75 (m, 3H), 1.44-1.28 (m, 1H), 1.04-0.99 (m, 3H).

### Example 110: synthesis of 6-((4-(2,3-dichlorophenyl)piperazin-1-yl)sulfonyl)-3,4-dihydroquinolin-2(1H)-one (compound 158)

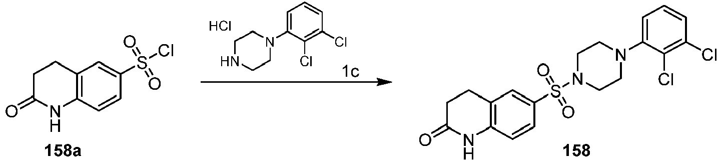

1-(2,3-Dichlorophenyl)piperazine hydrochloride (1c) (200 mg, 0.75 mmol, 1.0 eq.) was dissolved in dichloromethane (5 mL), and triethylamine (227 mg, 2.24 mmol, 3.0 eq.) was added, followed by slow addition of **158a** (220 mg, 0.90 mmol, 1.2 eq.). The reaction was allowed to proceed at room temperature for 3 hours. The mixture was concentrated under reduced pressure, and methanol (5 mL) was added to the residue with stirring. The mixture was filtered, the filter cake was washed with methanol (2×4 mL), and dried to obtain the title compound **158** (white solid) (215 mg, yield: 65.32%).

LCMS: 440 [M+H]⁺

¹H NMR (400 MHz, DMSO-*d₆*) δ 10.56 (s, 1H), 7.65-7.57 (m, 2H), 7.36-7.30 (m, 2H), 7.22-7.16 (m, 1H), 7.10 (d, 1H), 3.13-3.00 (m, 10H), 2.56 (t, 2H).

### Example 111: synthesis of 6-((4-hydroxy-4-(3-(trifluoromethyl)phenyl)piperidin-1-yl)sulfonyl)-3,4-dihydroquinolin-2(1H)-one (compound 159)

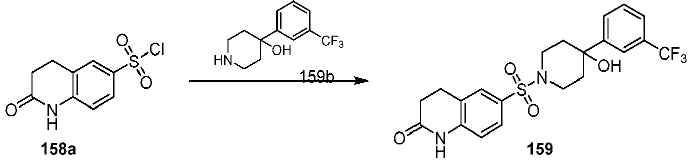

The title compound **159** (white solid) was prepared using the same synthetic route as in Example 110, except that 1c in Example 110 was replaced with 159b.

LCMS: 455 [M+H]⁺

¹H NMR (400 MHz, DMSO-*d₆*) δ 10.53 (s, 1H), 7.81-7.75 (m, 2H), 7.66-7.55 (m, 4H), 7.09 (d, 1H), 5.23 (s, 1H), 3.62-3.53 (m, 2H), 3.04 (t, 2H), 2.65 (t, 2H), 2.58-2.54 (m, 2H), 2.06 (td, 2H), 1.72-1.64 (m, 2H).

### Example 112: synthesis of 6-((4-(4-fluorobenzyl)piperidin-1-yl)sulfonyl)-3,4-dihydroquinolin-2(1H)-one (compound 160)

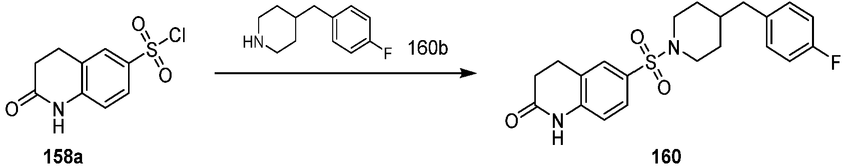

The title compound **160** (white solid) was prepared using the same synthetic route as in Example 110, except that 1c in Example 110 was replaced with 160b.

LCMS: 403 [M+H]⁺

¹H NMR (400 MHz, DMSO-*d₆*) δ 10.49 (s, 1H), 7.55-7.47 (m, 2H), 7.19-7.14 (m, 2H), 7.11-7.05 (m, 2H), 7.03 (d, 1H), 3.63-3.55 (m, 2H), 2.98 (t, 2H), 2.54-2.52 (m, 2H), 2.50-2.47 (m, 2H), 2.15 (t, 2H), 1.65-1.55 (m, 2H), 1.54-1.39 (m, 1H), 1.26-1.13 (m, 2H).

### Example 113: synthesis of 2-oxo-N-(2-oxo-2-(4-(3-(trifluoromethyl)phenyl)piperazin-1-yl)ethyl)-1,2,3,4-tetrahydroquinoline-6-s ulfonamide (compound 161)

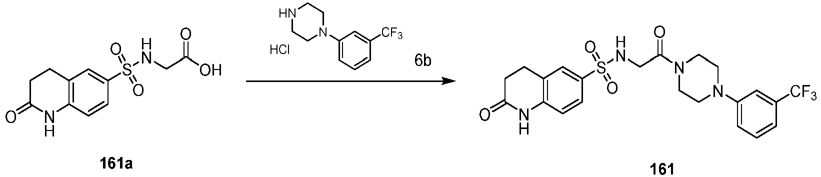

**161a** (100 mg, 0.35 mmol, 1.0 eq.) was dissolved in N,N-dimethylformamide (3 mL), and HATU (161 mg, 0.42 mmol, 1.2 eq.) was added. After reacting at room temperature for 0.5 hour, 1-(3-trifluoromethylphenyl)piperazine hydrochloride (6b) (113 mg, 0.42 mmol, 1.2 eq.) and DIPEA (168 mg, 1.30 mmol, 3.7 eq.) were added, and the reaction was allowed to proceed at room temperature overnight. The mixture was concentrated under reduced pressure, and the residue was purified by C18 reversed-phase column chromatography (eluent: 0.1% aqueous formic acid:methanol = 30:70) to obtain the title compound **161** (white solid) (100 mg, yield: 57.26%).

LCMS: 497 [M+H]⁺

¹H NMR (400 MHz, DMSO-*d₆*) δ 10.44 (s, 1H), 7.65 (s, 1H), 7.62 (dd, 1H), 7.57 (br, 1H), 7.45 (t, 1H), 7.24 (dd, 1H), 7.19 (s, 1H), 7.11 (d, 1H), 6.98 (d, 1H), 3.78 (s, 2H), 3.56-3.50 (m, 4H), 3.27-3.15 (m, 4H), 2.95 (t, 2H), 2.49 (t, 2H).

### Example 114: synthesis of N-(2-(4-(2,3-dichlorophenyl)piperazin-1-yl)-2-oxoethyl)-2-oxo-1,2,3,4-tetrahydroquinoline-6-sulfona mide (compound 162)

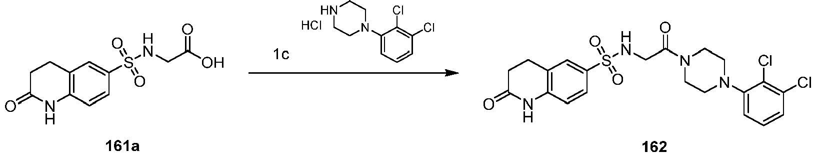

The title compound **162** (white solid) was prepared using the same synthetic route as in Example 113, except that 6b in Example 113 was replaced with 1c.

LCMS: 497 [M+H]+

1H NMR (400 MHz, DMSO-*d₆*) δ 10.46 (s, 1H), 7.66 (s, 1H), 7.62 (dd, 1H), 7.55 (t, 1H), 7.37-7.33 (m, 2H), 7.13 (dd, 1H), 6.99 (d, 1H), 3.77 (d, 2H), 3.57-3.50 (m, 4H), 2.99-2.88 (m, 6H), 2.49 (t, 2H).

### Example 115: synthesis of N-(2-(4-hydroxy-4-(3-(trifluoromethyl)phenyl)piperidin-1-yl)-2-oxoethyl)-2-oxo-1,2,3,4-tetrahydroqui noline-6-sulfonamide (compound 163)

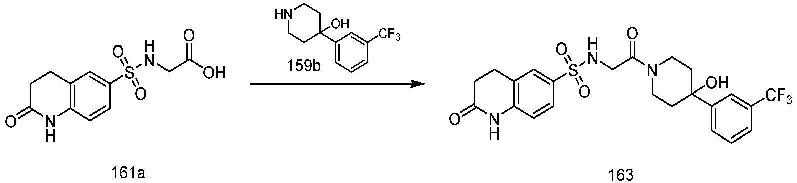

The title compound **163** (white solid) was prepared using the same synthetic route as in Example 113, except that 6b in Example 113 was replaced with 159b.

LCMS: 512 [M+H]⁺

¹H NMR (400 MHz, DMSO-*d₆*) δ 10.46 (s, 1H), 7.84 (s, 1H), 7.75 (d, 1H), 7.67 (d, 1H), 7.65-7.56 (m, 3H), 7.46 (s, 1H), 6.99 (d, 1H), 5.42 (s, 1H), 4.25 (d, 1H), 3.83-3.61 (m, 3H), 3.34-3.30 (m, 1H), 2.99-2.88 (m, 3H), 2.48 (t, 2H), 2.04-1.94 (m, 1H), 1.81-1.69 (m, 1H), 1.64-1.54 (m, 2H).

### Example 116: synthesis of N-(2-(4-(4-fluorobenzyl)piperidin-1-yl)-2-oxoethyl)-2-oxo-1,2,3,4-tetrahydroquinoline-6-sulfonamide (compound 164)

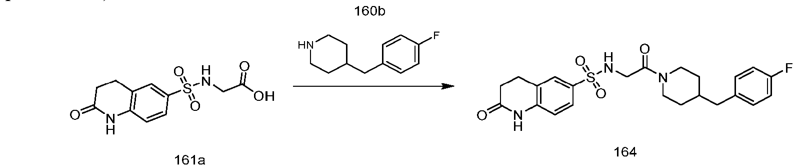

The title compound **164** (white solid) was prepared using the same synthetic route as in Example 114, except that 6b in Example 114 was replaced with 160b.

LCMS: 460 [M+H]⁺

¹H NMR (400 MHz, DMSO-*d₆*) δ 10.49 (s, 1H), 7.67-7.58 (m, 2H), 7.45 (s, 1H), 7.25-7.19 (m, 2H), 7.16-7.09 (m, 2H), 6.99 (d, 1H), 4.23 (d, 1H), 3.77-3.59 (m, 3H), 2.97 (t, 2H), 2.87 (t, 1H), 2.52-2.40 (m, 5H), 1.79-1.64 (m, 1H), 1.58-1.48 (m, 2H), 1.09-0.81 (m, 2H).

### Example 117: synthesis of 1-(6-(4-(3-(trifluoromethyl)phenyl)piperazine-1-carbonyl)-3,4-dihydroquinolin-1(2H)-yl)ethan-1-one (compound 165)

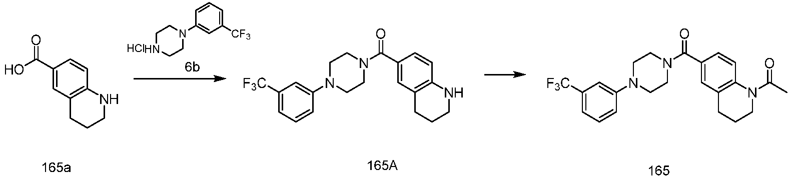

**Step 1:** 165a (0.10 g, 0.56 mmol, 1.0 eq.), 6b (0.17 g, 0.62 mmol, 1.1 eq.), HATU (0.26 g, 0.68 mmol, 1.2 eq.), DIPEA (0.27 g, 2.09 mmol, 3.7 eq.), and DMF (3 mL) were added to a reaction flask and stirred at room temperature for 1 hour. The mixture was concentrated to obtain a crude product, which was purified by C18 reversed-phase column chromatography (eluent: 0.5% aqueous formic acid:MeOH = 25:75). The target fraction was collected and concentrated to obtain off-white solid 165A (150 mg, yield: 68.8%).

**Step 2: 165A** (0.16 g, 0.41 mmol, 1.0 eq.), TEA (0.08 g, 0.82 mmol, 2.0 eq.), and DCM (6 mL) were added to a reaction flask, cooled to 0°C, and acetyl chloride (0.04 g, 0.45 mmol, 1.1 eq.) was added dropwise. After reacting for 1 hour, the mixture was purified by silica gel column chromatography (eluent: DCM:MeOH = 90:10) to obtain the title compound 165 (off-white solid) (166 mg, yield: 93.8%).

LCMS: 432 [M+H]⁺

¹H NMR (400 MHz, CDCl₃) δ 7.52-7.31 (m, 7H), 4.11-3.95 (m, 4H), 3.83 (t, 2H), 3.37 (s, 4H),2.82 (t, 2H), 2.31 (s, 3H), 2.07-2.00 (m, 2H).

### Example 118: synthesis of 1-(6-(4-(2,3-dichlorophenyl)piperazine-1-carbonyl)-3,4-dihydroquinolin-1(2H)-yl)ethan-1-one (compound 166)

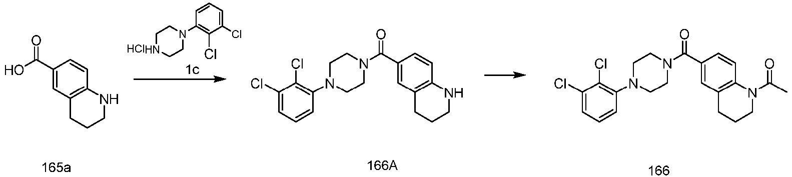

The title compound 166 (off-white solid) was prepared using the same synthetic route as in Example 117, except that 6b in Example 117 was replaced with 1c.

LCMS: 432 [M+H]⁺

¹H NMR (500 MHz, DMSO-*d₆*) δ 7.59 (s, 1H), 7.35-7.30 (m, 2H), 7.29-7.22 (m, 2H), 7.20-7.14 (m, 1H), 3.73 (m, 6H), 3.00 (br, 4H), 2.75 (t, 2H), 2.20 (s, 3H), 1.91-1.85 (m, 2H).

### Example 119: synthesis of 1-(6-(4-hydroxy-4-(3-(trifluoromethyl)phenyl)piperidine-1-carbonyl)-3,4-dihydroquinolin-1(2H)-yl)et han-1-one (compound 167)

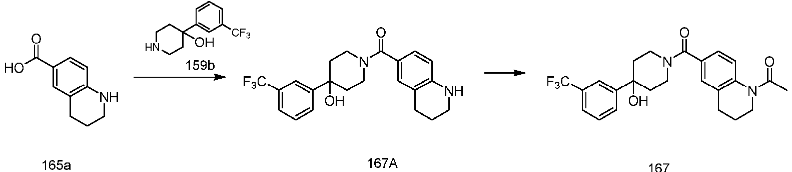

The title compound 167 (off-white solid) was prepared using the same synthetic route as in Example 117, except that 6b in Example 117 was replaced with 159b.

LCMS: 447 [M+H]⁺

¹H NMR (500 MHz, DMSO-*d₆*) δ 7.90 (s, 1H), 7.85 (d, 1H), 7.64-7.50 (m, 3H), 7.30-7.26 (m, 2H), 4.45 (s, 1H), 3.70(t, 2H), 3.64-3.44 (m, 2H), 3.24-3.04 (m, 2H), 2.75 (t, 2H), 2.20 (s, 3H), 2.00 (td, 2H), 1.92-1.85 (m, 2H), 1.68-1.58 (m, 2H).

### Example 120: synthesis of 1-(6-(4-(4-fluorobenzyl)piperidine-1-carbonyl)-3,4-dihydroquinolin-1(2H)-yl)ethan-1-one (compound 168)

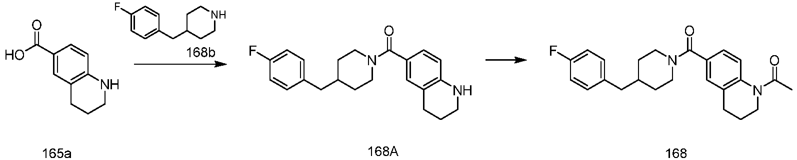

The title compound 168 (off-white solid) was prepared using the same synthetic route as in Example 118, except that 6b in Example 118 was replaced with 168b.

LCMS: 395 [M+H]⁺

¹H NMR (500 MHz, CDCl₃) δ 7.30-7.27 (m, 1H), 7.25-7.17 (m, 2H), 7.13-7.05 (m, 2H), 7.03-6.95 (m, 2H), 4.69 (s, 1H), 3.94-3.75 (m, 3H), 2.92-2.49 (m, 6H), 2.26 (s, 3H), 2.18-1.92 (m, 3H), 1.85-1.65 (m, 4H).

### Example 121: synthesis of N-(3-(4-(2,3-dichlorophenyl)piperazin-1-yl)-3-oxopropyl)-3,4-dimethoxybenzamide (compound 169)

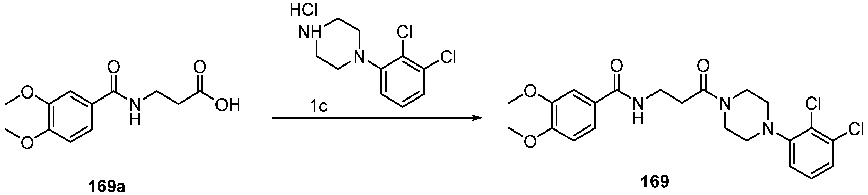

The title compound 169 (colorless oily liquid) was prepared using the same synthetic route as in Step 3 of Example 40, except that 1-(3,5-bis(trifluoromethyl)phenyl)piperazine (45c) in Step 3 of Example 40 was replaced with 1-(2,3-dichlorophenyl)piperazine hydrochloride (1c), and 45B was replaced with 169a.

LCMS: 466 [M+H]⁺

¹H NMR (400 MHz, DMSO-*d₆*) δ 8.43 (t, 1H), 7.48-7.41 (m, 2H), 7.34-7.26 (m, 2H), 7.05 (d, 1H), 7.00 (d, 1H), 3.79 (s, 3H), 3.78 (s, 3H), 3.64-3.58 (m, 4H), 3.49 (q, 2H), 2.92 (br, 4H), 2.63 (t, 2H).

### Example 122: synthesis of N-(4-(2,3-dichlorophenyl)piperazin-1-yl)-4-oxobutyl)-3,4-dimethoxybenzamide (compound 170)

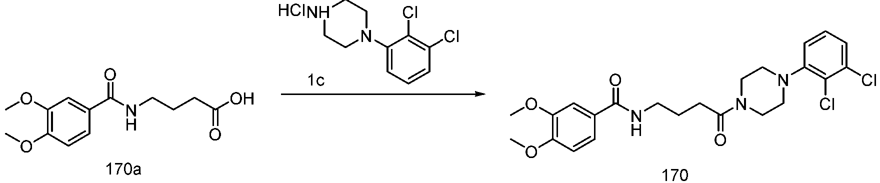

The title compound 170 (colorless oily liquid) was prepared using the same synthetic route as in Step 3 of Example 40, except that 1-(3,5-bis(trifluoromethyl)phenyl)piperazine (45c) in Step 3 of Example 40 was replaced with 1-(2,3-dichlorophenyl)piperazine hydrochloride (1c), and 45B was replaced with 170a.

LCMS: 480 [M+H]⁺

¹H NMR (400 MHz, DMSO-*d₆*) δ 8.34 (t, 1H), 7.47-7.42 (m, 2H), 7.35-7.26 (m, 2H), 7.13 (dd, 1H), 6.99 (d, 1H), 3.78 (s, 6H), 3.60 (br, 4H), 3.30 (q, 2H), 2.96-2.90 (m, 4H), 2.41 (t, 2H), 1.81-1.74 (m, 2H).

### Example 123: synthesis of N-(5-(4-(2,3-dichlorophenyl)piperazin-1-yl)-5-oxopentyl)-3,4-dimethoxybenzamide (compound 171)

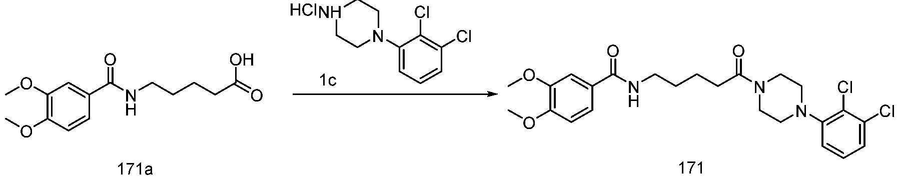

The title compound 171 (white solid) was prepared using the same synthetic route as in Step 3 of Example 40, except that 1-(3,5-bis(trifluoromethyl)phenyl)piperazine (45c) in Step 3 of Example 40 was replaced with 1-(2,3-dichlorophenyl)piperazine hydrochloride (1c), 45B was replaced with 171a, and the reagent DCM was replaced with DMF.

LCMS: 494 [M+H]⁺

¹H NMR (400 MHz, DMSO-*d₆*) δ 8.32 (t, 1H), 7.48-7.39 (m, 2H), 7.33-7.27 (m, 2H), 7.12 (dd, 1H), 6.98 (d, 1H), 3.78 (s, 6H), 3.59 (br, 4H), 3.26 (q, 2H), 2.95-2.86 (m, 4H), 2.38 (br, 2H), 1.55 (br, 4H).

### Example 124: synthesis of 3,4-dimethoxy-N-(5-oxo-5-(4-(3-(trifluoromethyl)phenyl)piperazin-1-yl)pentyl)benzamide (compound 172)

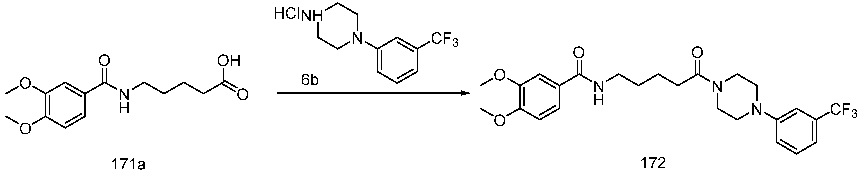

The title compound 172 (white solid) was prepared using the same synthetic route as in Step 3 of Example 40, except that 1-(3,5-bis(trifluoromethyl)phenyl)piperazine (45c) in Step 3 of Example 40 was replaced with 6b, 45B was replaced with 171a, and the reagent DCM was replaced with DMF.

LCMS: 494 [M+H]⁺

¹H NMR (400 MHz, DMSO-*d₆*) δ 8.32 (t, 1H), 7.46-7.40 (m, 3H), 7.22-7.15 (m, 2H), 7.08 (d, 1H), 6.98 (d, 1H), 3.78 (s, 6H), 3.61-3.55 (m, 4H), 3.26-3.18 (m, 6H), 2.38 (br, 2H), 1.54 (br, 4H).

### Example 125: synthesis of methyl 2-(4-(2,3-dichlorophenyl)piperazine-1-carbonyl)-4,5-dimethoxybenzoate (compound 173)

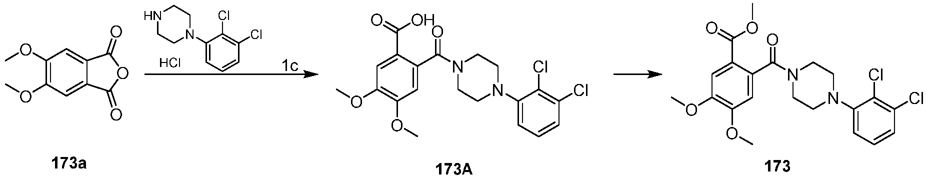

**Step 1: 173a** (200 mg, 0.96 mmol, 1.0 eq.) was dissolved in toluene (5 mL), and 1-(2,3-dichlorophenyl)piperazine hydrochloride (1c) (308 mg, 1.15 mmol, 1.2 eq.) was added. The reaction was heated to 100°C, and allowed to proceed overnight. The mixture was concentrated under reduced pressure, and purified by C18 reversed-phase column chromatography (0.1% aqueous formic acid:methanol = 70:30) to obtain yellow solid 173A (400 mg, yield: 95%).

**Step 2: 173A** (400 mg, 0.91 mmol, 1.0 eq.) was dissolved in N,N-dimethylformamide (5 mL), potassium carbonate (315 mg, 2.28 mmol, 2.5 eq.) was added, and the reaction was allowed to proceed at room temperature for 0.5 hour. Iodomethane (194 mg, 1.37 mmol, 1.5 eq.) was added, and the reaction was allowed to proceed at room temperature overnight. The reaction solution was purified by C18 reversed-phase column chromatography (0.1% aqueous formic acid:methanol = 30:70), and further purified by prep-TLC (eluent: petroleum ether:ethyl acetate = 1:1) to obtain the title compound 173 (pale yellow solid) (144 mg, yield: 35%).

LCMS: 453 [M+H]⁺

¹H NMR (400 MHz, DMSO-*d₆*) δ 7.46 (s, 1H), 7.36 (d, 2H), 7.19 (t, 1H), 6.95 (s, 1H), 3.89 (s, 3H), 3.87 (s, 3H), 3.85-3.77 (m, 5H), 3.27 (br, 2H), 3.10 (t, 2H), 2.93 (t, 2H).

### Example 126: synthesis of (R)-7-(2-hydroxy-3-((2-(2-methoxyphenoxy)ethyl)amino)propoxy)quinolin-2(1H)-one (compound 174)

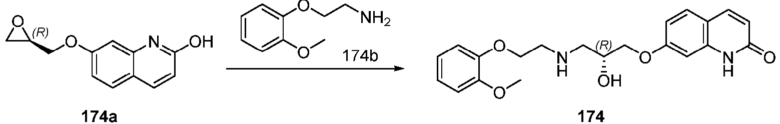

174a (868 mg, 4.0 mmol, 1.0 eq.) and ethanol (20 mL) were added to a reaction flask, followed by addition of 2-(2-methoxyphenoxy)ethylamine (668 mg, 4.0 mmol, 1.0 eq.). The reaction was stirred at 90°C in a sealed tube for 12 hours. After the reaction was complete, the reaction solution was concentrated to dryness, and directly purified by C18 reversed-phase column chromatography to obtain the title compound 174 (768 mg, yield: 50%).

LCMS: 385 [M+H]⁺

¹H NMR (400 MHz, DMSO-*d₆*) δ 11.58 (s, 1H), 7.79 (d, 1H), 7.54 (d, 1H), 6.95-6.73 (m, 6H), 6.29 (d, 1H), 4.06 (t, 2H), 4.00-3.94 (m, 3H), 3.73 (s, 3H), 3.01 (t, 2H), 2.90-2.88 (m, 1H), 2.81-2.72 (m, 1H).

### Example 127: synthesis of (S)-7-(3-((2-(3,4-dimethoxyphenoxy)ethyl)amino)-2-hydroxypropoxy)-3,4-dihydroquinolin-2(1H)-one (compound 175)

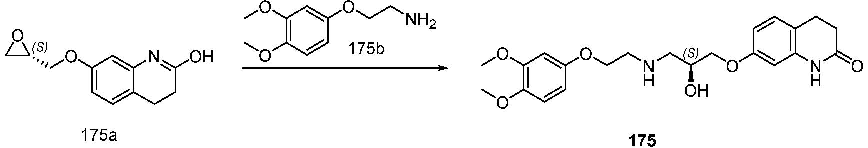

The title compound 175 was prepared using the same synthetic route as in Example 126, except that 174a in Example 126 was replaced with 175a, and 174b was replaced with 175b.

LCMS: 417 [M+H]⁺

¹H NMR (400 MHz, DMSO-*d₆*) δ 9.98 (s, 1H), 7.03 (d, 1H), 6.83 (d, 1H), 6.56 (s, 1H), 6.48-6.41 (m, 3H), 4.02 (t, 2H), 3.99-3.91 (m, 1H), 3.90-3.77 (m, 2H), 3.71 (s, 3H), 3.67 (s, 3H), 3.01 (t, 2H), 2.92-2.83 (m, 1H), 2.81-2.68 (m, 3H), 2.40 (t, 2H).

### Example 128: synthesis of N-(2-(7-hydroxynaphthalen-1-yl)ethyl)-3-((1-(pyridin-2-yl)piperidin-4-yl)amino)propanamide (compound 176)

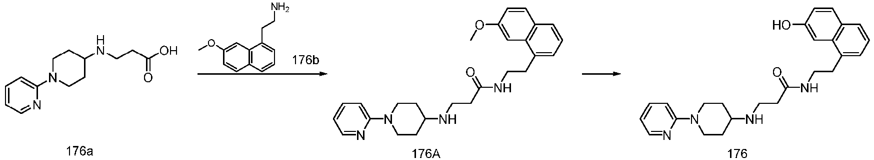

**Step 1:** 176a (0.25 g, 0.001 mol, 1 eq.), 2-(7-methoxynaphthalen-1-yl)ethan-1-amine (176b) (0.23 g, 0.001 mol, 1 eq.), HATU (0.76 g, 0.002 mol, 2 eq.), DIPEA (0.26 g, 0.002 mol, 2 eq.), and dichloromethane (50 mL) were added to a 250 mL three-necked flask, cooled to 0°C, and reacted for 2 hours. After the reaction was complete, the reaction was quenched with saturated sodium bicarbonate solution, and extracted twice with dichloromethane. The organic phase was dried over anhydrous sodium sulfate, concentrated, and purified by silica gel column chromatography (dichloromethane:methanol = 7:1) to obtain the product, which was concentrated to dryness to obtain off-white solid 176A (0.43 g, yield: 100%).

**Step 2:** 176A (0.6 g, 0.0015 mol, 1 eq.), dichloromethane (30 mL) and boron tribromide (7.5 mL, 0.015 mol, 10 eq.) were added to a 250 mL three-necked flask, and the reaction was allowed to proceed at 20°C for 5 hours. After the reaction was complete, the reaction was quenched with a saturated sodium bicarbonate solution, and extracted twice with dichloromethane. The organic phase was dried over anhydrous sodium sulfate, concentrated, and purified by preparative liquid chromatography to obtain the title compound 176 (off-white solid) (0.08 g, yield: 12.7%).

LCMS: 419 [M+1]⁺

¹H NMR (400 MHz, CD₃OD): δ 8.02-7.94 (m, 2H), 7.71 (d, 1H), 7.62 (d, 1H), 7.38-7.32 (m, 2H), 7.27 (d, 1H), 7.20 (t, 1H), 7.09 (d, 1H), 6.99 (t, 1H), 4.29 (d, 2H), 3.54 (t, 3H), 3.35-3.27 (m, 4H), 3.19 (t, 2H), 2.64 (t, 2H), 2.30 (d, 2H), 1.83-1.75 (m, 2H).

### Example 129: synthesis of 1-(4-(4-(((6-methyl-2-oxo-2,3-dihydro-1H-benzo[d]imidazol-5-yl)amino)methyl)phenoxy)butyl)-5-(2-( trifluoromethyl)phenyl)-1H-indazole-6-carbonitrile (compound 177)

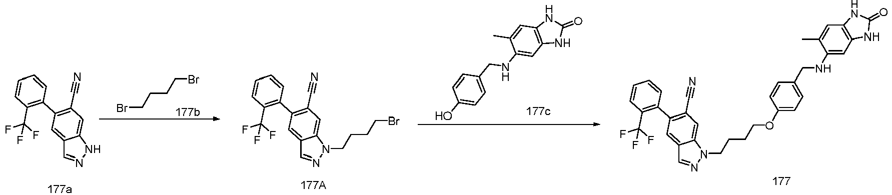

**Step 1:** 177a (0.5 g, 0.00174 mol, 1.0 eq.), 1,4-dibromobutane (177b) (0.75 g, 0.00348 mol, 2.0 eq.), potassium carbonate (0.72 g, 0.00522 mol, 3.0 eq.), and anhydrous acetonitrile (15 mL) were added to a 100 mL single-necked flask and heated to reflux for 20 minutes. The solvent was removed from the reaction solution, and the crude product was purified by column chromatography (eluent: DCM:MeOH = 100:0~70:30). The product fraction was collected and concentrated to remove the solvent, thereby obtaining yellow **oil 177A** (0.2 g, yield: 27.4%).

**Step 2: 177A** (0.2 g, 0.00048 mol, 1.0 eq.), **177c** (0.19 g, 0.000707 mol, 1.5 eq.), potassium carbonate (0.17 g, 0.0012 mol, 2.5 eq.), anhydrous DMF (5 mL), and water (1 mL) were added to a 50 mL single-necked flask and reacted at room temperature overnight. The mixture was filtered, the filter cake was washed with water, and the filtrate was purified by reversed-phase column chromatography (eluent: 0.5‰ aqueous formic acid:MeOH = 100:0~10:90). The product fraction was collected and lyophilized, and the product was further purified by preparative liquid chromatography (eluent: 0.5‰ aqueous formic acid:MeOH = 100:0~25:75) to obtain the title compound 177 (pale yellow solid) (0.03 g, yield: 10.3%).

LCMS: 611 [M+1]⁺

¹H NMR (400 MHz, DMSO-*d₆*): δ 10.06 (s, 1H), 10.01 (s, 1H), 8.60 (s, 1H), 8.28 (d, 1H), 7.93 (d, 1H), 7.87 (s, 1H), 7.82 (t, 1H), 7.75 (t, 1H), 7.54 (d, 1H), 7.24 (d, 2H), 6.85 (d, 2H), 6.59 (s, 1H), 6.03 (s, 1H), 5.09 (br, 1H), 4.66-4.54 (m, 2H), 4.22 (s, 2H), 3.97 (t, 2H), 2.12 (s, 3H), 2.07-1.99 (m, 2H), 1.75-1.68 (m, 2H).

### Example 130: synthesis of N-(2-(7-hydroxynaphthalen-1-yl)ethyl)-3-((6-(piperidin-1-yl)pyridin-3-yl)methoxy)propanamide (compound 178)

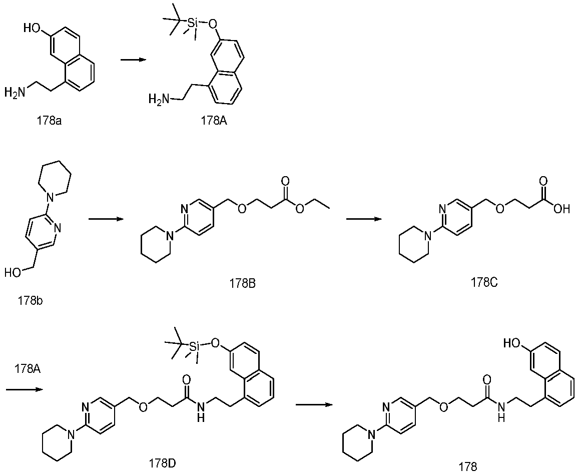

**Step 1:** 178a (1.0 g, 5.34 mmol, 1.0 eq), imidazole (0.726 g, 10.68 mmol, 2.0 eq), and DMF (10 mL) were added to a 100 mL single-neck flask, followed by addition of TBDMSCl (1.2 g, 8.02 mmol, 1.5 eq). The mixture was stirred at room temperature for 12 hours. The mixture was added with water, extracted with EA, concentrated, and purified with MTBE to obtain white solid **178A** (0.3 g, yield: 18.7%).

**Step 2:** 178b (2.5 g, 13 mmol, 1.0 eq), cesium carbonate (8.5 g, 26 mmol, 2.0 eq), ethyl acrylate (5.5 g, 65 mmol, 5.0 eq), and toluene (20 mL) were added to a 100 mL single-neck flask. The mixture was heated to 50°C for 12 hours, cooled, and filtered. The filtrate was concentrated to obtain a crude product, which was purified by silica gel column chromatography (eluent: DCM:MeOH = 30:1~10:1) to give pale yellow oily substance 178B (0.8 g, yield: 21%).

**Step 3:** 178B (800 mg, 2.74 mmol, 1.0 eq), methanol (10 mL), and water (2 mL) were added to a 100 mL single-neck flask, followed by addition of LiOH (197 mg, 8.22 mmol, 3.0 eq). The mixture was stirred at room temperature for 5 hours, concentrated to remove methanol, adjusted to pH 4 with dilute hydrochloric acid, filtered, and the filter cake was dried to obtain white solid **178C** (0.5 g, yield: 69%).

**Step 4:** 178C (200 mg, 0.76 mmol, 1.0 eq), 178A (274 mg, 0.9 mmol, 1.2 eq), HATU (578 mg, 1.52 mmol, 2 eq), DIEA (588 mg, 4.56 mmol, 6.0 eq), and DMF (10 mL) were added to a 100 mL single-necked flask and stirred at room temperature for 2 hours. The product was purified by reversed-phase column chromatography to obtain the title compound **178** (white solid) (0.052 g, yield: 12.5%).

LCMS: 434 [M+1]⁺

¹H NMR (400 MHz, DMSO-*d*₆) δ 9.71 (s, 1H), 8.00 (s, 2H), 7.74 (d,1 H), 7.62 (d, 1H), 7.41 (d, 1H), 7.30 (s, 1H), 7.21 (d, 1H), 7.17-7.11 (m, 1H), 7.07 (d, 1H), 6.74 (d, 1H), 4.28 (s, 2H), 3.57 (t, 2H), 3.47 (s, 4H), 3.35 (s, 2H), 3.03 (t, 2H), 2.32 (t, 2H), 1.65-1.40 (m, 6H).

### Experimental Example 1. Pharmacodynamic Experiment (TPK Enzyme Activity Test)

### 1.1 Experimental Purpose

The purpose of this example was to test the promoting effect of compounds on TPK enzyme activity and evaluate the in vitro activity of the compounds based on EC₅₀ and Eₘₐₓ.

### 1.2 Experimental Method

### 1.2.1 Experimental MaterialsTable 1

| **Reagent (Equipment) Name** | **Supplier Company** | **Catalog Number** |
|---|---|---|
| Tris-HCl (Trizma hydrochloride) | VETEC | V900312-500G |
| EDTA Disodium Salt | Sigma | 27285-500G-R |
| 2-Mercaptoethanol | Sigma | M6250-100ML |
| MgSO₄ | Sigma | 63136-250G-F |
| ATP | Sigma | A26209-5G |
| TM (Thiamine Hydrochloride) | Sigma | T4625-100G |
| NaOH | Sigma | S8045-500G |
| HCl | Sinopharm/Shanghai Reagent | 10011018 |
| 72% Perchloric Acid | Sinopharm | 20181207 |
| Distilled Water | Watson's | 400 mL/bottle |
| High-speed Low-temperature Centrifuge | eppendorf | 5430R |
| Constant-temperature Water Bath | Shanghai Bilon Instrument Co., Ltd. | DK-8D |
| High performance Liquid Chromatograph | Shimadzu | Agilent 1260 |

### 1.2.2 Experimental Procedures

1.1) The constant-temperature water-bath shaker was pre-heated half an hour in advance at a pre-heating temperature of 37 °C.
1.2) The stock solution of the compound to be tested was taken out, allowed to thaw at room temperature, and then diluted to the required concentration.
1.3) The required reagents were placed on ice to thaw.
2.1) For each reaction system, TPK enzyme solution, Tris-HCl buffer solution, and ATP solution were added. The concentration of ATP used was 1 - 500 mM. The above three solutions were formulated into a homogeneous mixture.
2.2) The mixture of the above three solutions was dispensed into centrifuge tubes. Then, the compound at the required concentration was added to each centrifuge tube. Finally, thiamine solution (1 - 100 µM) was added. After the addition of the above system was completed, the centrifuge tubes were tightly capped, inserted into a floating board, and placed in the constant-temperature water-bath shaker that had been pre-heated to 37 °C for incubation for 0.5 - 2.0 hours.
2.3) After the incubation was completed, perchloric acid stop solution was added and mixed well. Samples were taken into 1.5 mL centrifuge tubes and stored at -20 °C.
3.1) The samples stored at -20 °C were taken out, thawed at room temperature, and then subjected to derivatization treatment. The samples were placed in centrifuge tubes, a potassium ferricyanide derivatization reagent was added, and finally phosphoric acid stop solution was added to stop the reaction.
3.2) The derivatized samples were placed in sample vials for liquid chromatography analysis, and the contents of TDP/thiamine were detected by high performance liquid chromatography. TKP enzyme activity = TDP (nM)/mg protein/min.

### 1.3 Experimental Results

The effects of the compounds of the present invention on TPK enzyme activity at different concentrations were determined according to the above procedures. The measured Eₘₐₓ (calculated with the effect of DMSO on TPK enzyme activity as 100%) and EC₅₀ data are shown in the following Table 2.

**Table 2**

| Compound No. | Eₘₐₓ |
|---|---|
| 1 | 182.87 |
| 2 | 155.22 |
| 3 | 205.07 |
| 4 | 194.23 |
| 5 | 183.53 |
| 6 | 215.30 |
| 7 | 223.69 |
| 9 | 131.42 |
| 10 | 183.14 |
| 19 | 195.86 |
| 21 | 166.12 |
| 22 | 167.12 |
| 23 | 226.05 |
| 24 | 189.19 |
| 25 | 137.67 |
| 26 | 190.05 |
| 28 | 184.80 |
| 29 | 191.33 |
| 30 | 131.94 |
| 31 | 124.23 |
| 34 | 188.57 |
| 35 | 162.65 |
| 36 | 138.88 |
| 37 | 223.93 |
| 38 | 156.61 |
| 39 | 121.26 |
| 40 | 122.35 |
| 42 | 169.92 |
| 44 | 234.72 |
| 45 | 151.53 |
| 46 | 149.40 |
| 47 | 199.18 |
| 48 | 155.73 |
| 50 | 127.86 |
| 53 | 132.80 |
| 55 | 134.23 |
| 56 | 180.15 |
| 57 | 135.15 |
| 58 | 223.21 |
| 59 | 206.07 |
| 60 | 233.72 |
| 62 | 136.65 |
| 64 | 184.19 |
| 65 | 132.86 |
| 68 | 144.21 |
| 69 | 193.69 |
| 70 | 147.47 |
| 71 | 188.79 |
| 72 | 171.48 |
| 73 | 154.81 |
| 74 | 182.28 |
| 75 | 135.54 |
| 76 | 146.91 |
| 77 | 145.52 |
| 78 | 130.44 |
| 79 | 132.97 |
| 80 | 150.02 |
| 82 | 156.05 |
| 84 | 154.70 |
| 85 | 167.74 |
| 91 | 200.91 |
| 92 | 137.51 |
| 93 | 164.79 |
| 94 | 129.32 |
| 98 | 138.51 |
| 102 | 170.24 |
| 103 | 137.24 |
| 115 | 158.61 |
| 116 | 176.21 |
| 118 | 175.98 |
| 119 | 191.25 |
| 121 | 130.85 |
| 123 | 179.75 |
| 124 | 164.09 |
| 132 | 120.37 |
| 133 | 166.83 |
| 134 | 149.17 |
| 135 | 132.99 |
| 136 | 137.43 |
| 137 | 146.22 |
| 138 | 180.39 |
| 139 | 190.99 |
| 140 | 203.28 |
| 141 | 124.28 |
| 142 | 125.57 |
| 143 | 121.65 |
| 144 | 120.54 |
| 145 | 120.23 |
| 146 | 175.10 |
| 147 | 146.60 |
| 148 | 164.95 |
| 149 | 182.02 |
| 150 | 212.26 |
| 151 | 154.55 |
| 152 | 161.16 |
| 153 | 201.34 |
| 154 | 131.72 |
| 155 | 124.80 |
| 156 | 120.29 |
| 157 | 155.15 |
| 165 | 147.50 |
| 166 | 156.02 |
| 168 | 150.78 |
| 169 | 148.27 |
| 170 | 155.61 |
| 171 | 127.26 |
| 173 | 132.10 |
| 178 | 120.07 |

**Table 3**

| Compound No. | EC₅₀ (µM) |
|---|---|
| 1 | 1.159 |
| 2 | 0.46 |
| 4 | 1.043 |
| 5 | 1.201 |
| 6 | 1.27 |
| 7 | 1.176 |
| 10 | 0.13 |
| 19 | 5.012 |
| 21 | 1.062 |
| 22 | 1.189 |
| 23 | 1.009 |
| 25 | 0.7542 |
| 26 | 1.221 |
| 28 | 1.543 |
| 29 | 2.284 |
| 34 | 1.302 |
| 35 | 1.369 |
| 36 | 1.318 |
| 37 | 1.17 |
| 38 | 1.18 |
| 42 | 1.004 |
| 44 | 1.141 |
| 45 | 1.07 |
| 46 | 1.312 |
| 47 | 4.646 |
| 48 | 3.798 |
| 58 | 1.202 |
| 59 | 1.223 |
| 60 | 1.279 |
| 62 | 0.9176 |
| 64 | 4.708 |
| 69 | 2.724 |
| 71 | 1.193 |
| 72 | 3.236 |
| 73 | 0.65 |
| 74 | 12.1 |
| 75 | 0.22 |
| 76 | 3.46 |
| 80 | 2.55 |
| 82 | 2.52 |
| 84 | 3.864 |
| 91 | 25.78 |
| 93 | 1.453 |
| 102 | 12.45 |
| 103 | 17.25 |
| 118 | 2.66 |
| 119 | 1.132 |
| 121 | 5.6 |
| 123 | 1.201 |
| 124 | 1.107 |
| 133 | 10.77 |
| 136 | 2.795 |
| 137 | 109.8 |
| 138 | 1.01 |
| 139 | 8.086 |
| 140 | 8.746 |
| 146 | 1.337 |
| 148 | 1.234 |
| 150 | 0.1391 |
| 151 | 3.359 |
| 152 | 9.635 |
| 153 | 7.862 |
| 157 | 2.605 |
| 165 | 9.799 |
| 166 | 4.249 |
| 168 | 5.431 |
| 169 | 7.23 |
| 170 | 4.39 |

Various modifications of the invention in addition to those described herein will become apparent to those skilled in the art from the foregoing description. Such modifications are intended to fall within the scope of the appended claims. Each reference, including all patents, applications, journal articles, books and any other disclosure, referred to herein is hereby incorporated by reference in its entirety.

## Claims

1. A method for the prophylaxis or treatment of neurodegenerative diseases or alleviating symptoms of neurodegenerative diseases, which comprises administering to a subject in need thereof a prophylactically or therapeutically effective amount of a thiamine pyrophosphokinase (TPK) agonist;
wherein the TPK agonist is a compound of Formula (I), or a pharmaceutically acceptable salt, ester, stereoisomer, polymorph, solvate, N-oxide, isotopically labeled compound, metabolite, or prodrug thereof:
A-L-B (I)
wherein:
A and B are each independently C₃₋₁₀ hydrocarbon ring, 3- to 14-membered heterocycle, C₆₋₁₀ aromatic ring or 5- to 14-membered heteroaromatic ring;
L is selected from the group consisting of -Q¹-, -W-, -Q¹-W-, -W-Q¹-, -Q¹-Q²-, -W-W'-, -W-Q¹-Q²-, -W-Q¹-W'-, -Q¹-W-Q²-, -Q¹-W-Q²-W'-, -W-Q¹-W'-Q²-, -Q¹-Q²-W-W'- and -W-Q¹-Q²-W'-;
Q¹ and Q² are each independently selected from the group consisting of -C₁₋₆ alkylene-, -C₂₋₆ alkenylene-, -C₂₋₆ alkynylene-, -C₃₋₁₀ cyclic hydrocarbylene-, -(3- to 14-membered heterocyclylene)-, -C₆₋₁₀ arylene- and -(5- to 14-membered heteroarylene)-, wherein the alkylene, alkenylene and alkynylene are each optionally interrupted by one group selected from the following or interrupted by multiple adjacent or non-adjacent groups independently selected from the following: -C₃₋₁₀ cyclic hydrocarbylene-, -(3- to 14-membered heterocyclylene)-, -C₆₋₁₀ arylene-, -(5- to 14-membered heteroarylene)-, -O-, -C(=O)-, -C(=O)O-, -NR-, -C(=O)NR-, -NR-C(=O)-NR'-, -NR-C(=O)O-, -(S=O)NR-, -S(=O)₂NR-, -S-, -S(=O)- and -S(=O)₂-;
W and W', at each occurrence, are each independently selected from the group consisting of -O-, -C(=O)-, -C(=O)O-, -NR-, -C(=O)NR-, -NR-C(=O)-NR'-, -NR-C(=O)O-, -(S=O)NR-, -S(=O)₂NR-, -S-, -S(=O)- and -S(=O)₂-;
R and R', at each occurrence, are each independently selected from the group consisting of H, C₁₋₆ alkyl, C₂₋₆ alkenyl, C₂₋₆ alkynyl, C₃₋₁₀ cyclic hydrocarbyl, 3- to 14-membered heterocyclyl, C₆₋₁₀ aryl, 5- to 14-membered heteroaryl and C₆₋₁₂ aralkyl;
the above alkyl, alkylene, alkenyl, alkenylene, alkynyl, alkynylene, cyclic hydrocarbyl, cyclic hydrocarbylene, hydrocarbon ring, heterocyclyl, heterocyclylene, heterocycle, aryl, arylene, aromatic ring, heteroaryl, heteroarylene, heteroaromatic ring and aralkyl, at each occurrence, are each optionally substituted with one or more substituents independently selected from the group consisting of: halogen, -OH, =O, -NH₂, -CN, -NO₂, C₁₋₆ alkyl, haloC₁₋₆ alkyl, C₃₋₁₀ cyclic hydrocarbyl, 3- to 14-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-OR^{a}, -C₁₋₆ alkylene-NR^{a}R^{b} and -O-C₁₋₆ alkylene-NR^{a}R^{b}, the alkyl, alkylene, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₋₆ haloalkyl, C₃₋₁₀ cyclic hydrocarbyl, 3- to 14-membered heterocyclyl, C₆₋₁₀ aryl, 5- to 14-membered heteroaryl, C₆₋₁₂ aralkyl, -O-C₁₋₆ alkyl and -C₁₋₆ alkylene-O-C₁₋₆ alkyl; and
R^{a} and R^{b}, at each occurrence, are each independently selected from the group consisting of H, C₁₋₆ alkyl, C₃₋₁₀ cyclic hydrocarbyl, 3- to 14-membered heterocyclyl, C₆₋₁₀ aryl, 5- to 14-membered heteroaryl and C₆₋₁₂ aralkyl, the alkyl, cyclic hydrocarbyl, heterocyclyl, aryl, heteroaryl and aralkyl are further optionally substituted with one or more substituents independently selected from the group consisting of: halogen, -OH, =O, -C(=O)O-tert-butyl, -NH₂, -CN, -NO₂, C₁₋₆ alkyl, C₁₋₆ haloalkyl, C₃₋₁₀ cyclic hydrocarbyl, 3- to 14-membered heterocyclyl, C₆₋₁₀ aryl, 5- to 14-membered heteroaryl, C₆₋₁₂ aralkyl and -C₁₋₆ alkylene-O-C₁₋₆ alkyl;
preferably, the neurodegenerative disease is Alzheimer's disease;
more preferably, the Alzheimer's disease is one in which the subject has decreased TPK enzyme activity, decreased TPK expression level, and/or decreased TDP level.

2. The method according to claim 1, wherein A is

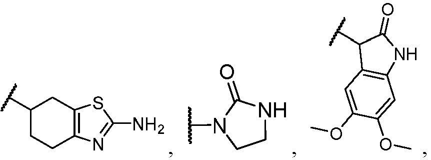

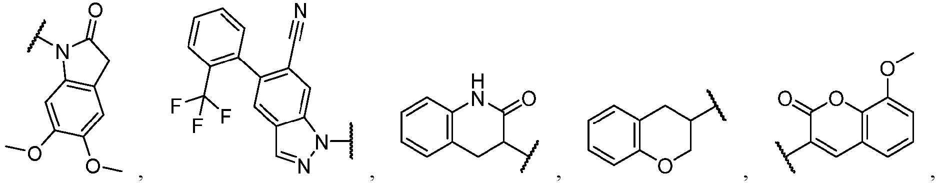

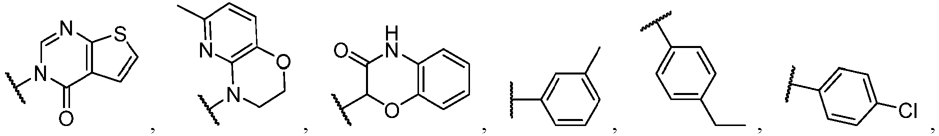

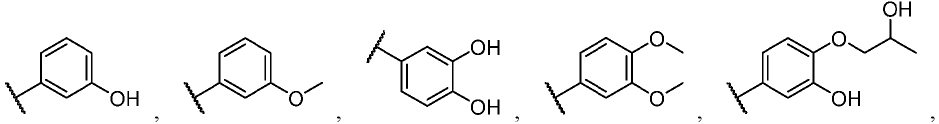

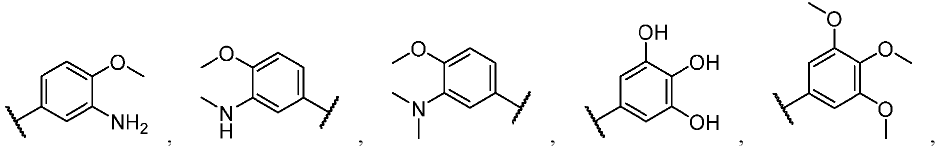

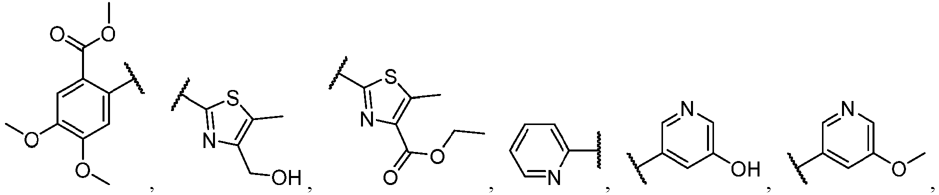

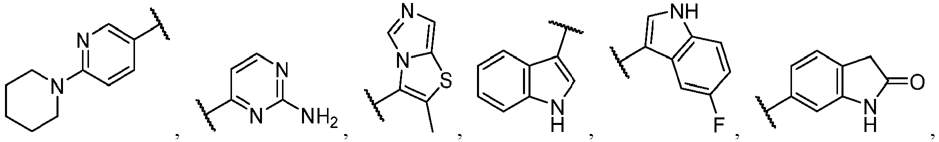

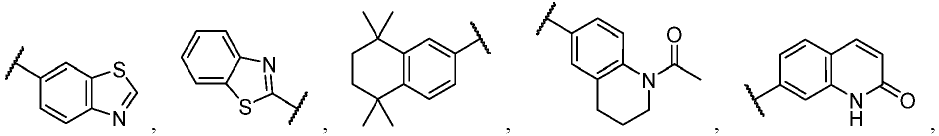

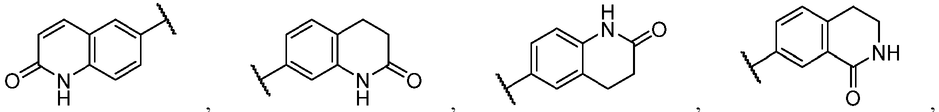

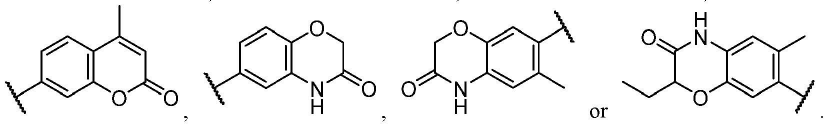

3. The method according to claim 1 or 2, wherein L is selected from the group consisting of -Q¹-W-, -W-Q¹-, -Q¹-Q²-, -W-Q¹-Q²-, -W-Q¹-W'-, -Q¹-W-Q²-, -Q¹-W-Q²-W'-, -W-Q¹-W'-Q²-, -Q¹-Q²-W-W'- and -W-Q¹-Q²-W'-;
preferably, L is

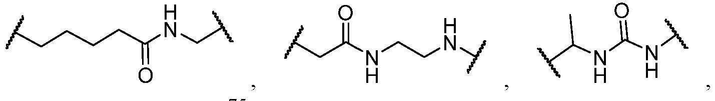

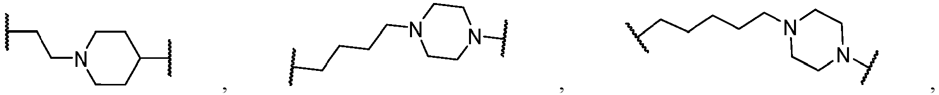

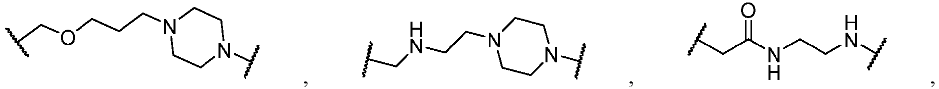

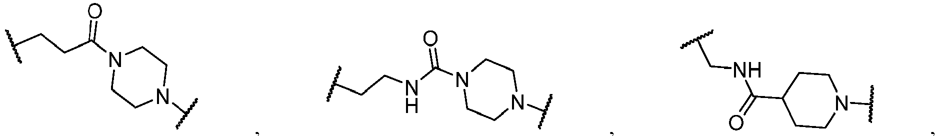

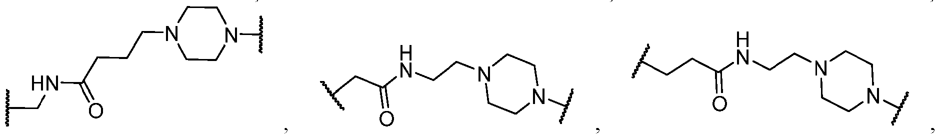

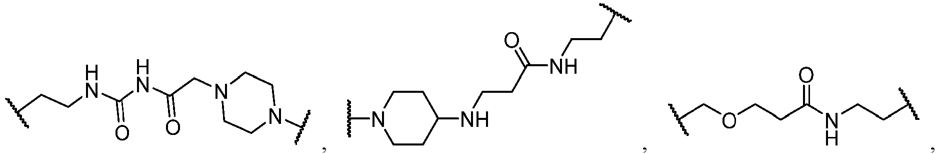

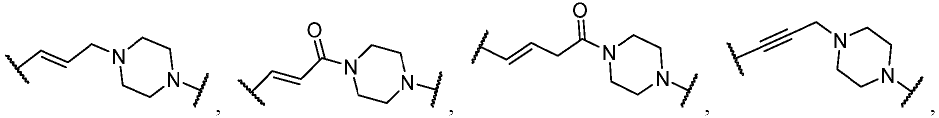

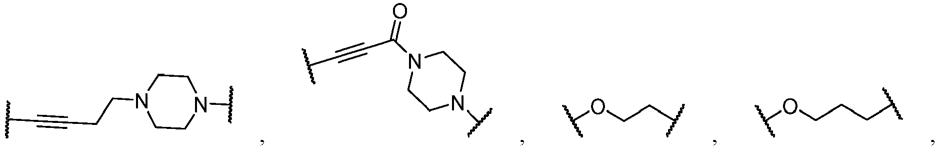

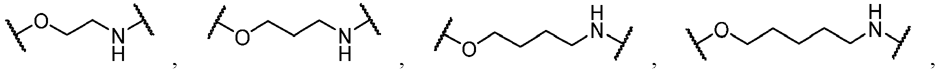

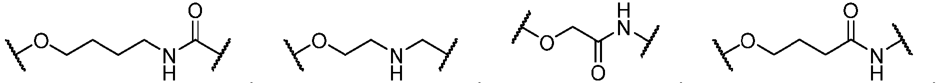

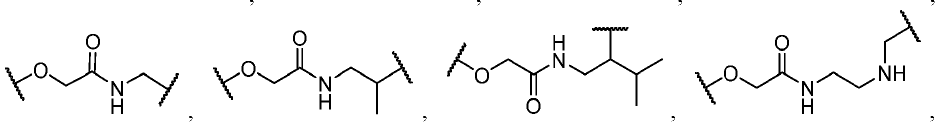

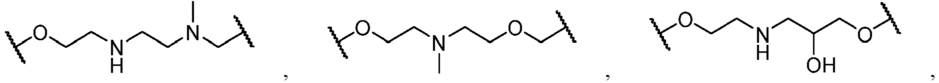

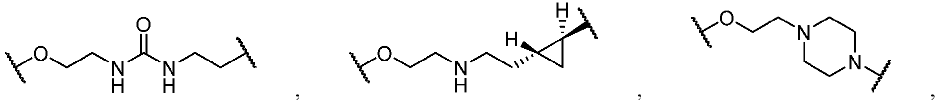

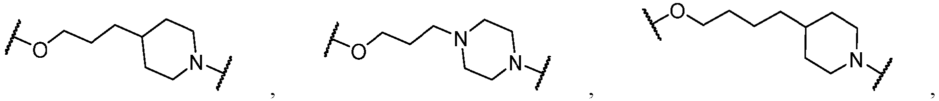

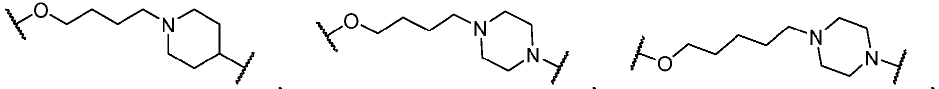

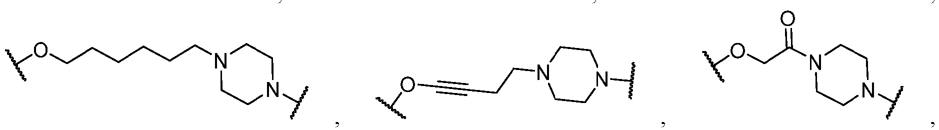

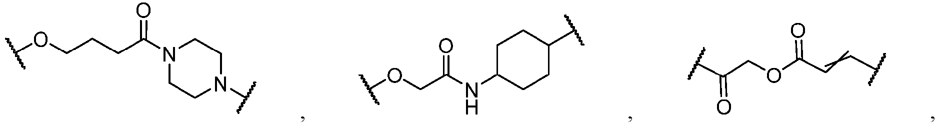

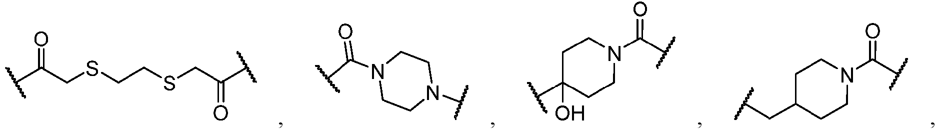

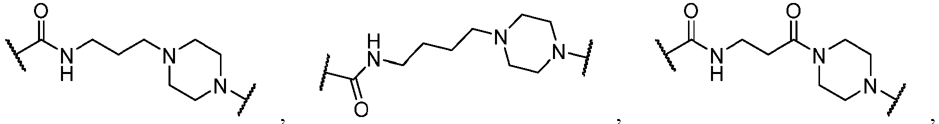

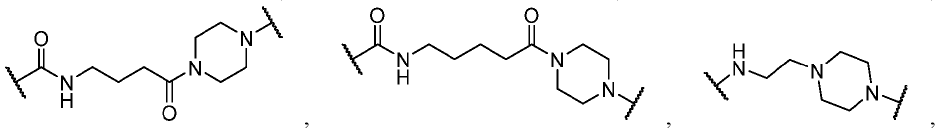

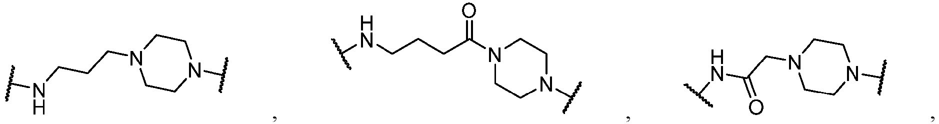

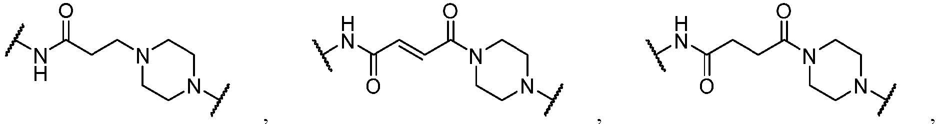

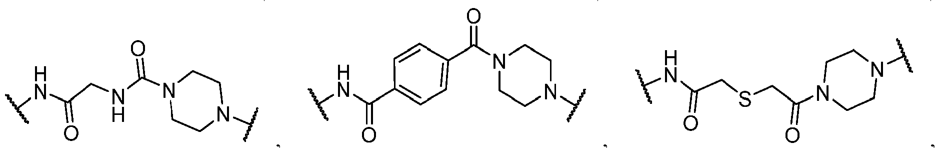

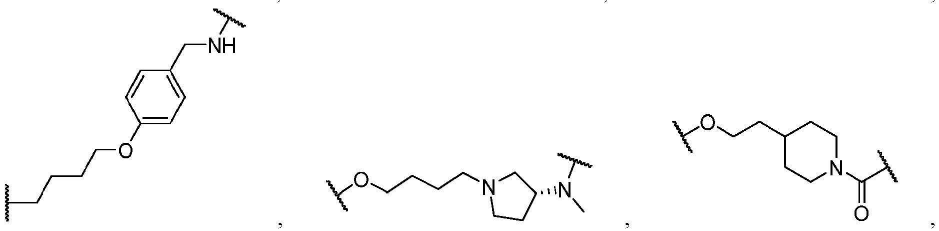

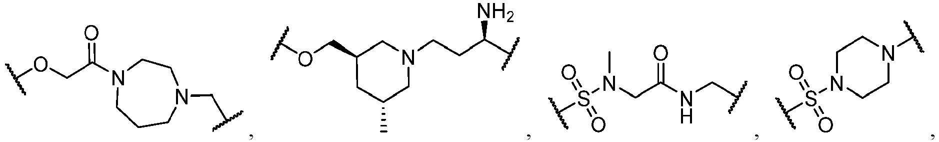

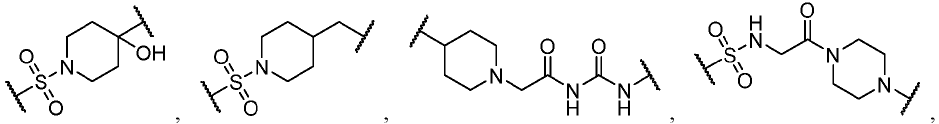

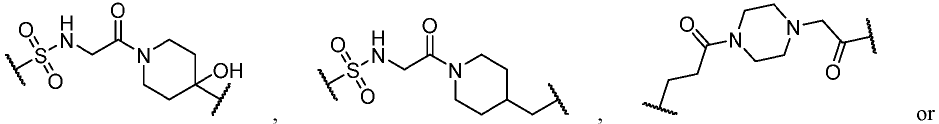

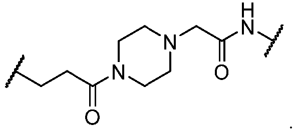

4. The method according to any one of claims 1 to 3, wherein B is

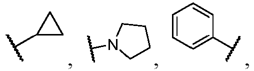

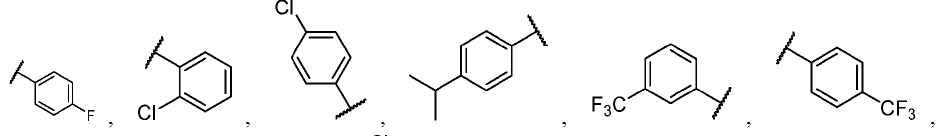

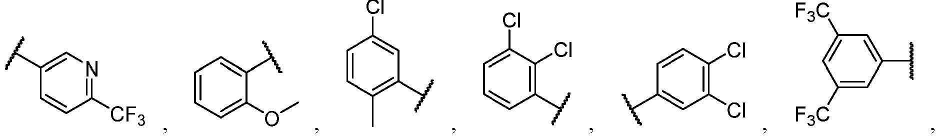

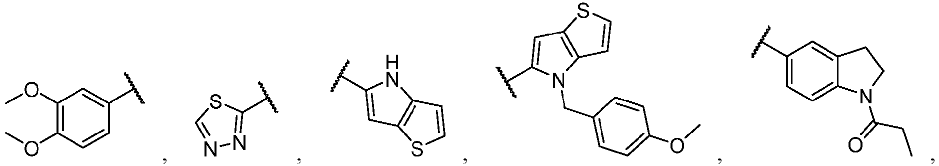

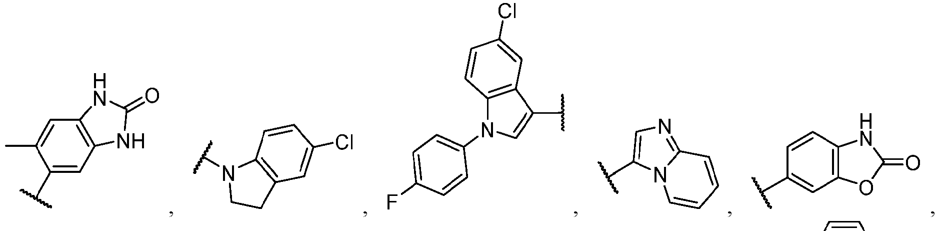

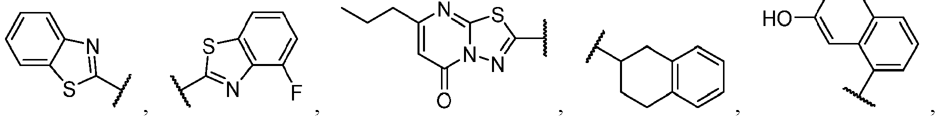

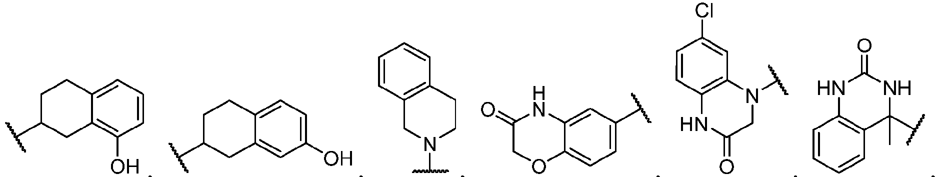

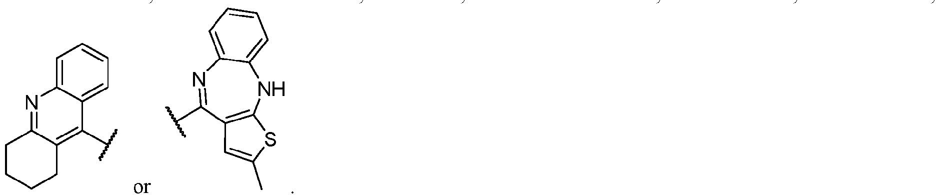

5. The method according to any one of claims 1 to 4, wherein the TPK agonist is a compound of Formula (II), or a pharmaceutically acceptable salt, ester, stereoisomer, polymorph, solvate, N-oxide, isotopically labeled compound, metabolite, or prodrug thereof:
A-W-Q¹-Q²-B (II)
wherein:
A is a benzene ring optionally fused with a 5- to 6-membered heterocycle or 5- to 6-membered heteroaromatic ring, wherein the benzene ring is optionally substituted with one or more substituents independently selected from the group consisting of: halogen, -OH, -NH₂, C₁₋₆ alkyl, -O-C₁₋₆ alkyl, -NH(C₁₋₆ alkyl) and -N(C₁₋₆ alkyl)₂; preferably, the benzene ring is optionally substituted with one or more substituents independently selected from the group consisting of: -Cl, -OH, -NH₂, -NH(CH₃), -N(CH₃)₂, methyl, ethyl and methoxy; most preferably, A is 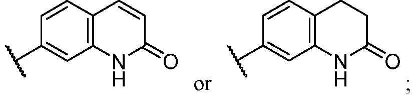
B is C₃₋₁₀ hydrocarbon ring, 3- to 14-membered heterocycle, C₆₋₁₀ aromatic ring or 5- to 14-membered heteroaromatic ring; preferably is a benzene ring, the benzene ring is optionally substituted with one or more substituents independently selected from the group consisting of: halogen, C₁₋₆ alkyl, haloC₁₋₆ alkyl, -NH-C(=O)-C₁₋₆ alkyl, -C(=O)-(3- to 14-membered heterocyclyl), -S(=O)₂-N(C₁₋₆ alkyl)₂ and -S(=O)₂-(3-to 14-membered heterocyclyl); preferably, the benzene ring is optionally substituted with one or more substituents independently selected from the group consisting of: -F, -Cl, methyl, isopropyl, trifluoromethyl, -NHC(=O)CH₃, -C(=O)-piperidinyl, -S(=O)₂-N(CH₃)₂, -S(=O)₂-N(CH₂CH₃)₂, -S(=O)₂-piperidinyl and -S(=O)₂-azepanyl;
Q¹ is selected from the group consisting of -C₁₋₆ alkylene-, -C₂₋₆ alkenylene- and -C₂₋₆ alkynylene-;
Q² is selected from the group consisting of -C₃₋₁₀ cyclic hydrocarbylene-, -(3- to 14-membered heterocyclylene)-, -C₆₋₁₀ arylene- and -(5- to 14-membered heteroarylene)-; preferably is -(3- to 14-membered heterocyclylene)-; and more preferably is piperidinylene or piperazinylene;
W, at each occurrence, is each independently selected from the group consisting of -O-, -C(=O)-, -C(=O)O-, -NR-, -C(=O)NR-, -NR-C(=O)-NR'-, -NR-C(=O)O-, -(S=O)NR-, -S(=O)₂NR-, -S-, -S(=O)- and -S(=O)₂-; preferably is -O-, -NH- or -NH-C(=O)-;
the remaining groups are as defined in any of claims 1 to 4.

6. A method for the prophylaxis or treatment of neurodegenerative diseases or alleviating symptoms of neurodegenerative diseases, which comprises administering to a subject in need thereof a prophylactically or therapeutically effective amount of a thiamine pyrophosphokinase (TPK) agonist;
preferably, the neurodegenerative disease is Alzheimer's disease;
more preferably, the Alzheimer's disease is one in which the subject has decreased TPK enzyme activity, decreased TPK expression level, and/or decreased TDP level;
wherein the TPK agonist is selected from the following compounds, or a pharmaceutically acceptable salt, ester, stereoisomer, polymorph, solvate, N-oxide, isotopically labeled compound, metabolite, or prodrug thereof:
| **No.** | **Structural Formula** |
|---|---|
| **1** | 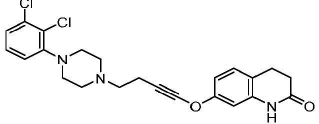 |
| **2** | 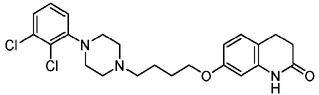 |
| **3** | 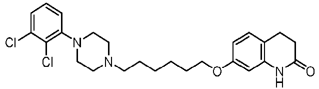 |
| **4** | 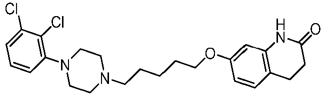 |
| **5** | 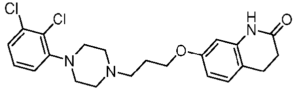 |
| **6** | 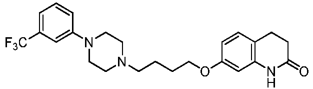 |
| **7** | 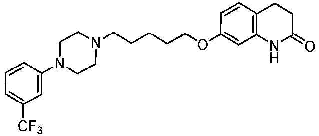 |
| **9** | 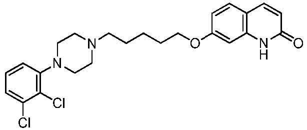 |
| **10** | 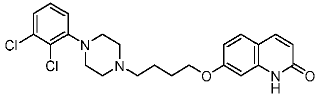 |
| **17** | 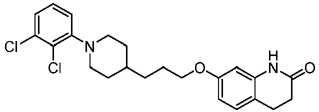 |
| **18** | 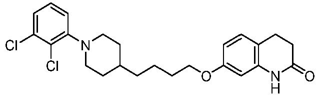 |
| **19** | 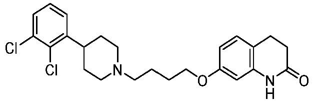 |
| **20** | 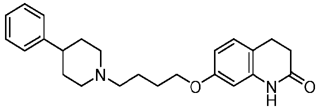 |
| **21** | 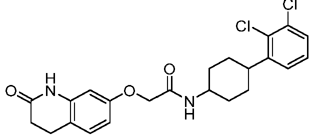 |
| **22** | 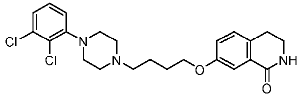 |
| **23** | 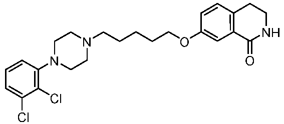 |
| **24** | 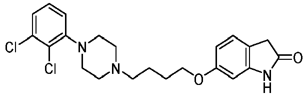 |
| **25** | 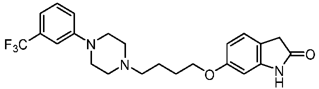 |
| **26** | 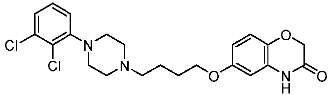 |
| **27** | 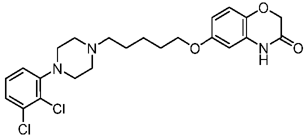 |
| **28** | 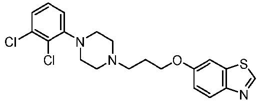 |
| **29** | 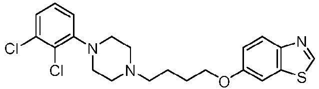 |
| **30** | 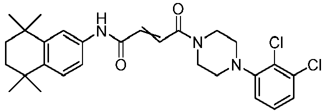 |
| **31** | 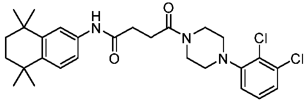 |
| **32** | 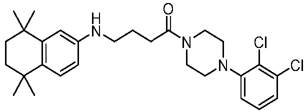 |
| **33** | 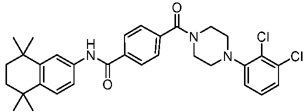 |
| **34** | 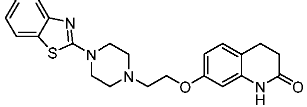 |
| **35** | 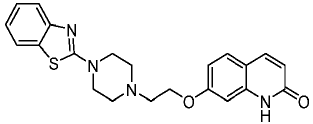 |
| **36** | 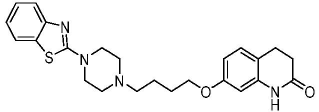 |
| **37** | 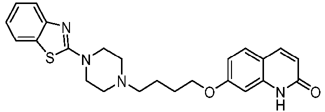 |
| **38** | 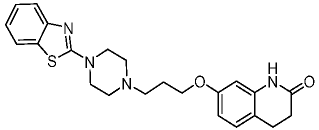 |
| **39** | 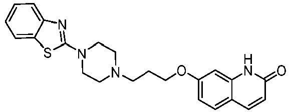 |
| **40** | 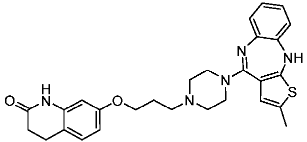 |
| **41** | 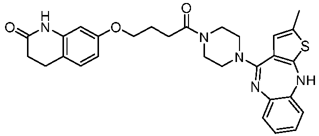 |
| **42** | 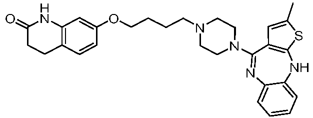 |
| **43** | 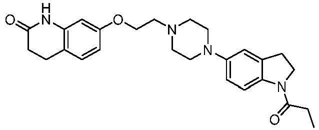 |
| **44** | 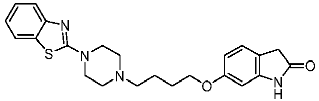 |
| **45** | 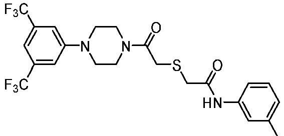 |
| 46 | 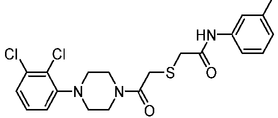 |
| 47 | 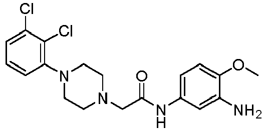 |
| 48 | 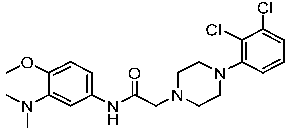 |
| 49 | 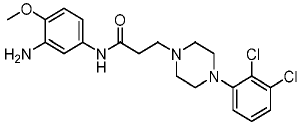 |
| 50 | 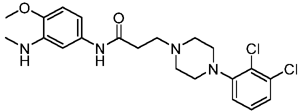 |
| **51** | 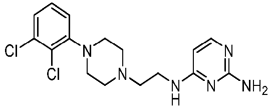 |
| **52** | 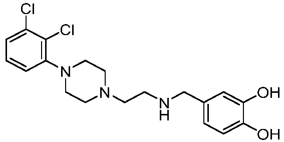 |
| **53** | 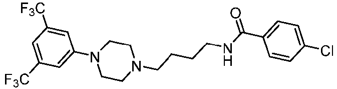 |
| **54** | 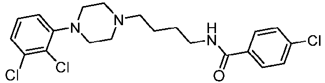 |
| **55** | 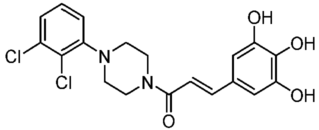 |
| **56** | 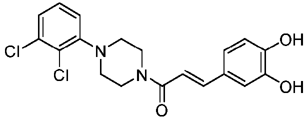 |
| **57** | 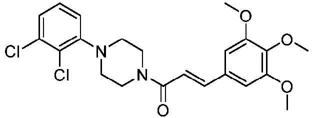 |
| **58** | 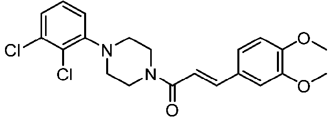 |
| **59** | 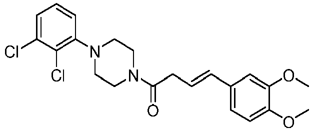 |
| **60** | 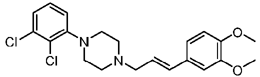 |
| **61** | 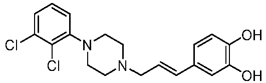 |
| **62** | 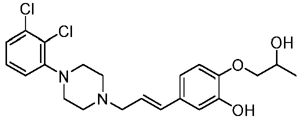 |
| **63** | 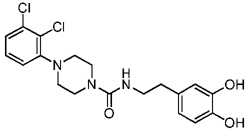 |
| **64** | 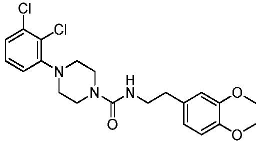 |
| **65** | 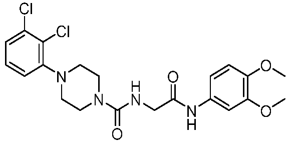 |
| **66** | 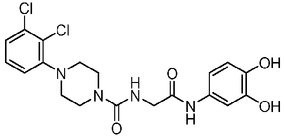 |
| **67** | 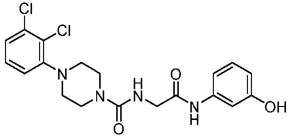 |
| **68** | 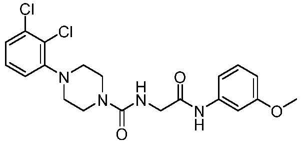 |
| **69** | 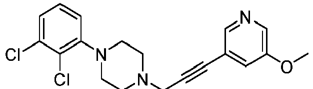 |
| **70** | 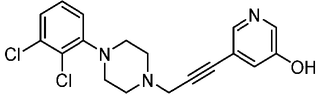 |
| **71** | 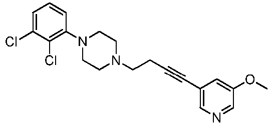 |
| **72** | 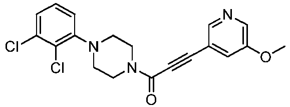 |
| **73** | 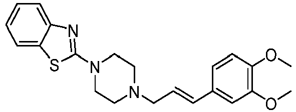 |
| **74** | 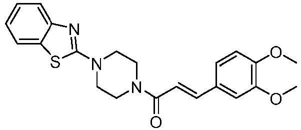 |
| **75** | 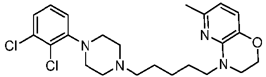 |
| **76** | 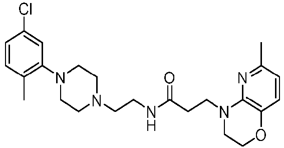 |
| **77** | 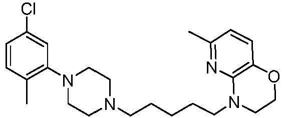 |
| **78** | 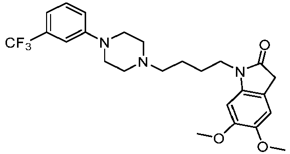 |
| **79** | 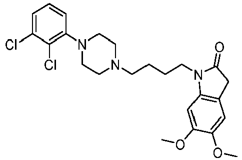 |
| **80** | 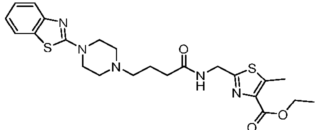 |
| **81** | 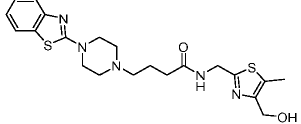 |
| **82** | 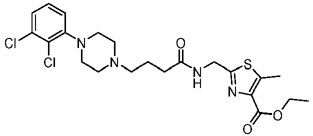 |
| **83** | 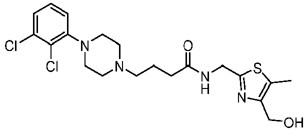 |
| **84** | 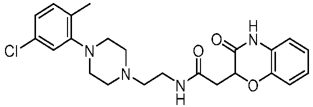 |
| **85** | 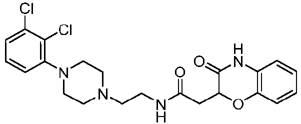 |
| **86** | 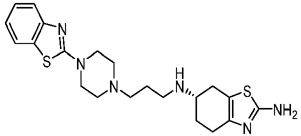 |
| **87** | 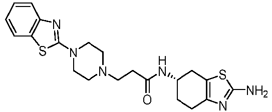 |
| **88** | 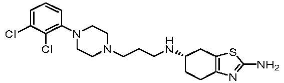 |
| **89** | 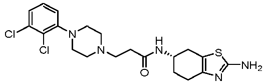 |
| **90** | 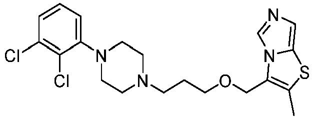 |
| **91** | 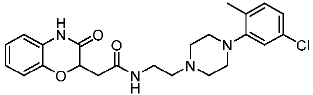 |
| **95** | 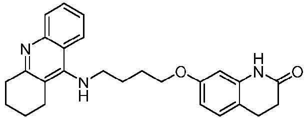 |
| **96** | 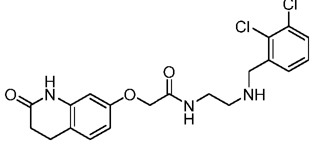 |
| **97** | 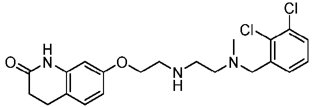 |
| **98** | 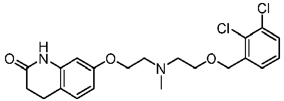 |
| **99** | 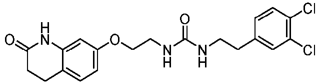 |
| **100** | 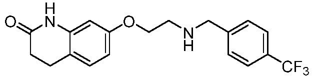 |
| **101** | 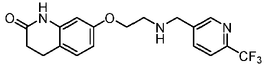 |
| **102** | 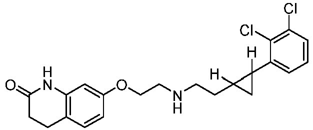 |
| **103** | 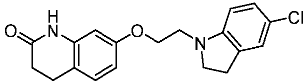 |
| **104** | 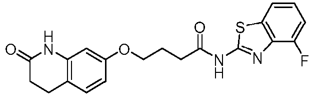 |
| **105** | 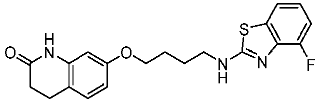 |
| **106** | 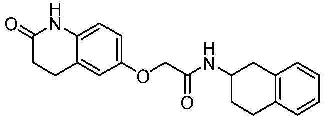 |
| **107** | 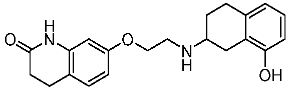 |
| **108** | 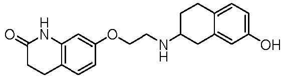 |
| **109** | 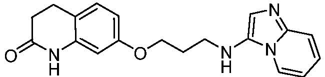 |
| **110** | 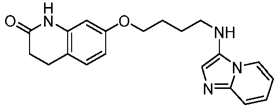 |
| **111** | 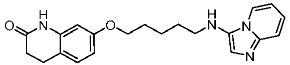 |
| **112** | 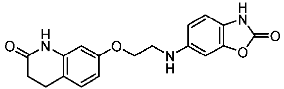 |
| **113** | 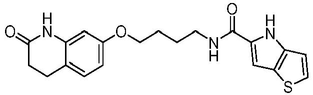 |
| **114** | 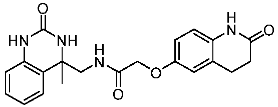 |
| **115** | 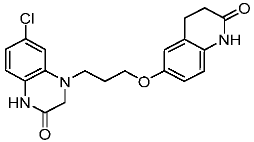 |
| **130** | 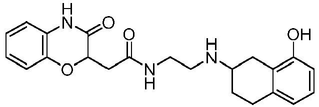 |
| **131** | 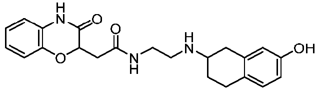 |
| **132** | 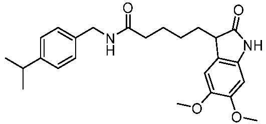 |
| **133** | 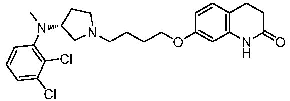 |
| **134** | 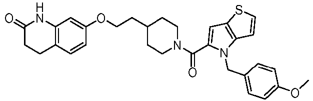 |
| **135** | 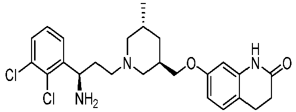 |
| **136** | 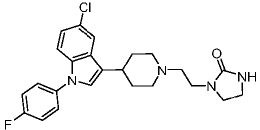 |
| **137** | 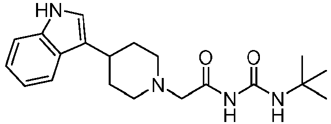 |
| **138** | 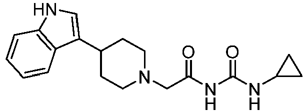 |
| **139** | 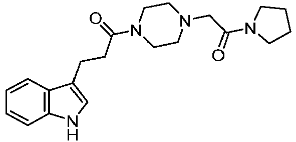 |
| **140** | 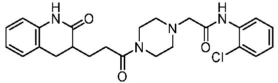 |
| **141** | 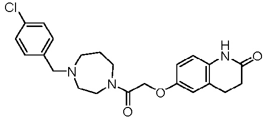 |
| **142** | 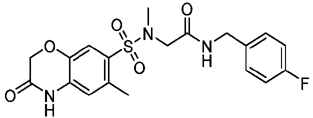 |
| **143** | 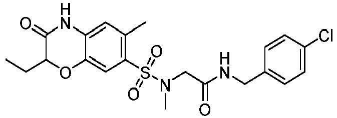 |
| **144** | 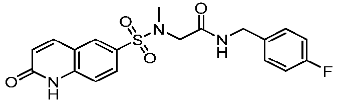 |
| **145** | 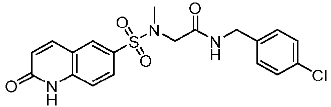 |
| **146** | 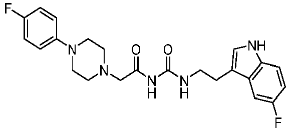 |
| **147** | 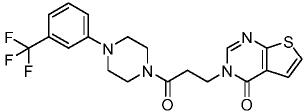 |
| **148** | 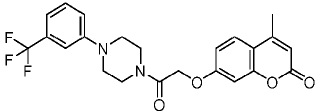 |
| **149** | 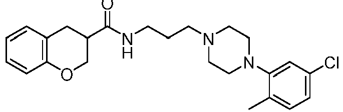 |
| **150** | 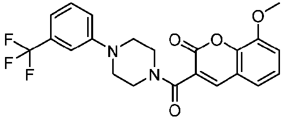 |
| **151** | 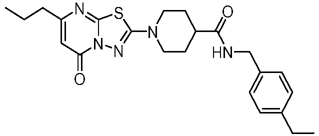 |
| **152** | 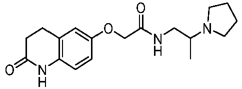 |
| **153** | 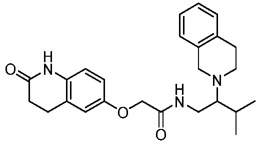 |
| **154** | 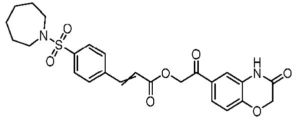 |
| **155** | 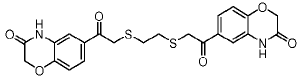 |
| **156** | 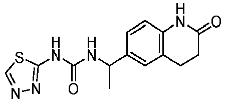 |
| **157** | 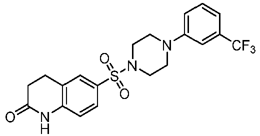 |
| **158** | 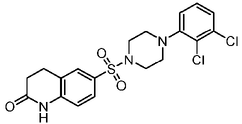 |
| **159** | 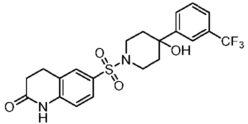 |
| **160** | 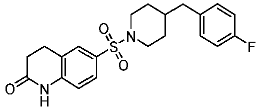 |
| **161** | 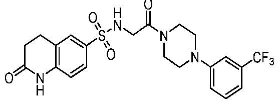 |
| **162** | 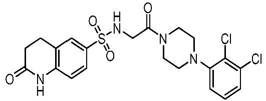 |
| **163** | 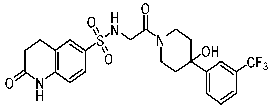 |
| **164** | 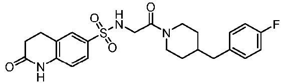 |
| **165** | 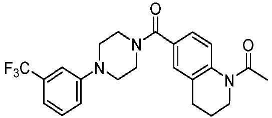 |
| **166** | 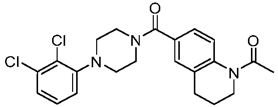 |
| **167** | 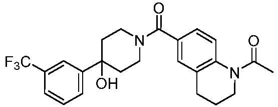 |
| **168** | 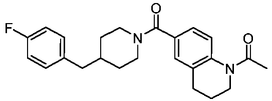 |
| **169** | 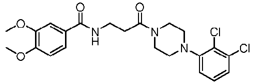 |
| **170** | 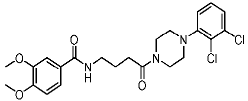 |
| **171** | 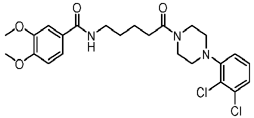 |
| **172** | 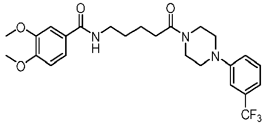 |
| **173** | 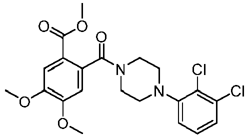 |
| **174** | 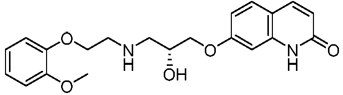 |
| **175** | 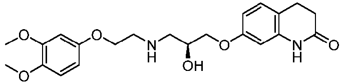 |
| **176** | 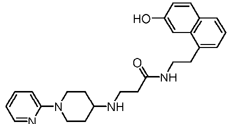 |
| **177** | 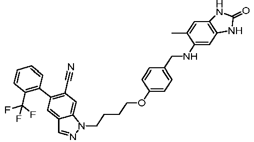 |
| **178** | 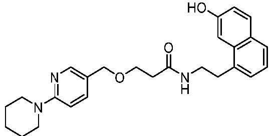 |

7. The method according to any one of claims 1 to 6, wherein the TPK agonist is administered in an amount of about 0.005 mg/day to about 5000 mg/day, e.g., in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

8. The method according to any one of claims 1 to 7, wherein the TPK agonist is administered in an amount of about 1 ng/kg to about 200 mg/kg, about 1 µg/kg to about 100 mg/kg or about 1 mg/kg to about 50 mg/kg body weight per day, e.g., is administered in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg or about 300 mg/kg body weight per day.

9. The method according to any one of claims 1 to 8, wherein the daily dose of the TPK agonist is administered at one time or is administered in two, three or four doses.

10. The method according to any one of claims 1 to 9, wherein the TPK agonist is administered continuously for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days.

11. The method according to any one of claims 1 to 10, wherein the TPK agonist is administered for one or more (e.g., 1, 2, 3, 4, 5, 6, 7, 8, 9 or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days; and the interval between every two courses of treatment is 0, 1, 2, 3, 4, 5, 6, 7, 8, 9, 10 days, two weeks, three weeks, or four weeks.

12. The method according to any one of claims 1 to 11, wherein the TPK agonist is administered through injection (e.g., intravenous, intraarterial, subcutaneous, intraperitoneal, intramuscular injection, including dripping), or transdermal administration, or is administered via oral, buccal, nasal, transmucosal, or topical route, as an ophthalmic formulation, or via inhalation.

13. The method according to any one of claims 1 to 12, wherein the TPK agonist is administered in a dosage form selected from the group consisting of tablet, capsule, lozenge, hard candy, powder, spray, cream, salve, suppository, gel, paste, lotion, ointment, aqueous suspensions, injectable solution, elixir, and syrup.

14. The method according to any one of claims 1 to 13, wherein the method improves the following pathophysiological manifestations **in** the subject: abnormal cognitive behavior, neurodegenerative changes (e.g., progressive synaptic/neuronal loss and brain atrophy), β-amyloid deposition, abnormal phosphorylation of Tau and the resulting neurofibrillary tangles, glial cell activation and inflammation, and/or impaired cerebral glucose metabolism.

15. The method according to any one of claims 1 to 14, further comprising administering one or more additional therapeutic agents.

16. A compound, or a pharmaceutically acceptable salt, ester, stereoisomer, polymorph, solvate, N-oxide, isotopically labeled compound, metabolite, or prodrug thereof, wherein the compound is selected from the group consisting of:
| **No.** | **Structural Formula** |
|---|---|
| **1** | 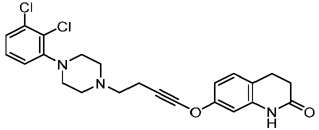 |
| **3** | 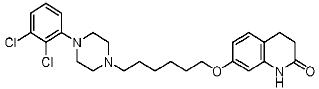 |
| **4** | 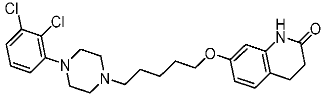 |
| **6** | 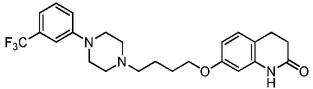 |
| **7** | 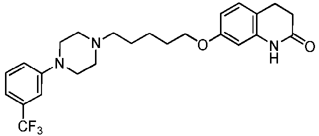 |
| **9** | 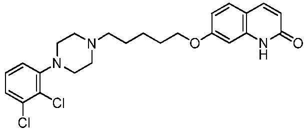 |
| **10** | 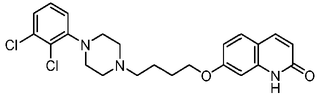 |
| **17** | 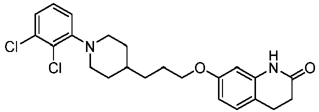 |
| **18** | 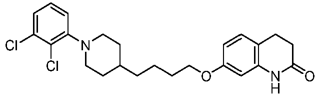 |
| **20** | 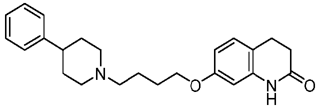 |
| **21** | 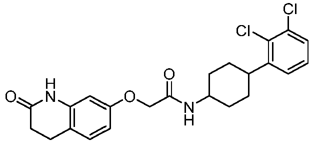 |
| **23** | 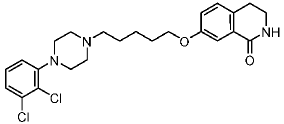 |
| **24** | 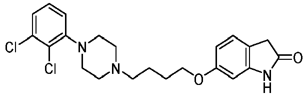 |
| **25** | 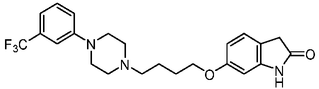 |
| **27** | 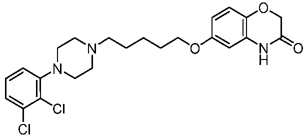 |
| **28** | 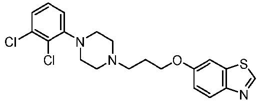 |
| **29** | 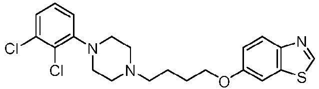 |
| **30** | 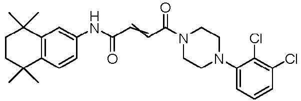 |
| **31** | 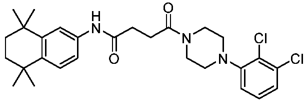 |
| **32** | 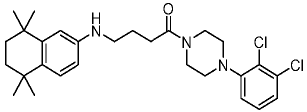 |
| **33** | 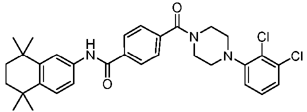 |
| **34** | 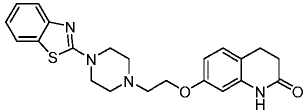 |
| **35** | 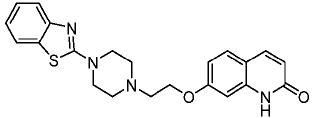 |
| **36** | 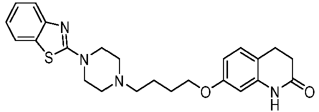 |
| **37** | 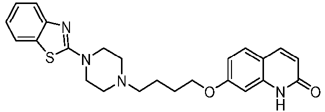 |
| **38** | 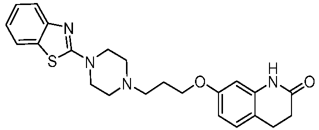 |
| **39** | 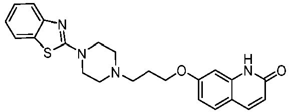 |
| **40** | 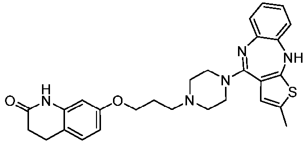 |
| **41** | 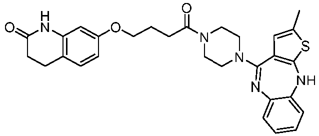 |
| **42** | 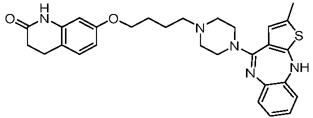 |
| **43** | 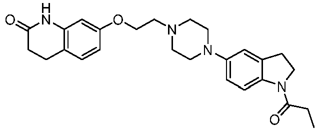 |
| **44** | 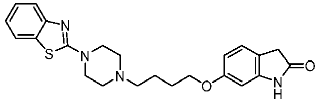 |
| **45** | 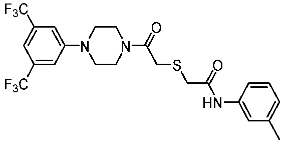 |
| **46** | 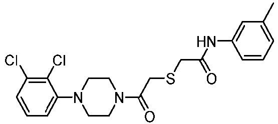 |
| **47** | 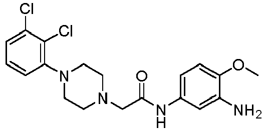 |
| **48** | 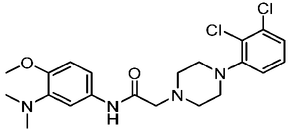 |
| **49** | 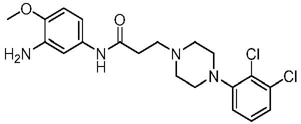 |
| **50** | 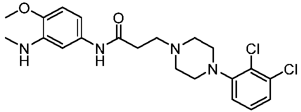 |
| **51** | 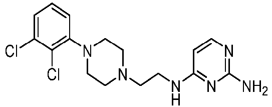 |
| **52** | 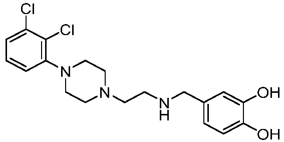 |
| **53** | 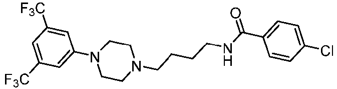 |
| **55** | 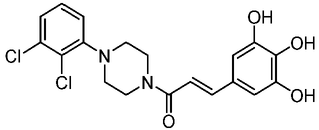 |
| **59** | 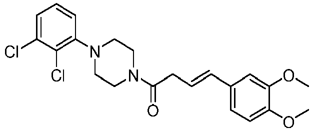 |
| **60** | 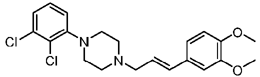 |
| **61** | 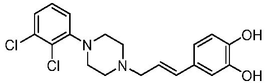 |
| **62** | 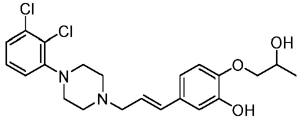 |
| **63** | 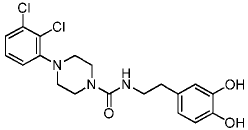 |
| **64** | 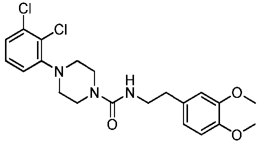 |
| **65** | 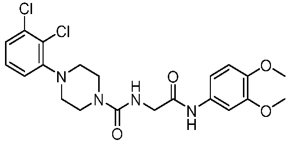 |
| **66** | 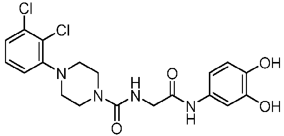 |
| **67** | 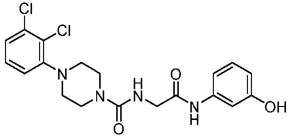 |
| **68** | 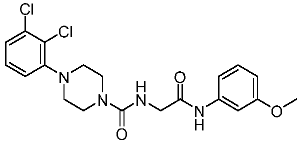 |
| **69** | 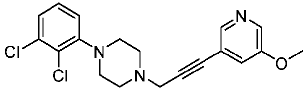 |
| **70** | 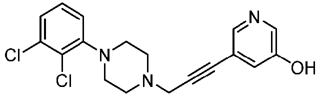 |
| **71** | 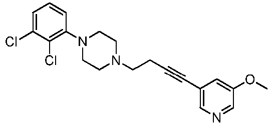 |
| **72** | 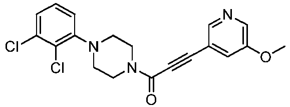 |
| **73** | 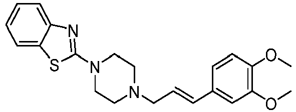 |
| **74** | 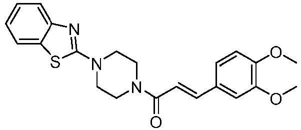 |
| **75** | 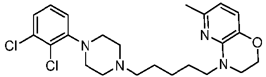 |
| **76** | 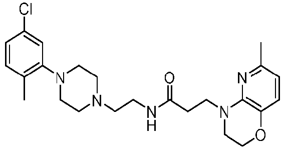 |
| **77** | 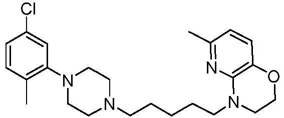 |
| **78** | 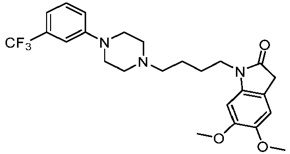 |
| **79** | 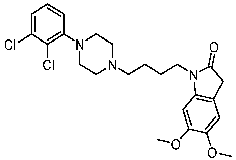 |
| **80** | 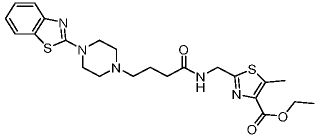 |
| **81** | 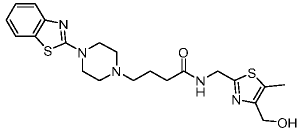 |
| **82** | 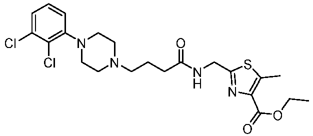 |
| **83** | 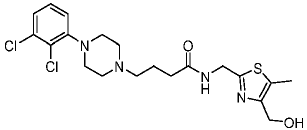 |
| **85** | 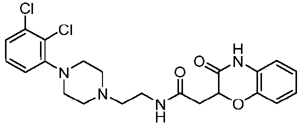 |
| **86** | 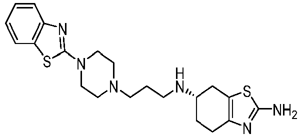 |
| **87** | 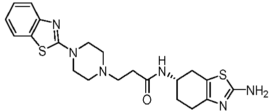 |
| **88** | 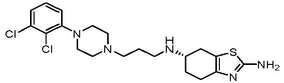 |
| **89** | 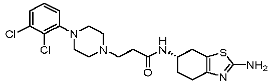 |
| **90** | 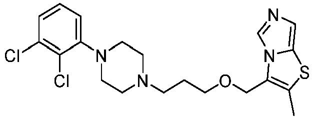 |
| **91** | 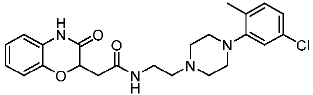 |
| **95** | 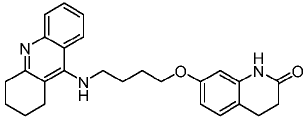 |
| **96** | 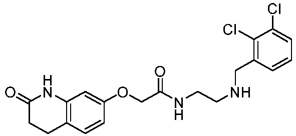 |
| **97** | 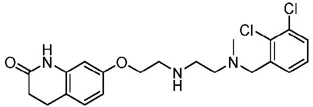 |
| **98** | 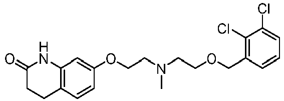 |
| **99** | 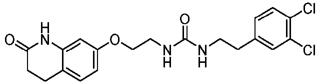 |
| **100** | 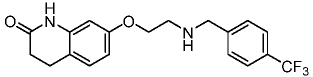 |
| **101** | 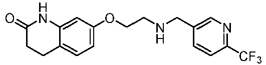 |
| **102** | 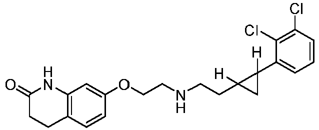 |
| **103** | 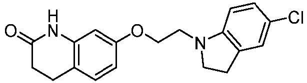 |
| **104** | 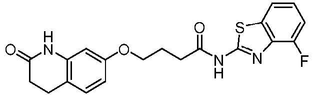 |
| **105** | 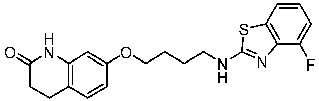 |
| **107** | 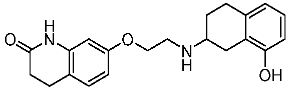 |
| **108** | 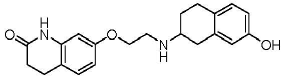 |
| **109** | 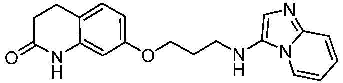 |
| **110** | 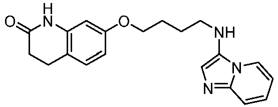 |
| **111** | 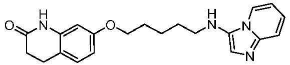 |
| **112** | 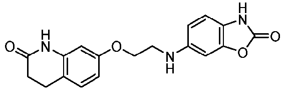 |
| **113** | 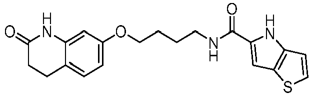 |
| **114** | 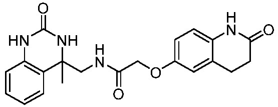 |
| **115** | 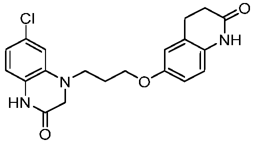 |
| **130** | 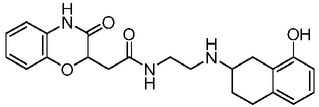 |
| **131** | 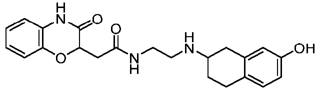 |
| **132** | 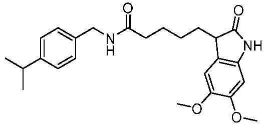 |
| **133** | 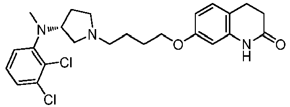 |
| **134** | 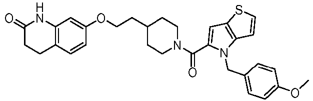 |
| **135** | 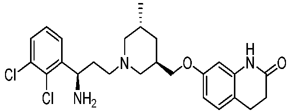 |
| **158** | 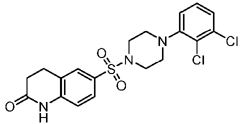 |
| **159** | 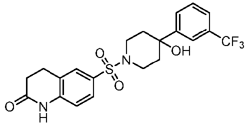 |
| **160** | 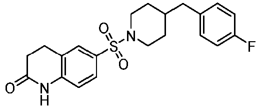 |
| **161** | 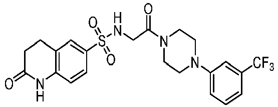 |
| **162** | 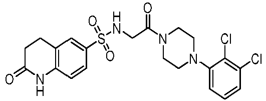 |
| **163** | 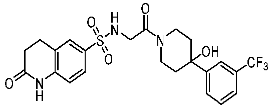 |
| **164** | 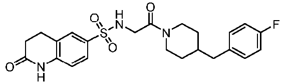 |
| **165** | 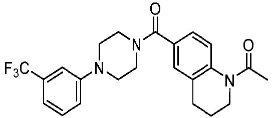 |
| **166** | 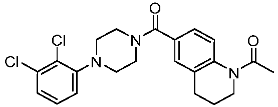 |
| **167** | 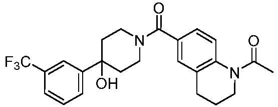 |
| **168** | 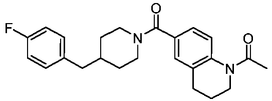 |
| **169** | 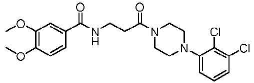 |
| **170** | 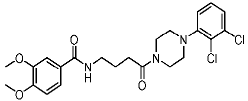 |
| **171** | 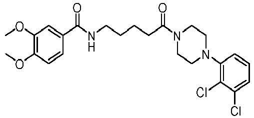 |
| **172** | 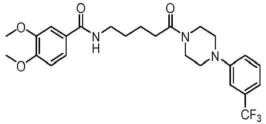 |
| **173** | 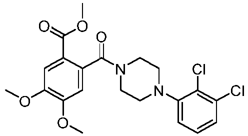 |
| **174** | 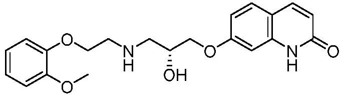 |
| **175** | 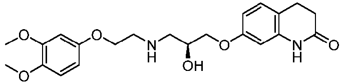 |
| **176** | 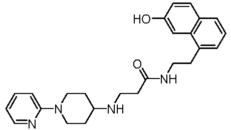 |
| **177** | 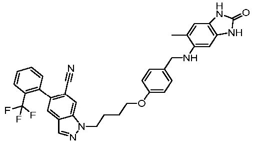 |
| **178** | 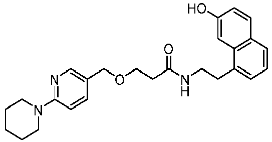 |
